# EUROPÄISCHE PATENTSCHRIFT

(11) **EP 2 766 112 B1**
(45) Veröffentlichungstag und Bekanntmachung des Hinweises auf die Patenterteilung: **18.01.2017**
(21) Anmeldenummer: 12769325.7
(22) Anmeldetag: 27.09.2012
(51) Int. Cl.: B01J 20/20, B01J 20/22, B01J 20/28, B01J 20/32, B01J 20/02, A61L 2/232, B01D 53/02

(54) **VERFAHREN ZUR SELBSTDETOXIFIZIERUNG VON ADSORPTIONSMATERIAL SOWIE FILTER UND FILTERMATERIALIEN AUFWEISEND EIN SOLCHES ADSORPTIONSMATERIAL**
METHOD FOR SELF-DETOXIFICATION OF ADSORBENT MATERIAL AND FILTER AND FILTERMATERIAL FEATURING SUCH ADSORBENT MATERIAL
PROCÉDÉ D'AUTO-DÉTOXIFICATION D'UN MATÉRIAU ADSORBANT ET FILTRE ET MATÉRIAU FILTRANT PRÉSENTANT UN TEL MATÉRIAU ADSORBANT

(30) Priorität: 30.12.2011 DE 102011122587; 04.01.2012 DE 102012000087; 30.03.2012 DE 102012006421
(43) Veröffentlichungstag der Anmeldung: 20.08.2014
(73) Patentinhaber: Blücher GmbH, 40699 Erkrath (DE)
(72) Erfinder: BÖHRINGER, Bertram, 42115 Wuppertal (DE); GIEBELHAUSEN, Jann-Michael, 14712 Rathenow (DE); SCHRAGE, Christian, 01097 Dresden (DE); FICHTNER, Sven, 14727 Premnitz (DE)
(74) Vertreter: Von Rohr Patentanwälte Partnerschaft mbB
(86) Internationale Anmeldenummer: PCT/EP2012/004057
(87) Internationale Veröffentlichungsnummer: WO 2013/097912

(56) Entgegenhaltungen:
- US-A1- 2008 293 565
- BROWN P N ET AL: "Effect of ageing and moisture on the retention of hydrogen cyanide by impregnated activated charcoals", CARBON, ELSEVIER, OXFORD, GB, Bd. 27, Nr. 6, 1. Januar 1989 (1989-01-01) , Seiten 821-833, XP024032627, ISSN: 0008-6223, DOI: 10.1016/0008-6223(89)90032-8 [gefunden am 1989-01-01]
- PRASAD G K ET AL: "Reactions of sulphur mustard on impregnated carbons", JOURNAL OF HAZARDOUS MATERIALS, ELSEVIER, AMSTERDAM, NL, Bd. 116, Nr. 3, 31. Dezember 2004 (2004-12-31), Seiten 213-217, XP004682199, ISSN: 0304-3894, DOI: 10.1016/J.JHAZMAT.2004.09.003
- SHARMA ET AL: "In-situ degradation of sulphur mustard and its simulants on the surface of impregnated carbon systems", JOURNAL OF HAZARDOUS MATERIALS, ELSEVIER, AMSTERDAM, NL, Bd. 133, Nr. 1-3, 20. Mai 2006 (2006-05-20), Seiten 106-112, XP005424604, ISSN: 0304-3894, DOI: 10.1016/J.JHAZMAT.2005.09.053
- NICKOLOV R ET AL: "Comparative study on removal efficiency of impregnated carbons for hydrogen cyanide vapors in air depending on their phase composition and porous textures", JOURNAL OF COLLOID AND INTERFACE SCIENCE, ACADEMIC PRESS, NEW YORK, NY, US, Bd. 273, Nr. 1, 1. Mai 2004 (2004-05-01), Seiten 87-94, XP027132325, ISSN: 0021-9797 [gefunden am 2004-04-02]

## Beschreibung

Die vorliegende Erfindung betrifft das technische Gebiet adsorptiver Materialien insbesondere auf Basis von Aktivkohle, welche selbstdetoxifizierende bzw. selbstreinigende bzw. selbstregenerierende Eigenschaften aufweisen.

Insbesondere betrifft die vorliegende Erfindung ein Verfahren zur Selbstdetoxifizierung bzw. Selbstreinigung von Adsorptionsmaterialien in Form von partikulärer und ein inneres Porensystem aufweisender Aktivkohle.

Zudem betrifft die vorliegende Erfindung auch Filter bzw. Filtermaterialien, welche unter Verwendung eines selbstdetoxifizierenden bzw. selbstreinigenden Adsorptionsmaterials bzw. der Adsorbentien nach der Erfindung hergestellt sind bzw. welche ein selbstdetoxifizierendes bzw. selbstreinigendes Adsorptionsmaterial aufweisen.

Chemische und biologische Schad- bzw. Giftstoffe können in vielfältigster Weise in der Umwelt bzw. in der Umgebung des Menschen auftreten und stellen ein großes Gefährdungspotential für die menschliche Gesundheit sowie für das menschliche Leben dar. In diesem Zusammenhang können die zugrundeliegenden chemischen und biologischen Gift- bzw. Schadstoffe unterschiedlichsten Ursprungs sein:
So fallen derartige gesundheitsschädliche bzw. toxische Verbindungen oftmals im Rahmen industrieller Verfahren und Herstellungsprozesse als unerwünschte Neben- bzw. Abfallprodukte an, von denen insbesondere bei unvorhersehbarer bzw. unkontrollierter Freisetzung eine hohe Gefahr ausgeht.
Zudem werden chemische und biologische Schad- bzw. Giftstoffe auch als chemische bzw. biologische Kampfstoffe eingesetzt, welche synonym auch als so genannte *Chemical Warfare Agents* (CWAs) bezeichnet werden. Derartige Kampfstoffe wurden beispielsweise im Ersten Weltkrieg eingesetzt und sind bis zum heutigen Tage Bestandteil des Waffenarsenals zahlreicher Staaten. Zudem besteht aufgrund der prinzipiell leichten Herstellbarkeit und einfachen Transporteigenschaften die Gefahr, dass terroristische Gruppen in den Besitz derartiger Kampfstoffe gelangen, so dass auch diesbezüglich eine permanente Gefahr des Missbrauchs vorliegt. Als bekannteste Vertreter der Gruppe der Kampfstoffe sind insbesondere Senfgas (synonym auch als "HD" bezeichnet), Soman, Sarin, Phosgen sowie Tabun zu nennen.

Im Stand der Technik sind in diesem Zusammenhang zahlreiche Methoden bekannt, auf deren Basis ein Schutz gegenüber den zuvor angeführten Schad- bzw. Giftstoffen, insbesondere gegenüber Kampfstoffen, gewährleistet werden soll. Hierzu zählt beispielsweise der Einsatz von flüssigkeits- bzw. gasundurchlässigen Barrierematerialien, welche in entsprechender Weise beispielsweise zu Schutzanzügen verarbeitet werden können. Die hierzu eingesetzten Materialien weisen jedoch aufgrund der mit den entsprechenden Materialeigenschaften einhergehenden Luft- und Wasserdampfundurchlässigkeit mitunter einen nur unzureichenden Tragekomfort auf.

Eine universelle wie gleichsam effiziente Methode zur Gewährleistung einer Schutzfunktion gegenüber den in Rede stehenden chemischen und biologischen Schad- bzw. Giftstoffen stellt die gezielte Verwendung von Adsorptionsmaterialien, wie beispielsweise Zeolithen, Molekularsieben, Aktivkohle oder dergleichen dar. Derartige Materialien können z. B. in Form von Filtern, beispielsweise auf Basis von Filtern für ABC-Schutzmasken oder dergleichen, eingesetzt werden. Zudem kommt ein Einsatz derartiger Adsorptionsmaterialien beispielsweise in Schutzanzügen in Betracht, wobei die zugrundeliegenden Adsorptionsmaterialien oftmals auf einer Trägerstruktur fixiert sind. Dabei können grundsätzlich auch luft- und wasserdampfdurchlässige Materialien realisiert werden, was insbesondere den Tragekomfort der auf dieser Basis hergestellten Schutzanzüge erhöht - und dies ohne das Schutzpotential signifikant zu verringern.

Aktivkohle ist in diesem Zusammenhang aufgrund ihrer recht unspezifischen und damit breitbandigen adsorptiven Eigenschaften - was eine Schutzfunktion gegenüber zahlreichen chemischen bzw. biologischen Schad- bzw. Giftstoffen unterschiedlichster Natur gewährleistet - sowie ihrer hohen Adsorptionskapazität das am häufigsten eingesetzte Adsorbens. Gesetzliche Auflagen, aber auch das steigende Bewusstsein der Verantwortung für die Umwelt, führen zu einem steigenden Bedarf an Aktivkohle.

Aktivkohle wird im Allgemeinen durch Carbonisierung (synonym auch als Schwelung, Pyrolyse, Abbrand etc. bezeichnet) und anschließende Aktivierung kohlenstoffhaltiger Ausgangsverbindungen erhalten, wobei solche Ausgangsverbindungen bevorzugt werden, die zu ökonomisch vernünftigen Ausbeuten führen. Denn die Gewichtsverluste durch Abspalten flüchtiger Bestandteile bei der Carbonisierung und durch den nachfolgenden Abbrand bei der Aktivierung sind erheblich. Für weitergehende Einzelheiten zu der Aktivkohleherstellung kann beispielsweise verwiesen werden auf H. v. Kienle und E. Bäder, "Aktivkohle und ihre industrielle Anwendung", Enke Verlag Stuttgart, 1980.

Die Beschaffenheit der erzeugten Aktivkohle - fein- oder grobporig, fest oder brüchig etc. - hängt dabei auch vom Ausgangsmaterial ab. Übliche Ausgangsmaterialien sind Kokosnussschalen, Holzkohle und Holz (z. B. Holzabfälle), Torf, Steinkohle, Peche, aber auch besondere Kunststoffe, die unter anderem bei der Herstellung von Aktivkohlegeweben eine gewisse Rolle spielen.

Aktivkohle wird in verschiedenen Formen verwendet: Pulverkohle, Splitterkohle bzw. Kornkohle, Formkohle und seit Ende der 1970er Jahre auch kugelförmige Aktivkohle ("Kugelkohle"). Kugelförmige Aktivkohle hat gegenüber anderen Formen von Aktivkohle, wie Pulver-, Splitter-, Korn- und Formkohle und dergleichen, eine Reihe von Vorteilen, die sie für bestimmte Applikationen wertvoll oder sogar unverzichtbar macht: Sie ist rieselfähig, abriebfest bzw. staubfrei und hart. Kugelkohle ist wegen ihrer speziellen Form, aber auch wegen der hohen Abriebfestigkeit beispielsweise für besondere Einsatzgebiete sehr gefragt.

Kugelkohle wird heute noch meist durch mehrstufige und sehr aufwendige Verfahren hergestellt. Das bekannteste Verfahren besteht in der Herstellung von Kügelchen aus Steinkohlenteerpech und geeigneten asphaltartigen Rückständen der Erdölchemie, welche oxidiert - damit sie unschmelzbar werden - und nachfolgend geschwelt und aktiviert werden. Beispielsweise kann die Kugelkohle auch in einem mehrstufigen Verfahren ausgehend von Bitumen hergestellt werden. Diese mehrstufigen Verfahren sind sehr kostenintensiv, und der damit verbundene hohe Preis der so erhältlichen Kugelkohle verhindert viele Anwendungen, bei denen die Kugelkohle aufgrund ihrer Eigenschaften eigentlich bevorzugt werden müsste.

In der WO 98/07655 A1 wird ein Verfahren zur Herstellung von Aktivkohlekügelchen beschrieben, bei dem zunächst eine Mischung, die einen aus der Diisocyanatherstellung stammenden Destillationsrückstand, einen kohlenstoffhaltigen Verarbeitungshilfsstoff und gegebenenfalls einen oder mehrere weitere Zusatzstoffe umfasst, zu rieselförmigen Kügelchen verarbeitet wird und anschließend die auf diese Weise erhaltenen Kügelchen carbonisiert und dann aktiviert werden.

Aus dem Stand der Technik bekannt ist ferner die Herstellung von Kugelkohle durch Schwelung und anschließende Aktivierung von neuen oder gebrauchten Ionenaustauschern, die Sulfonsäuregruppen enthalten, bzw. durch Schwelung von Ionenaustauschervorstufen in Gegenwart von Schwefelsäure mit anschließender Aktivierung, wobei die Sulfonsäuregruppen bzw. die Schwefelsäure die Funktion eines Vernetzers haben. Solche Verfahren sind beispielsweise in der DE 43 28 219 A1 und in der DE 43 04 026 A1 sowie in der DE 196 00 237 A1 einschließlich der deutschen Zusatzanmeldung DE 196 25 069 A1 beschrieben.

Weiterhin sind aus dem Stand der Technik Verfahren bekannt, bei denen die Herstellung von Aktivkohle, insbesondere Kugelkohle, durch Schwelung und anschließende Aktivierung sulfonierter divinylbenzolvernetzter Polystyrole (d. h. sulfonierte Styrol/Divinylbenzol-Copolymere) erfolgt (vgl. z. B. DE 10 2007 O50 971 A1).

Bei speziellen Anwendungen ist aber nicht nur die Geometrie bzw. die äußere Gestalt der Aktivkohle von entscheidender Bedeutung, sondern auch deren Porosität, insbesondere das Gesamtporenvolumen und die Adsorptionskapazität einerseits und die Verteilung der Poren, d. h. der Anteil an Mikro-, Meso- und Makroporen in Bezug auf das Gesamtporenvolumen, andererseits; insbesondere kann die Porosität durch die Auswahl der Ausgangsmaterialien sowie die Verfahrensbedingungen gezielt gesteuert werden. Im Rahmen der vorliegenden Erfindung bezeichnet der Begriff der Mikroporen solche Poren mit Porendurchmessern von weniger als 2 nm, wohingegen der Begriff der Mesoporen solche Poren mit Porendurchmessern im Bereich von 2 nm (d. h. 2 nm einschließlich) bis 50 nm einschließlich bezeichnet und der Begriff der Makroporen solche Poren mit Porendurchmessern von mehr als 50 nm (d. h. > 50 nm) bezeichnet.

Aufgrund ihrer guten adsorptiven Eigenschaften kommt Aktivkohle für eine Vielzahl von Anwendungen zum Einsatz: So wird Aktivkohle beispielsweise in der Medizin oder Pharmazie eingesetzt, aber auch in der Lebensmittelindustrie. Auch für Filteranwendungen findet Aktivkohle weitreichende Anwendungen (z. B. Filtration von Gasen und Flüssigkeiten, Entfernung von unerwünschten oder schädlichen bzw. toxischen Gasen etc.).

Insbesondere kann Aktivkohle in Adsorptionsfiltermaterialien, insbesondere auch speziell in Schutzmaterialien gegenüber Giften, wie chemischbiologischen Kampfstoffen, wie beispielsweise ABC-Schutzbekleidung, zum Einsatz kommen. Zu diesem Zweck sind insbesondere luft- und wasserdampfdurchlässige Schutzanzüge gegen chemische Kampfstoffe bekannt; derartige luft- und wasserdampfdurchlässige Schutzanzüge besitzen oftmals eine Adsorptionsfilterschicht mit Aktivkohle, welche die chemischen Gifte adsorbieren.

Die mit der Verwendung von porösen Adsorbentien, insbesondere in Form von Aktivkohle, einhergehenden Nachteile sind insbesondere darin zu sehen, dass die Kapazität im Hinblick auf die Adsorption der in Rede stehenden chemischen bzw. biologischen Schad- bzw. Giftstoffe begrenzt ist. So führt eine insbesondere dauerhafte Bindung der adsorbierten Substanzen in dem Porensystem der zugrundeliegenden Adsorbentien zu einer gewissen Absättigung bzw. Erschöpfung, so dass die Aufnahme weiterer Substanzen begrenzt ist. Auch besteht hinsichtlich der mit Schad- bzw. Giftstoffen beladenen Adsorbentien die Gefahr der Desorption und somit der erneuten Freisetzung von zuvor adsorbierten Schad- bzw. Giftstoffen.

Aufgrund ihrer begrenzten Adsorptionskapazität werden Adsorbentien als solche und insbesondere Aktivkohle im Speziellen im Stand der Technik bislang häufig als Einwegmaterial eingesetzt, welches nach entsprechender Beladung mit den Schad- bzw. Giftstoffen in aufwendiger Weise entsorgt bzw. gelagert werden muss, wobei auch diesbezüglich jederzeit die Gefahr einer Desorption der adsorbierten Schad- bzw. Giftstoffe besteht.

Darüber hinaus sind im Stand der Technik bislang auch Versuche unternommen worden, mit Schad- bzw. Giftstoffen beladene Adsorbentien zu regenerieren, wobei diesbezüglich verschiedene Ansätze realisiert worden sind:
So ist im Stand der Technik eine thermische Behandlung unter hohen Temperaturen von mit Schad- bzw. Giftstoffen beladenen Adsorbentien vorgesehen, was jedoch mit dem grundsätzlichen Nachteil behaftet ist, dass hierzu nur separat bzw. isoliert vorliegende Adsorbentien als solche herangezogen werden können, und zwar insbesondere sofern diese in Form von losen Schüttungen vorliegen. Sofern die zu dekontaminierenden Adsorbentien an einer Trägerstruktur, wie beispielsweise einem textilen Flächenmaterial oder einer insbesondere luft- und wasserundurchlässigen Membran fixiert sind, ist die Möglichkeit der thermischen Regeneration sehr begrenzt, da die entsprechenden Trägermaterialien oftmals temperaturempfindlich sind. Zudem ist die Handhabung der freigesetzten Schad- bzw. Giftstoffe oftmals problematisch. Um dem Problem der Freisetzung wiederum zu entgegnen, werden im Rahmen der thermischen Regeneration von Adsorbentien oftmals hohe Temperaturen eingesetzt, welche zu einer Zerstörung der Schad- bzw. Giftstoffe führten sollen, was jedoch wiederum mit dem Nachteil einhergeht, dass auch die Adsorbentien als solche negativ beeinträchtigt werden können.

Im Stand der Technik ist des Weiteren eine chemische Dekontamination von mit Schad- bzw. Giftstoffen beladenen Adsorbentien bekannt, wobei diesbezüglich entsprechende chemische Dekontaminationslösungen eingesetzt werden müssen. Derartige Regenerationsverfahren implizieren jedoch mitunter aufwendige Waschvorgänge und eine erneute Aktivierung der so behandelten Aktivkohle. Zudem kann die Adsorptionskapazität einer derartig behandelten Aktivkohle durch in der Aktivkohle verbleibende Reste der Dekontaminationslösung eingeschränkt sein. Zudem sind die eingesetzten Dekontaminationslösungen als solche oftmals problematisch, insbesondere sofern diese mit den freigesetzten toxischen Substanzen kontaminiert sind.

Darüber hinaus ist im Stand der Technik auch eine Ausrüstung der Aktivkohle auf Basis von so genannten Salzimprägnierungen, insbesondere Metallsalzen, bekannt.

Zur Steigerung der Adsorptionsleistung werden permeable adsorptive Filtersysteme, insbesondere auf Basis von Aktivkohle, oft mit einer katalytisch aktiven oder reaktiven Komponente ausgerüstet, indem die Aktivkohle beispielsweise mit einem biozid bzw. biostatisch wirkenden Katalysator imprägniert wird.

Eine spezielle, in diesem Zusammenhang zum Einsatz kommende Imprägnierung ist beispielsweise eine so genannte ABEK-Imprägnierung, welche gegenüber spezifischen toxischen Substanzen eine katalytische Wirkung aufweist. In diesem Zusammenhang bezieht sich Typ A auf bestimmte organische Gase und Dämpfe mit einem Siedepunkt > 65°C, beispielsweise Cyclohexan. Typ B bezieht sich auf bestimmte anorganische Gase und Dämpfe, beispielsweise Cyanwasserstoff. Typ E bezieht sich auf eine abbauende bzw. schützende Wirkung gegenüber Schwefeldioxid und anderen sauren Gasen und Dämpfen. Typ K schließlich bezieht sich auf eine Schutzfunktion gegenüber Ammoniak und organischen Ammoniakderivaten. Für weiterführende Informationen kann auf die diesbezügliche Europäische Norm EN 14387 (Januar 2004) verwiesen werden.

Nachteilig bei herkömmlichen Imprägnierungen von Aktivkohle mit Metallsalzen ist insbesondere die Tatsache, dass durch die Imprägnierung ein Teil der Adsorptionskapazität der Aktivkohle, welche für die Adsorption und somit für die Unschädlichmachung von chemischen Schadstoffen benötigt wird, verlorengeht. Durch die aus dem Stand der Technik bekannten Imprägnierprozesse der Aktivkohle mit Metallsalzen wird somit die Leistungsfähigkeit der Aktivkohle nachteilig beeinflusst. Des Weiteren wird durch eine herkömmliche Imprägnierung nicht immer die gewünschte Wirksamkeit erreicht. Auch das Problem des Durchschlagens von Gift- bzw. Kampfstoffen bei hohen Konzentrationen wird durch dieses Prinzip nicht immer gelöst; andererseits wird bei sehr geringen Konzentrationen an zu entfernenden Schadstoffen oder störenden Gasen (z. B. bei der Luftaufbereitung für Reinraumbedingungen) oft nicht die gewünschte Effizienz erreicht, da eine Adsorption erst bei höheren Konzentrationen einsetzt. Schließlich benötigt der herkömmliche Imprägnierprozess mit Metallsalzen relativ große Mengen des Imprägniermaterials. Zudem ist eine gewünschte homogene Beladung der Adsorbentien mit dem Imprägniermaterial nicht im zufriedenstellenden Maße möglich.

Die wissenschaftliche Publikation gemäß Brown P N et al: "Effect of ageing and moisture on the retention of hydrogen cyanide by impregnated activated charcoals", Carbon, Elsevier, Oxford, GB, Bd. 27, Nr. 6, 1. Januar 1989, Seiten 821 bis 833*,* betrifft aktivierte Aktivkohlepartikel bzw. -fasern, welche mit Kupfer und Chromat ausgerüstet sind, wobei diesbezüglich das Aufnahmeverhalten gegenüber Cyanwasserstoff bzw. Cyanogen unter Einfluss von Alterungsprozessen untersucht werden soll.

Weiterhin betrifft die wissenschaftliche Publikation gemäß Prasad G K et al: "Reactions of Sulphur Mustard on Impregnated Carbons", Journal of Hazardous Materials, Elsevier, Amsterdam, NL, Bd. 116, Nr. 3, 31. Dezember 2004 , Seiten 213 bis 217*,* das Reaktionsverhalten von Senfgas bzw. *sulphur mustard* in Verbindung mit einer imprägnierten Aktivkohle, welche neben weiteren katalytischen Komponenten auch Chrom(VI)-Oxid aufweisen soll.

Weiterhin betrifft die wissenschaftliche Publikation gemäß Sharma et al: "Insitu Degradation of Sulphur Mustard and Its Simulants on the Surface of Impregnated Carbon Systems", Journal of Hazardous Materials, Elsevier, Amsterdam, NL, Bd. 133, Nr. 1-3, 20. Mai 2004, Seiten 106 bis 112*,* wissenschaftliche Untersuchungen zum *in-situ-*Abbau von Senfgas und vergleichbaren Substanzen unter Verwendung eines imprägnierten Aktivkohlesystems.

Darüber hinaus betriff die wissenschaftliche Publikation gemäß Nickolov R et al: "Comparative Study on Removal Efficiency of Impregnated Carbons for Hydrogen Cyanide Vapors in Air Depending on their Phase Composition and Porous Textures", Journal of Colloid and Interface Science, Academic Press, New York, NY, US, Bd. 273, Nr. 1, 1. Mai 2004, Seiten 87 bis 94, wissenschaftliche Vergleichsstudien zum Abbauverhalten von imprägnierten Aktivkohlen in Bezug auf Cyanwasserstoff bzw. Blausäure enthaltenden Atmosphären.

Die US 2008/293565 A1 betrifft ein Verfahren zur Herstellung einer metallimprägnierten Aktivkohle, wobei die Aktivkohle zunächst mit einer Metallkomponente imprägniert und anschließend mit einer wässrigen alkalischen Lösung behandelt werden soll. Unter Erwärmung soll dann die resultierende Metallhydroxidkomponente zerfallen, um auf diese Weise eine katalytisch aktive Ausrüstung zu erhalten.

Die WO 93/10896 A1 betrifft ein imprägniertes Aktivkohleadsorbens, welches neben Sulfat und Molybdän mindestens ein weiteres Imprägnat auf Basis von Kupfer bzw. Zink aufweist, wobei das Adsorbens zur Entfernung von toxischen Substanzen aus Gasströmen im Rahmen von Filteranwendungen eingesetzt werden soll.

Die WO 2010/094368 A1 betrifft ein textiles Flächenmaterial mit verbesserter mechanischer Stabilität und mit Schutzfunktion gegenüber chemischen bzw. biologischen Giften, wobei das Flächenmaterial einen mehrschichtigen Schichtaufbau aufweist, wobei der Schichtaufbau mindestens eine stich- bzw. schussfeste Schicht und mindestens eine Adsorptionsschicht auf Basis diskreter Adsorbenspartikel aufweist.

Im Lichte der obigen Ausführungen ist daher eine Aufgabe der vorliegenden Erfindung darin zu sehen, ein Verfahren zur Selbstdetoxifizierung bzw. Selbstreinigung von Adsorptionsmaterialien bereitzustellen, wobei die zuvor geschilderten Nachteile des Standes der Technik zumindest weitgehend vermieden oder aber wenigstens abgeschwächt werden sollen.

Insbesondere soll im Rahmen der vorliegenden Erfindung ein Verfahren zur Selbstdetoxifizierung bzw. Selbstreinigung von Adsorbentien bzw. Adsorptionsmaterialien bereitgestellt werden, bei welchem es gewährleistet ist, dass mit chemischen Schadstoffen beladene Adsorbentien bzw. Adsorptionsmaterialien auf Basis von partikulärer Aktivkohle einer effektiven Selbstdetoxifizierung bzw. Selbstdekontamination zugänglich sind, wobei in diesem Zusammenhang insbesondere ein mehrfacher bzw. wiederholter Einsatz der auf Basis des erfindungsgemäßen Verfahrens regenerierten bzw. detoxifizierten Adsorbentien bzw. Adsorptionsmaterialien zu Zwecken der Adsorption bzw. Unschädlichmachung von Schadstoffen ermöglicht werden soll.

Wie die Anmelderin nun in völlig überraschender Weise herausgefunden hat, kann die zuvor angeführte und der vorliegenden Erfindung zugrundeliegende Aufgabe in nicht erwarteter Weise dadurch gelöst werden, dass im Rahmen der vorliegenden Erfindung ein Adsorptionsmaterial in Form einer mit einem inneren Porensystem ausgestatteten partikulären Aktivkohle mit einer speziellen metallbasierten Komponente auf Basis eines Metalloxids *in-situ* ausgerüstet wird, so dass das so behandelte Adsorptionsmaterial aufgrund seiner modifizierten inhärenten Eigenschaften insofern selbsregenerierend ist, als von dem Adsorptionsmaterial adsorbierte Gift- bzw. Schadstoffe zumindest teilweise zu Substanzen bzw. Verbindungen mit geringerem bzw. ohne Gefährdungspotential abgebaut bzw. umgesetzt werden, wie nachfolgend im Detail angeführt.

Zur Lösung der zuvor geschilderten Aufgabe schlägt die vorliegende Erfindung somit - gemäß einem **ersten** Aspekt der vorliegenden Erfindung - ein Verfahren zur Selbstdetoxifizierung bzw. Selbstreinigung eines Adsorptionsmaterials bzw. von Adsorbentien gemäß Anspruch 1 vor. Weitere, insbesondere vorteilhafte Ausgestaltungen des erfindungsgemäßen Verfahrens sind Gegenstand der diesbezüglichen Verfahrensunteransprüche.

Schließlich sind weiterer Gegenstand der vorliegenden Erfindung - gemäß einem **zweiten** Aspekt der vorliegenden Erfindung - Filter und Filtermaterialien zur Entfernung von Schad-, Geruchs- und Giftstoffen aller Art gemäß dem betreffenden unabhängigen Anspruch. Weitere, insbesondere vorteilhafte Ausgestaltungen der erfindungsgemäßen Filter und Filtermaterialien sind Gegenstand der diesbezüglichen Unteransprüche.

Es versteht sich von selbst, dass bei der nachfolgenden Beschreibung der vorliegenden Erfindung solche Ausgestaltungen, Ausführungsformen, Vorteile, Beispiele oder dergleichen, welche nachfolgend - zu Zwecken der Vermeidung unnötiger Wiederholungen - nur zu einem einzelnen Erfindungsaspekt ausgeführt werden, selbstverständlich auch in Bezug auf die übrigen Erfindungsaspekte entsprechend gelten, ohne dass es einer ausdrücklichen Erwähnung bedarf.

Weiterhin versteht es sich von selbst, dass bei nachfolgenden Angaben von Werten, Zahlen und Bereichen die diesbezüglichen Werte-, Zahlen- und Bereichsangaben nicht beschränkend zu verstehen sind; es versteht sich für den Fachmann von selbst, dass einzelfallbedingt oder anwendungsbezogen von den angegebenen Bereichen bzw. Angaben abgewichen werden kann, ohne dass der Rahmen der vorliegenden Erfindung verlassen ist.

Zudem gilt, dass sämtliche im Folgenden genannten Werte- bzw. Parameterangaben oder dergleichen grundsätzlich mit genormten bzw. standardisierten oder explizit angegebenen Bestimmungsverfahren oder aber andernfalls mit dem Fachmann auf diesem Gebiet an sich geläufigen Bestimmungs- bzw. Messmethoden ermittelt bzw. bestimmt werden können.

Im Übrigen gilt, dass bei allen nachstehend aufgeführten relativen bzw. prozentualen, insbesondere gewichtsbezogenen Mengenangaben, zu beachten ist, dass diese Angaben im Rahmen der vorliegenden Erfindung vom Fachmann derart auszuwählen bzw. zu kombinieren sind, dass in der Summe - gegebenenfalls unter Einbeziehung weiterer Komponenten bzw. Inhaltsstoffe, insbesondere wie nachfolgend definiert - stets 100 % bzw. 100 Gew.-% resultieren. Dies versteht sich für den Fachmann aber von selbst.

Dies vorausgeschickt, wird im Folgenden die vorliegende Erfindung näher beschrieben.

Gemäß einem **ersten** Aspekt der vorliegenden Erfindung betrifft die vorliegende Erfindung ein Verfahren zur Selbstdetoxifizierung bzw. Selbstreinigung mindestens eines ein inneres Porensystem aufweisenden Adsorptionsmaterials, wobei das Adsorptionsmaterial aus kugelförmiger Aktivkohle gebildet wird, wobei die Aktivkohle erhalten wird durch Carbonisierung und anschließende Aktivierung von kugelförmigen, sulfonierten organischen Polymeren. Das erfindungsgemäße Verfahren zeichnet sich dadurch aus, dass zunächst das innere Porensystem des Adsorptionsmaterials mit mindestens einer Metallkomponente (synonym auch als metallhaltige Aktivkomponente bezeichnet) ausgerüstet wird, wobei die Metallkomponente bzw. metallhaltige Aktivkomponente in das innere Porensystem eingebracht bzw. eingelagert wird, wobei die Metallkomponente in Form eines Metalloxids in dem inneren Porensystem des Adsorptionsmaterials *in-situ* generiert wird, wobei die Metallkomponente bzw. die metallhaltige Aktivkomponente als das Metalloxid in zumindest partiell kristalliner Form ausgebildet wird und wobei das Adsorptionsmaterial die Metallkomponente in Mengen im Bereich von 0,001 Gew.-% bis 40 Gew.-%, berechnet als Metall und bezogen auf das Gesamtgewicht des mit der Metallkomponente ausgerüsteten Adsorptionsmaterials, aufweist, wobei die Metallkomponente mindestens ein Metall in einer positiven Oxidationsstufe aufweist und wobei die Metallkomponente mindestens ein Metall, ausgewählt aus der Gruppe von Zn, Ag, Sn, Ni und Cu, vorzugsweise Cu, aufweist. Das erfindungsgemäße Verfahren zeichnet sich weiterhin dadurch aus, dass nachfolgend das auf diese Weise ausgerüstete Adsorptionsmaterial mit mindestens einem chemischen Schadstoff in Kontakt gebracht wird, so dass eine zumindest partielle Zersetzung bzw. ein zumindest partieller Abbau des chemischen Schadstoffs erfolgt, wobei der chemische Schadstoff mindestens eine gasförmige und/oder ein Aerosol ausbildende Verbindung umfasst und wobei der chemische Schadstoff ausgewählt wird aus der Gruppe von Abgasen aus Industrieprozessen und Kraftmaschinen oder wobei als chemischer Schadstoff ein chemischer Kampfstoff und/oder dessen Surrogat und/oder Ersatzstoff eingesetzt wird und wobei das Adsorptionsmaterial nach der Ausbildung der Metallkomponente zu dem Metalloxid und/oder während des Inkontaktbringens mit dem chemischen Schadstoff mit Wasser in Kontakt gebracht wird, wobei das Adsorptionsmaterial einen Feuchte- und/oder Wassergehalt im Bereich von 1 Gew.-% bis 60 Gew.-%, bezogen auf das Trockengewicht des mit der Metallkomponente ausgerüsteten Adsorptionsmaterials, aufweist.

Die grundlegende Idee der vorliegenden Erfindung ist somit darin zu sehen, ein spezielles Verfahren bereitzustellen, auf dessen Basis eine Selbstdetoxifizierung bzw. Selbstreinigung und somit eine Regeneration auf Basis der inhärenten Eigenschaften eines in spezieller Weise modifizierten Adsorptionsmaterials auf Basis eines porösen Adsorbens ermöglicht wird. In diesem Zusammenhang fokussiert die vorliegenden Erfindung insbesondere auf die Bereitstellung eines speziellen Adsorptionsmaterials, welches eine adsorptive Funktionalität einerseits und eine reaktive bzw. katalytische Funktionalität andererseits in einem Material vereint, wobei - ohne sich auf diese Theorie beschränken zu wollen - die adsorptive Funktionalität insbesondere durch die Porenstruktur des Adsorptionsmaterials bereitgestellt wird, während die insbesondere die Selbstdetoxifizierung ermöglichende reaktive bzw. katalytische Funktionalität durch die spezielle Ausrüstung des Adsorptionsmaterials mit einer Metallkomponente bzw. einer metallhaltigen Aktivkomponente in Form eines speziellen Metalloxids bereitgestellt wird.

Aufgrund der erfindungsgemäßen Konzeption wird dabei gewährleistet, dass zuvor adsorbierte toxische Substanzen in Form von chemischen bzw. biologischen Schadstoffen durch das Adsorptionsmaterial selbst abgebaut bzw. zersetzt bzw. unschädlich gemacht werden, um auf diese Weise die Selbstdetoxifizierung bzw. Selbstreinigung des im Rahmen des erfindungsgemäßen Verfahrens eingesetzten Adsorptionsmaterials zu gewährleisten. Dabei ergänzen sich die adsorptiven Eigenschaften sowie reaktiven bzw. katalytischen Eigenschaften in über die Wirkung der Einzelmaßnahmen hinausgehender Weise, insbesondere da - gleichermaßen ohne sich auf diese Theorie beschränken zu wollen - aufgrund des zumindest teilweisen Abbaus bzw. der zumindest teilweisen Zersetzung von zuvor adsorbierten chemischen Schadstoffen eine Überführung in weniger bzw. nicht toxische Substanzen vorliegt, welche zudem aufgrund ihrer physikochemischen Eigenschaften, wie Flüchtigkeit bzw. Dampfdruck, desorbiert werden können, was neben einer weiterführenden und dauerhaft gewährleisteten Adsorptionskapazität zu einer effizienten Selbstregenerierbarkeit der zugrundeliegenden Adsorbentien führt.

Insbesondere wird aufgrund des erfindungsgemäßen Verfahrens eine Detoxifizierung bzw. Selbstdetoxifizierung von mit insbesondere beständigen Toxinen, wie chemischen Schadstoffen bzw. CWAs, beladenen Adsorbentien erreicht, insbesondere wobei mittels der Metallkomponente bzw. der metallhaltigen Aktivkomponente die zugrundeliegenden Sorptive (d. h. insbesondere die chemischen Schadstoffe bzw. CWAs) chemisch verändert und zu weniger toxischen bzw. unschädlichen Substanzen abgebaut werden.

In diesem Zusammenhang ist ein zentraler Vorteil der vorliegenden Erfindung darin zu sehen, dass aufgrund des Verfahrens nach der Erfindung mit der Zersetzung bzw. dem zweckgerichteten Abbau von zuvor adsorbierten bzw. aufgenommenen Substanzen auf Basis der zweckgerichteten Bereitstellung einer reaktiven bzw. katalytischen Aktivität des eingesetzten Adsorptionsmaterials aufwendige und kostenintensive Entsorgungsvorgänge von kontaminiertem Adsorptionsmaterial entfallen und auf Basis der erfindungsgemäßen Konzeption insbesondere ein mehrfacher bzw. dauerhafter Einsatz der Adsorptionsmaterialien zu Zwecken der Schadstoffaufnahme möglich ist.

In diesem Zusammenhang entfällt auch eine aufwendige Dekontamination der zugrundeliegenden Adsorptionsmaterialien insbesondere im Hinblick auf den Einsatz von kostenintensiven und mitunter toxischen Dekontaminationslösungen. Gleichermaßen entfällt der Einsatz aufwendiger Salzimprägnierungen, welche zudem zu einer Verringerung der Adsorptionskapazität insbesondere auf Basis eines Verstopfens des Porensystems des zugrundeliegenden Adsorptionsmaterials führen könnten.

Demgegenüber wird im Rahmen der vorliegenden Erfindung aufgrund der vorzugsweise kristallinen Struktur des in dem Porensystem vorliegenden Metalloxids eine hohe Zugänglichkeit des Porensystems für zu adsorbierende Schadstoffe gewährleistet, so dass das im Rahmen des erfindungsgemäßen Verfahrens eingesetzte Adsorptionsmaterial weiterhin über ausgezeichnete Adsorptionseigenschaften verfügt. Zudem wird insbesondere auch durch die erfindungsgemäß vorgesehene *in-situ* Herstellung des Metalloxids in dem Porensystem des Adsorptionsmaterials ausgehend von speziellen und im Folgenden noch näher spezifizierten Vorläuferverbindungen der Metallkomponente eine zumindest im Wesentlichen gleichmäßige Beladung bzw. Ausrüstung mit dem Metalloxid bei definierter Kristall- bzw. Kristallitstruktur über das gesamte Porensystem des Adsorptionsmaterials erreicht, so dass folglich auch das gesamte Porensystem über selbstdetoxifizierende Eigenschaften verfügt.

Aufgrund des Abbaus bzw. der Zersetzung von zuvor absorbierten Schadstoffen wird auf Basis des erfindungsgemäßen Verfahrens in effizienter Weise eine Desorption der in Rede stehenden Schadstoffe mit der Gefahr einer Kreuzkontamination unterbunden, worin ein weiterer zentraler Vorteil der vorliegenden Erfindung zu sehen ist.

Aufgrund der erfindungsgemäßen Konzeption wird ein effizientes und zudem einfaches Verfahren zur Selbstdetoxifizierung bzw. Selbstreinigung von mit chemischenSchadstoffen beladenen Adsorptionsmaterialien bereitgestellt, welches zu einem effektiven Abbau toxischer Substanzen durch das Adsorptionsmaterial selbst mit den einhergehenden selbstregenerativen Eigenschaften führt, was so in keiner Weise vorhersehbar war.

Im Rahmen der vorliegenden Erfindung wird im Ergebnis ein Verfahren unter Verwendung eines optimierten Adsorptionssystems mit kombinierten und sich ergänzenden Eigenschaften von Physisorption und Chemisorption einhergehend mit einer katalytischen bzw. reaktiven Aktivität zur Verfügung gestellt, welches in völlig überraschender Weise zu einer effizienten Selbstregenerierung eines mit Schadstoffen beladenen Adsorptionsmaterials führt, so dass das auf diese Weise gewissermaßen selbstaufgereinigte Adsorptionsmaterial seine Schutzfunktion über einen langen Zeitraum beibehält und somit nicht erschöpft und somit wiederholt bzw. dauerhaft im Rahmen von Adsorptionsprozessen eingesetzt werden kann.

Die Begriffe "Selbstdetoxifizierung" bzw. "Selbstreinigung" bzw. "Selbstregenerierung", wie sie erfindungsgemäß verwendet werden, zielen insbesondere darauf ab, dass das im Rahmen des erfindungsgemäßen Verfahrens eingesetzte Adsorptionsmaterial mit speziellen Eigenschaften ausgestattet wird, welche einen stöchiometrischen bzw. reaktiven und/oder katalytischen Abbau von adsorbierten bzw. von durch das Adsorptionsmaterial aufgenommenen, insbesondere chemischen Schadstoffen, wie militärischen Kampfstoffen, in dem Adsorptionsmaterial selbst ermöglichen, so dass ein Adsorptionsmaterial bereitgestellt wird, welches zur Selbstdekontamination imstande ist. In diesem Zusammenhang werden erfindungsgemäß toxische Ausgangsstoffe zu weniger toxische Substanzen bzw. zu nicht toxische Substanzen abgebaut bzw. zersetzt, was insbesondere mit einer Desorption bzw. Freisetzung der entsprechenden Produkte aus dem Adsorptionsmaterial einhergeht. Gleichermaßen können zuvor mit adsorbierten chemischen Schadstoffen belegte Adsorptionsstellen in dem Porensystem des Adsorptionsmaterials erneut mit Schadstoffen belegt werden, so dass gleichermaßen auch die Adsorptionskapazität verbessert ist. Die erfindungsgemäß bereitgestellte Eigenschaft der Selbstdekontamination wird dabei maßgeblich durch die Ausrüstung des Adsorptionsmaterials mit der Metallkomponente in Form des Metalloxids gewährleistet.

Weiterhin ist der Begriff "zumindest partielle Zersetzung" bzw. "zumindest partieller Abbau", wie er erfindungsgemäß verwendet wird, insbesondere derart zu verstehen, dass im Rahmen des erfindungsgemäßen Verfahrens der in Rede stehende chemische Schadstoff, welcher durch das Adsorptionsmaterial aufgenommen bzw. adsorbiert worden ist, auf Basis von reaktiven bzw. stöchiometrischen und/oder katalytischen Vorgängen unter Anwesenheit der Metallkomponente in Form des Metalloxids abgebaut bzw. unschädlich gemacht wird, insbesondere gefolgt von einer Freisetzung bzw. Desorption der (nicht mehr schädlichen bzw. nicht mehr toxischen) Abbauprodukte, was zu der in Rede stehenden Selbstdetoxifizierung bzw. Selbstreinigung des Adsorptionsmaterials führt. Dabei ist der Begriff "zumindest partielle Zersetzung" derart zu verstehen, dass insbesondere eine zumindest teilweise Zersetzung der in Rede stehenden Schadstoffe erfolgt, wobei der Grad des Abbaus durch die entsprechenden Abbaubedingungen, beispielsweise durch Auswahl der Abbauzeiten und/oder der Umgebungs- bzw. Reaktionsatmosphäre, in der sich das Adsorptionsmaterial befindet, und/oder durch Auswahl der Temperatur und/oder durch Einstellung der Feuchte bzw. des Wassergehalts, weiterführend gesteuert bzw. maßgeschneidert werden kann.

In diesem Zusammenhang können die dem erfindungsgemäßen Verfahren zugrundeliegenden Abbau- bzw. Zersetzungsvorgänge auch derart verstanden werden, dass pro Zeiteinheit ein gewisser Teil bzw. Prozentsatz des von dem Adsorptionsmaterial aufgenommenen Schadstoffs insbesondere infolge der Anwesenheit der Metallkomponente in Form des Metalloxids zersetzt bzw. abgebaut wird, was insbesondere in Bezug auf ein nach Art eines stationären Systems mit einer definierten Menge an Schadstoff einmalig beladenes Adsorptionsmaterial nach einer bestimmten Zeitdauer zu einem zumindest im Wesentlichen vollständigen Abbau des in Rede stehenden Schadstoffs, gegebenenfalls einhergehend mit einer Freisetzung der Abbauprodukte, führt. Gleichermaßen ist der erfindungsgemäß verwendete Begriff, wie zuvor angeführt, in Bezug auf ein nach Art eines dynamischen Systems über einen bestimmten Zeitraum kontinuierlich mit einem Schadstoff beladenes Adsorptionsmaterial derart zu verstehen, dass in einem definierten Zeitraum bzw. pro Zeiteinheit ein bestimmter Anteil bzw. Prozentsatz des Schadstoffs abgebaut bzw. zersetzt wird und die resultierenden Produkte zumindest teilweise freigesetzt bzw. desorbiert werden, so dass über einen bestimmten Zeitraum - quasi ohne Eintritt einer Erschöpfung des Adsorptionsmaterials - kontinuierlich zugeführte Mengen an chemischen bzw. biologischen Schadstoff in entsprechender Weise adsorbiert werden können.

In diesem Zusammenhang wird unter dem Begriff "stationäre Bedingungen" im Rahmen der vorliegenden Erfindung insbesondere ein derartiges Vorgehen bzw. eine solche Verfahrensführung verstanden, bei welcher das mit dem Metalloxid ausgerüstete Adsorptionsmaterial dem in Rede stehenden Schadstoff für einen definierten Zeitraum ausgesetzt wird, wobei das Adsorbens beispielsweise für einen definierten Zeitraum in eine den Schadstoff enthaltende Atmosphäre eingebracht wird, ohne dass weiterer Schadstoff dem System zugeführt wird, so dass gewissermaßen eine einmalige Beladung mit dem Schadstoff vorliegt. Nach bzw. einhergehend mit der Aufnahme bzw. Adsorption des Schadstoffs erfolgt die Zersetzung bzw. der Abbau des Schadstoffs zu weniger toxischen bzw. nicht toxischen Substanzen, wie zuvor beschrieben.

Des Weiteren wird unter dem Begriff "dynamische Bedingungen", wie er im Rahmen der vorliegenden Erfindung verwendet wird, insbesondere eine solche Vorgehensweise bzw. eine solche Verfahrensführung verstanden, bei welcher das mit dem Metalloxid ausgerüstete Adsorptionsmaterial auf Basis eines kontinuierlichen Systems sozusagen dauerhaft bzw. über einen längeren Zeitraum einem kontinuierlich zugeführten Schad Schadstoff ausgesetzt wird, beispielsweise in Form einer kontinuierlichen Durchströmung des Adsorptionsmaterials mit einer den Schadstoff enthaltenden Atmosphäre, wobei in diesem Zusammenhang die zugrundeliegenden Adsorptionsprozesse und der Abbau bzw. die Zersetzung des Schadstoffs sich überlagern bzw. miteinander einhergehen können. Ein Beispiel für eine Verfahrensführung unter dynamischen Bedingungen stellen so genannte Durchbruchuntersuchungen bzw. -verfahren dar.

Bei dem erfindungsgemäß eingesetzten Adsorptionsmaterial handelt es sich aufgrund der zuvor spezifizierten Eigenschaften somit um selbstregenerierende Materialien, welche gleichermaßen als *Self-Detoxifying Materials* (SDM) bezeichnet werden können.

Zusammenfassend wird im Rahmen der vorliegenden Erfindung ein effizientes und einfach durchzuführendes Verfahren zur Selbstdetoxifizierung bzw. Selbstreinigung eines speziellen Adsorptionsmaterials bereitgestellt, welches in Form eines partikulären porösen Adsorbens, nämlich partikulärer Aktivkohle, vorliegt.

Im Rahmen der vorliegenden Erfindung hat es sich dabei als vorteilhaft erwiesen, wenn die Metallkomponente zumindest teilweise in Teilchenform bzw. in partikulärer Form, insbesondere in kristalliner Form, vorzugsweise in Form von Kristalliten, insbesondere in dem Porensystem des Adsorptionsmaterials vorliegt bzw. gebildet wird. Denn, wie die Anmelderin in völlig überraschender Weise herausgefunden hat, die spezielle Ausbildung der Metallkomponente bzw. des Metalloxids in Teilchenform bzw. in kristalliner Form führt zum einen zu einer hohen Effizienz hinsichtlich der Zersetzung bzw. des Abbaus des in Rede stehenden Schadstoffs, insbesondere da - ohne sich auf diese Theorie beschränken zu wollen - eine optimierte Oberflächenstruktur der Metallkomponente bzw. des Metalloxids im Hinblick auf eine Interaktion mit dem Schadstoff vorliegt. Zum anderen wird aufgrund der kristallinen Struktur des Metalloxids eine hohe Zugänglichkeit des Porensystems des zugrundeliegenden Adsorptionsmaterials für zu adsorbierenden Schadstoffe gewährleistet, so dass die Adsorptionskapazität zumindest im Wesentlichen nicht negativ beeinflusst wird. Die im Rahmen des erfindungsgemäßen Verfahrens eingesetzten Adsorptionsmaterialien weisen somit auch nach der Ausrüstung der Metallkomponente in Form des Metalloxids eine hohe Adsorptionskapazität auf.

In diesem Zusammenhang kann es erfindungsgemäß vorgesehen sein, dass die Teilchen und/oder Partikel, insbesondere die Kristallite, der Metallkomponente bzw. des Metalloxids eine mittlere Teilchen- und/oder Partikelgröße, insbesondere eine mittlere Kristallitgröße, im Bereich von 0,1 nm bis 500 nm, insbesondere 1 nm bis 500 nm, vorzugsweise im Bereich von 2 nm bis 400 nm, bevorzugt im Bereich von 5 nm bis 300 nm, besonders bevorzugt im Bereich von 10 nm bis 200 nm, ganz besonders bevorzugt im Bereich von 15 nm bis 150 nm, aufweisen. Insbesondere kann die Größe der Kristallite durch die Beladungsmenge des Adsorptionsmaterials mit der Metallkomponente bzw. durch Auswahl und Menge der nachfolgend noch definierten diesbezüglich eingesetzten Präkursoren bzw. Vorläuferverbindungen und/oder durch die Temperatur bei der Umwandlung der eingesetzten Präkursoren zur Herstellung des Metalloxids in dem Adsorptionsmaterial gesteuert bzw. maßgeschneidert werden, wobei im Allgemeinen höhere Beladungsmengen und höhere Umwandlungstemperaturen zum Erhalt des Metalloxids zu größeren Kristallitgrößen bzw. -durchmessern führen. Zur entsprechenden Größenbestimmung der Teilchen bzw. Kristallite können dem Fachmann in diesem Zusammenhang an sich bekannte Verfahren eingesetzt werden, wie Röntgendiffraktometrie (XRD) sowie Transmissionselektronenmikroskopie (TEM).

Weiterhin ist es erfindungsgemäß bevorzugt, wenn die Metallkomponente bzw. das Metalloxid eine Kristallinität, insbesondere einen Kristallinitätsgrad, (d. h. eine partielle Kristallinität) von mindestens 10 %, insbesondere mindestens 30 %, vorzugsweise mindestens 50 %, bevorzugt mindestens 80 %, besonders bevorzugt mindestens 90 %, ganz besonders bevorzugt mindestens 95 %, bezogen auf die Metallkomponente, aufweist. In diesem Zusammenhang kann die Metallkomponente eine Kristallinität, insbesondere einen Kristallinitätsgrad, im Bereich von 10 % bis 99,5 %, insbesondere im Bereich von 30 % bis 99 %, vorzugsweise im Bereich von 50 % bis 98 %, bevorzugt im Bereich von 80 % bis 95 %, aufweisen. Auf Basis der hohen Kristallinität der Metallkomponente in Form des Metalloxids wird die Effizienz im Hinblick auf die Zersetzung bzw. den Abbau des in Rede stehenden Schadstoffs weiterführend verbessert.

Erfindungsgemäß weist das im Rahmen des erfindungsgemäßen Verfahrens eingesetzte Adsorptionsmaterial, insbesondere das Adsorbens, die Metallkomponente bzw. das Metalloxid in Mengen von mindestens 0,001 Gew.-%, insbesondere mindestens 0,01 Gew.-%, vorzugsweise mindestens 0,5 Gew.-%, bevorzugt mindestens 1 Gew.-%, besonders bevorzugt mindestens 2 Gew.-%, berechnet als Metall und bezogen auf das Gesamtgewicht des mit der Metallkomponente ausgerüsteten Adsorptionsmaterials, insbesondere Adsorbens, auf. In diesem Zusammenhang kann das Adsorptionsmaterial, insbesondere das Adsorbens, die Metallkomponente in Mengen von höchstens 20 Gew.-%, insbesondere höchstens 25 Gew.-%, vorzugsweise höchstens 30 Gew.-%, bevorzugt höchstens 35 Gew.-%, berechnet als Metall und bezogen auf das Gesamtgewicht des mit der Metallkomponente ausgerüsteten Adsorptionsmaterials, insbesondere Adsorbens, aufweisen.

Zudem ist es erfindungsgemäß vorgesehen, dass das Adsorptionsmaterial, insbesondere das Adsorbens, die Metallkomponente bzw. das Metalloxid in Mengen im Bereich von 0,001 Gew.-% bis 40 Gew.-%, insbesondere im Bereich von 0,01 Gew.-% bis 35 Gew.-%, vorzugsweise im Bereich von 0,5 Gew.-% bis 30 Gew.-%, bevorzugt im Bereich von 1 Gew.-% bis 25 Gew.-%, besonders bevorzugt im Bereich von 2 Gew.-% bis 20 Gew.-%, berechnet als Metall und bezogen auf das Gesamtgewicht des mit der Metallkomponente ausgerüsteten Adsorptionsmaterials, insbesondere Adsorbens, aufweist.

Die Menge an Metallkomponente bzw. Metalloxid in dem Adsorptionsmaterial kann beispielsweise auf Basis von Elementaranalysen sowie Röntgendiffraktometrie (XRD) bestimmt werden.

Im Rahmen der vorliegenden Erfindung werden hinsichtlich des Abbaus bzw. der Zersetzung des in Rede stehenden Schadstoffs besonders gute Ergebnisse erhalten, wenn die Metallkomponente das Metall in einer positiven Oxidationsstufe aufweist. In diesem Zusammenhang ist es erfindungsgemäß vorteilhaft, wenn die Oxidationsstufe des Metalls im Bereich von +I bis +VII, insbesondere im Bereich von +I bis +IV, vorzugsweise im Bereich von +I bis +III, liegt und besonders bevorzugt +I oder +II ist.

Erfindungsgemäß ist es vorgesehen, dass die Metallkomponente bzw. das Metalloxid mindestens ein Metall, ausgewählt aus der Gruppe von Zn, Ag, Sn, Ni und Cu, vorzugsweise Cu, aufweist.

Gemäß einer erfindungsgemäß bevorzugten Ausführungsform sollte die Metallkomponente bzw. das Metalloxid mindestens ein Metall, ausgewählt aus der Gruppe von Zn(+II), Ag(+I), Sn(+II), Sn(+IV), Ni(+II), Cu(+I) und Cu(+II), vorzugsweise Cu(+I) und/oder Cu(+II), besonders bevorzugt Cu(+I), aufweisen.

Gemäß einer erfindungsgemäß besonders bevorzugten Ausführungsform kann es vorgesehen sein, dass die Metallkomponente bzw. das Metalloxid in Form eines Kupferoxids vorliegt. In diesem Zusammenhang kann die Metallkomponente in Form von CuO und/oder Cu₂O, insbesondere Cu₂O, vorliegen. Auf Basis der vorgenannten Kupferoxide werden besonders gute Ergebnisse hinsichtlich des Abbaus bzw. der Zersetzung des zugrundeliegenden Schadstoffs erreicht.

Was die Herstellung bzw. Ausbildung des Metalloxids in dem Porensystem des Adsorptionsmaterials anbelangt, so ist es im Rahmen des erfindungsgemäßen Verfahrens vorgesehen, dass die Metallkomponente in Form des Metalloxids in dem inneren Porensystem des Adsorptionsmaterials *in-situ* generiert bzw. ausgebildet wird, insbesondere auf Basis bzw. ausgehend von mindestens einer Vorläuferverbindung und/oder Präkursorverbindung, insbesondere mindestens einem Präkursorsalz, der erfindungsgemäß eingesetzten Metallkomponente. Mit anderen Worten stellt die Präkursor- bzw. Vorläuferverbindung eine chemisch von der resultierenden Metallkomponente bzw. dem Metalloxid verschiedene Verbindung des entsprechenden Metalls dar, wobei es sich diesbezüglich insbesondere um entsprechende Salzverbindungen handeln kann. Die Vorläuferverbindung enthält demnach das Metall des nachfolgend hergestellten Metalloxids.

Aufgrund der erfindungsgemäß vorgesehenen *in-situ*-Generierung des Metalloxids in dem Porensystem des Adsorptionsmaterials wird eine besonders effiziente und gleichmäßige bzw. durchgängige Beladung des Adsorptionsmaterials mit dem Metalloxid über das gesamte Teilchenvolumen des Adsorptionsmaterials bei möglichst homogener Kristallitstruktur und -größenverteilung ermöglicht, was insgesamt zu einer weiteren Effizienzsteigerung des Adsorptionsmaterials im Hinblick auf die Adsorption und die Zersetzung bzw. den Abbau des in Rede stehenden Schadstoffs führt, da durch die gleichmäßige Beladung sowohl eine hohe Zugänglichkeit für den Schadstoff in Bezug auf die adsorptiven Stellen des Adsorptionsmaterials als auch eine weitere Oberflächenoptimierung der Metalloxidkristallite resultiert und zudem das Adsorptionsmaterial zumindest im Wesentlichen über sein gesamtes Porensystem über selbstdetoxifizierende Eigenschaften verfügt.

In diesem Zusammenhang hat es sich als vorteilhaft erwiesen, wenn im Rahmen der vorliegenden Erfindung die Vorläuferverbindung bzw. die Präkursorverbindung, insbesondere das Präkursorsalz, in Form einer vorzugsweise wässrigen Lösung und/oder Dispersion eingesetzt wird. In diesem Zusammenhang kann die Lösung bzw. Dispersion die erfindungsgemäß eingesetzte Vorläuferverbindung bzw. Präkursorverbindung, insbesondere das Präkursorsalz, in Mengen im Bereich von 0,5 Gew.-% bis 80 Gew.-%, insbesondere im Bereich von 1 Gew.-% bis 70 Gew.-%, vorzugsweise im Bereich von 2 Gew.-% bis 60 Gew.-%, bevorzugt im Bereich von 5 Gew.-% bis 50 Gew.-%, bezogen auf die Lösung und/oder Dispersion, aufweisen. Gleichermaßen hat es sich in diesem Zusammenhang als vorteilhaft erwiesen, wenn die wässrige Lösung und/oder Dispersion die Vorläuferverbindung und/oder Präkursorverbindung, insbesondere das Präkursorsalz, in Konzentrationen im Bereich von 0,2 mol/l bis 8 mol/l, insbesondere im Bereich von 0,5 mol/l bis 7 mol/l, vorzugsweise im Bereich von 1 mol/l bis 6 mol/l, bevorzugt im Bereich von 1,5 mol/l bis 5 mol/l, bezogen auf die Lösung und/oder Dispersion, enthält.

In diesem Zusammenhang ist ein weiterer Vorteil der vorliegenden Erfindung darin zu sehen, dass aufgrund des Einsatzes der Präkursorverbindung, insbesondere auf Basis einer wässrigen Lösung bzw. Dispersion, eine optimale Ausrüstung des Adsorptionsmaterials mit der Metallkomponente ermöglicht wird, insbesondere da das Adsorptionsmaterial unter Einsatz der wässrigen Lösung gleichmäßig über das gesamte Porensystem mit der Präkursorverbindung bzw. der Vorläuferverbindung, beispielsweise auf Basis von Tauchmethoden oder dergleichen, getränkt bzw. befüllt wird, was hinsichtlich der nachträglichen Umwandlung der Vorläuferverbindung bzw. des Präkursorsalzes in das Metalloxid von großem Vorteil ist.

Im Rahmen der vorliegenden Erfindung kann es dabei insbesondere vorgesehen sein, dass die Metallkomponente bzw. das Metalloxid in dem inneren Porensystem des Adsorptionsmaterials erhalten wird durch (i) Ausrüstung, insbesondere Tränken und/oder Imprägnieren, des Adsorptionsmaterials mit mindestens einer vorzugsweise wässrigen Lösung bzw. Dispersion einer Vorläuferverbindung bzw. Präkursorverbindung der Metallkomponente, insbesondere mindestens eines Präkursorsalzes, und (ii) nachfolgende Umsetzung bzw. Umwandlung bzw. Zersetzung, insbesondere thermische Umsetzung bzw. thermische Umwandlung bzw. thermische Zersetzung, der Vorläuferverbindung und/oder Präkursorverbindung, insbesondere des Präkursorsalzes, zu dem Metalloxid.

Aufgrund der erfindungsgemäß vorgesehenen *in-situ-*Generierung des Metalloxids in dem inneren Porensystem des Adsorptionsmaterials auf Basis des gezielten Einsatzes und der Umwandlung eines hiervon verschiedenen Vorläufermaterials bzw. einer Präkursorverbindung wird im Ergebnis auch ein höherer Kristallinitätsgrad des resultierenden Metalloxids gewährleistet, da die Kristallbildungsvorgänge ungestört in dem Porensystem selbst ablaufen können und das nachträgliche Einbringen von Metalloxid entfällt, was auch zu einer weiteren Verbesserung der reaktiven bzw. katalytischen Wirksamkeit des Metalloxids führt. Insbesondere wird im Ergebnis eine vollständige Beladung des Adsorptionsmaterials mit dem Metalloxid im Sinne einer Ausrüstung des Porensystems auch in der inneren Kernregion der Teilchens bzw. Partikels ermöglicht.

Erfindungsgemäß kann der Oxidationsgrad des Metalloxids durch die entsprechenden Verfahrensparameter, wie Temperatur, Sauerstoffgehalt und/oder Feuchte- bzw. Wassergehalt der Umgebungsatmosphäre, wie sie bei der Umwandlung der Vorläuferverbindung zu dem Metalloxid vorliegen, gezielt eingestellt werden, was dem Fachmann an sich wohlbekannt ist.

In diesem Zusammenhang kann erfindungsgemäß insbesondere derart vorgegangen werden, dass nach der Ausrüstung, insbesondere nach dem Tränken bzw. Imprägnieren, bzw. vor der Umsetzung bzw. Umwandlung und/oder Zersetzung eine Trocknung des mit der Vorläuferverbindung bzw. Präkursorverbindung, insbesondere dem Präkursorsalz, ausgerüsteten Adsorptionsmaterials durchgeführt wird. Diesbezüglich kann die Trocknung bei Temperaturen im Bereich von 50 °C bis 200 °C, insbesondere im Bereich von 60° C bis 100° C, bzw. für eine Zeitdauer von bis zu 5 h oder weniger durchgeführt werden. Auf dieser Basis kann auch die Restfeuchte des Adsorptionsmaterials gezielt eingestellt werden.

Erfindungsgemäß kann als Vorläuferverbindung und/oder Präkursorverbindung, insbesondere Präkursorsalz, ein anorganisches Salz eingesetzt werden. Insbesondere kann die Vorläuferverbindung bzw. Präkursorverbindung, insbesondere das Präkursorsalz, ausgewählt werden aus der Gruppe von Halogenidsalzen, Sulfaten, Sulfiden, Sulfiten, Nitraten, Nitriten, Nitriden, Phosphaten, Phosphiden, Phosphiten, Carbamaten, Alkoholaten, Carbonaten und Hydrogencarbonaten, insbesondere Nitraten.

Gemäß einer erfindungsgemäß bevorzugten Ausführungsform kann die Vorläuferverbindung bzw. die Präkursorverbindung, insbesondere das Präkursorsalz, in Form eines Nitrats eingesetzt werden. Zudem hat es sich als vorteilhaft erwiesen, wenn als Vorläuferverbindung bzw. Präkursorverbindung, insbesondere Präkursorsalz, ein Kupfersalz, insbesondere ein Kupfer(II)-Salz, vorzugsweise Kupfernitrat, bevorzugt Kupfer(II)-nitrat (Cu(NO₃)₂), eingesetzt wird. Auf Basis der eingesetzten und zuvor spezifizierten Vorläuferverbindungen ist eine besonders effektive Umwandlung zu dem in Rede stehenden Metalloxid insbesondere auf Basis der zuvor angeführten Kupferoxide möglich.

Was die Umwandlung der Vorläuferverbindung bzw. der Präkursorverbindung zu dem Metalloxid weiterhin anbelangt, so kann es erfindungsgemäß vorgesehen sein, dass die Reaktions- bzw. Umsetzungsparameter der Umwandlung der Vorläuferverbindung bzw. Präkursorverbindung zu dem Metalloxid, insbesondere die Temperatur und/oder Atmosphäre, zur Einstellung und/oder Vorgabe der resultierenden Oxidationsstufe, insbesondere Oxidationszahl, des Metalls in dem resultierenden Metalloxid ausgewählt werden. Im Allgemeinen führen bei der Umsetzung höhere Temperaturen zu solchen Metallverbindungen bzw. Metalloxiden, bei denen das Metall in einer niedrigeren Oxidationsstufe vorliegt als es bei entsprechend niedrigeren Temperaturen während der Umsetzung der Fall ist.

Erfindungsgemäß ist es bevorzugt, wenn die Umsetzung bzw. Umwandlung der Vorläuferverbindung bzw. der Präkursorverbindung, insbesondere des Präkursorsalzes, zu dem Metalloxid in einer Inertgasatmosphäre durchgeführt wird. Hierbei kann es sich insbesondere um eine stickstoffhaltige bzw. edelgashaltige Inertgasatmosphäre handeln.

Die Umsetzung der Vorläuferverbindung bzw. Präkursorverbindung zu dem Metalloxid kann beispielsweise in einem beheizbaren Drehrohrofen durchgeführt werden.

Was die Umsetzung bzw. Umwandlung der Vorläuferverbindung bzw. Präkursorverbindung, insbesondere des Präkursorsalzes, zu dem Metalloxid weiterhin anbelangt, so kann diese im Allgemeinen bei Temperaturen im Bereich von 100 °C bis 1.000 °C, insbesondere im Bereich von 150 °C bis 950 °C, vorzugsweise im Bereich von 200 °C bis 920 °C, durchgeführt werden.

Gemäß einer erfindungsgemäßen Ausführungsform kann die Umsetzung bzw. Umwandlung der Vorläuferverbindung bzw. Präkursorverbindung, insbesondere des Präkursorsalzes, zu dem Metalloxid bei Temperaturen bis 400 °C bzw. bei Temperaturen im Bereich von 200 °C bis 400 °C durchgeführt werden. Gemäß dieser Ausführungsform kann als Vorläuferverbindung bzw. Präkursorverbindung, insbesondere Präkursorsalz, ein Kupfernitrat, wie Kupfer(II)-nitrat (Cu(NO₃)₂), eingesetzt werden. Gemäß dieser Verfahrensführung bei relativ niedrigen Umsetzungstemperaturen der Vorläuferverbindung zu dem Metalloxid wird als resultierendes Metalloxid insbesondere mindestens ein Kupferoxid, insbesondere Kupfer(II)-oxid (CuO), erhalten.

Gemäß einer weiteren Ausführungsform der vorliegenden Erfindung kann die Umsetzung bzw. Umwandlung der Vorläuferverbindung bzw. Präkursorverbindung, insbesondere des Präkursorsalzes, zu dem Metalloxid bei Temperaturen von mehr als 400 °C bzw. bei Temperaturen im Bereich von 400 °C bis 920 °C durchgeführt werden. Auch in diesem Fall kann als Vorläuferverbindung bzw. Präkursorverbindung, insbesondere Präkursorsalz, ein Kupfernitrat, insbesondere Kupfer(II)-nitrat (Cu(NO₃)₂), eingesetzt werden. Gemäß dieser Ausführungsform kann als resultierendes Metalloxid mindestens ein Kupferoxid, insbesondere Kupfer(I)-oxid (Cu₂O), erhalten werden.

Auf Basis des erfindungsgemäßen Verfahrens können somit durch die gezielte Auswahl der Umsetztemperatur zur Umwandlung der Vorläuferverbindung zu dem Metalloxid die auf diese Weise erhaltenen Metalloxide hinsichtlich ihrer konkreten Ausbildung und chemischen Natur eingestellt bzw. maßgeschneidert werden, insbesondere was die Oxidationsstufe bzw. die Oxidationszahl des Metalls in dem Metalloxid anbelangt. In diesem Zusammenhang werden bei höheren Temperaturen im Allgemeinen solche Metalloxide erhalten, bei denen das Metall im Vergleich zu einer Verfahrensführung bei niedrigeren Temperaturen eine geringere Oxidationsstufe bzw. eine kleinere Oxidationszahl aufweist. Durch Auswahl der Verfahrensparameter können zudem definierte Verhältnisse, insbesondere definierte Gewichtsverhältnisse, der erhaltenen Metalloxide in dem modifizierten Adsorptionsmaterial eingestellt bzw. maßgeschneidert werden insbesondere derart, dass Metalloxide mit unterschiedlichen Oxidationszahlen des eingesetzten Metalls in einem bestimmten Verhältnis zueinander erhalten werden können, beispielsweise in Form von definierten Kupfer(II)-oxid/Kupfer(I)-oxid-Verhältnissen.

Im Allgemeinen können die Haltedauern der entsprechenden Umwandlungstemperaturen zum Erhalt des Metalloxids in dem Porensystem des Adsorptionsmaterials in weiten Bereichen variieren. Beispielsweise kann die Umwandlungstemperatur für eine Zeitdauer im Bereich von 10 min bis 500 min, insbesondere im Bereich von 30 min bis 400 min, vorzugsweise im Bereich von 50 min bis 300 min, gehalten werden.

Wie zuvor angeführt, kann der eigentlichen Umsetzung der Vorläuferverbindung zu dem Metalloxid ein Trocknungsschritt vorgeschaltet werden, wobei hierzu im Allgemeinen im Vergleich zu der eigentlichen Umsetzung niedrigere Temperaturen realisiert werden. In diesem Zusammenhang kann mit entsprechenden Temperaturprofilen verfahren werden, wobei auch ein unmittelbarer Übergang des Trocknungsschritts in den Umwandlungsschritt der Vorläuferverbindung zu dem Metalloxid durch entsprechende Temperaturerhöhung erfolgen kann.

Im Allgemeinen schließt sich der Umsetzung der Vorläuferverbindung zu dem Metalloxid ein entsprechender Abkühlschritt des auf diese Weise erhaltenen und mit dem Metalloxid ausgerüsteten Adsorptionsmaterials an.

Was darüber hinaus den im Rahmen des erfindungsgemäßen Verfahrens einsetzbaren chemischen Schadstoff im Allgemeinen anbelangt, so umfasst dieser mindestens eine gasförmige bzw. ein Aerosol ausbildende Verbindung.

In diesem Zusammenhang kann es erfindungsgemäß vorgesehen sein, dass der chemische Schadstoff ausgewählt wird aus der Gruppe von Abgasen aus Industrieprozessen und Kraftmaschinen, wie Abgasen aus Verbrennungsmotoren. Diesbezüglich kann der chemische Schadstoff beispielsweise ausgewählt werden aus Halogenwasserstoffen, Stickstoffoxiden sowie Schwefeloxiden. Gleichermaßen ist es im Rahmen der vorliegenden Erfindung auch möglich, dass als chemischer Schadstoff ein chemischer Kampfstoff bzw. dessen Surrogat und/oder Ersatzstoff eingesetzt wird.

Erfindungsgemäß kann es beispielsweise vorgesehen sein, dass als chemischer Schadstoff ein chemischer Kampfstoff bzw. dessen Surrogat und/oder Ersatzstoff eingesetzt wird, wobei der chemische Kampfstoff ausgewählt werden kann aus der Gruppe von Senfgas, Soman, Sarin, Phosgen und Tabun sowie deren Mischungen. Gleichermaßen kann als chemischer Kampfstoff ein Ersatzstoff bzw. Surrogat von Senfgas, Soman, Sarin, Phosgen bzw. Tabun, insbesondere ein Ersatzstoff bzw. Surrogat von Senfgas, eingesetzt werden. Bei dem Ersatzstoff bzw. Surrogat kann es sich beispielsweise um Chloroethylethylsulfid (CEES) handeln.

Bei dem chemischen Kampfstoff Senfgas bzw. HD handelt es sich um einen hochreaktiven, mutagen, karzinogen und zytotoxisch wirkenden Kampfstoff, welcher ein ölartiges, hellgelbes bis dunkelbraunes Erscheinungsbild aufweist. Die chemische Summenformel von Senfgas ist C₄H₈SCl₂. Das entsprechende Surrogat zu dem chemischen Kampfstoff Senfgas stellt das zuvor beschriebene Chloroethylethylsulfid (CEES) mit der Summenformel C₄H₉SCl₂ dar, wobei es sich hierbei um eine farblose bis schwach gelbe Substanz handelt. Im Vergleich zu Senfgas ist CEES weniger toxisch und weist einen höheren Dampfdruck bzw. eine höhere Flüchtigkeit auf. CEES weist im Vergleich zu Senfgas ein ähnliches Adsorptions- bzw. Desorptionsverhalten sowie ein ähnliches Hydrolyseverhalten auf. CEES verfügt über eine chemisch ähnliche Struktur wie Senfgas, wobei ein Chloratom durch eine Methylgruppe ersetzt ist.

Senfgas (HD) sowie CEES weisen insbesondere die folgenden Strukturformen auf:

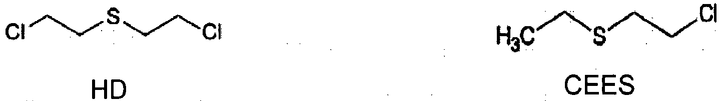

Als weitere Surrogate bzw. Ersatzstoffe können im Rahmen des erfindungsgemäßen Verfahrens Chloroethylmethylsulfid (CEMS), Methylsalicylat (MS), Diethyladipate und Diethylpimelate eingesetzt werden.

Im Rahmen des erfindungsgemäßen Verfahrens wird in Bezug auf die zuvor genannten Kampfstoffe bzw. Surrogate eine hervorragende Selbstdetoxifizierung bzw. Selbstreinigung des im Rahmen des erfindungsgemäßen Verfahrens eingesetzten Adsorptionsmaterials erreicht, so dass diesbezüglich ein hervorragender Schutz in Bezug auf die zuvor genannten chemischen Kampfstoffe im Rahmen des erfindungsgemäßen Verfahrens bzw. den nachfolgend noch angeführten Verwendungen erreicht wird.

Im Rahmen der vorliegenden Erfindung kann es insbesondere vorgesehen sein, dass das Inkontaktbringen des Adsorptionsmaterials mit dem chemischen Schadstoff derart erfolgt, dass der chemische Schadstoff zumindest teilweise in das innere Porensystem des Adsorptionsmaterials eindringt bzw. dass der chemische Schadstoff zumindest teilweise in das innere Porensystem des Adsorptionsmaterials einzudringen imstande ist. Dies kann zum Beispiel dadurch erfolgen, dass das mit dem Metalloxid ausgerüstete Adsorptionsmaterial einer Atmosphäre ausgesetzt wird, welche den in Rede stehenden Schadstoff enthält. Insbesondere kann die Beladung bzw. das Inkontaktbringen des Adsorptionsmaterials mit dem Schadstoff im Rahmen einer Verwendung bzw. eines Einsatzes des Adsorptionsmaterials erfolgen, beispielsweise wenn dieses im Rahmen von Gasfiltern oder Schutzanzügen oder dergleichen verwendet wird oder im Einsatz ist.

Beispielsweise kann im Rahmen des erfindungsgemäßen Verfahrens das Inkontaktbringen des Adsorptionsmaterials mit dem chemischen Schadstoff bzw. die Zersetzung bzw. der Abbau des chemischen Schadstoffs unter stationären oder dynamischen Bedingungen erfolgen.

In diesem Zusammenhang kann die Selbstdetoxifizierung bzw. Selbstreinigung des Adsorptionsmaterials unter dynamischen Bedingungen erfolgen insofern, als die Zersetzung bzw. der Abbau des chemischen Schadstoffs und/oder die Selbstdetoxifizierung bzw. Selbstreinigung des Adsorptionsmaterials insbesondere unter Anwesenheit von weiterem, in der das Adsorptionsmaterial umgebenden Atmosphäre enthaltenem (d. h. ungebundenem bzw. nicht adsorbiertem) Schadstoff durchgeführt werden kann bzw. erfolgen kann.

Gleichermaßen kann die Selbstdetoxifizierung bzw. Selbstreinigung des Adsorptionsmaterials aber auch unter stationären Bedingungen erfolgen insofern, als die Zersetzung bzw. der Abbau des chemischen Schadstoffs und/oder die Selbstdetoxifizierung bzw. Selbstreinigung des zuvor kontaminierten bzw. des zuvor mit dem Schadstoff in Kontakt gebrachten Adsorptionsmaterials insbesondere unter solchen Bedingungen erfolgen kann, wonach die das Adsorptionsmaterial umgebende Atmosphäre zumindest im Wesentlichen frei von weiterem ungebundenem bzw. nicht adsorbiertem Schadstoff ist. Diesbezüglich liegt insbesondere ein Prozess mit einem Abbau bzw. einer Zersetzung des Schadstoffs und einer nachfolgenden Desorption bzw. Freisetzung der Abbauprodukte vor.

Erfindungsgemäß können die vorgenannten Prozesse bzw. Mechanismen (d. h. dynamische und stationäre Selbstdetoxifizierung bzw. Selbstregeneration) aber auch ineinander übergehen bzw. sich aneinander anschließen.

Im Rahmen des erfindungsgemäßen Verfahrens kann der chemische Schadstoff einer Atmosphäre, insbesondere einer Reaktionsatmosphäre, zugesetzt werden bzw. in einer solchen Atmosphäre enthalten sein. In diesen Zusammenhang kann das Adsorptionsmaterial mit der den chemischen Schadstoff enthaltenden Atmosphäre in Kontakt gebracht werden. Diesbezüglich kann die Atmosphäre den chemischen Schadstoff in Mengen im Bereich von 0,1 g/m³ bis 20 g/m³, insbesondere im Bereich von 0,5 g/m³ bis 10 g/m³, vorzugsweise im Bereich von 0,75 g/m³ bis 5 g/m³, enthalten. Hierbei kann es sich beispielsweise um eine Atmosphäre unter Einsatzbedingungen des Adsorptionsmaterials handeln, beispielsweise wenn dieses im Rahmen von Gasfiltern oder Schutzanzügen verwendet wird.

Erfindungsgemäß kann es vorgesehen sein, dass der chemische Schadstoff insbesondere in dem inneren Porensystem des Adsorptionsmaterials zumindest teilweise gebunden wird. Die Bindung des Schadstoffs an das Adsorptionsmaterial kann dabei beispielsweise auf Basis von Sorption, vorzugsweise Adsorption, insbesondere auf Basis von Physisorption und/oder Chemisorption, erfolgen. Insbesondere kann die Bindung als solche durch Physisorption erfolgen. In diesem Zusammenhang kann das Adsorptionsmaterial den chemischen Schadstoff in Mengen im Bereich von 0,01 Gew.-% bis 80 Gew.-%, insbesondere im Bereich von 0,05 Gew.-% bis 60 Gew.-%, vorzugsweise im Bereich von 0,1 Gew.-% bis 40 Gew.-%, bevorzugt im Bereich von 1 Gew.-% bis 30 Gew.-%, bezogen auf das Gesamtgewicht des mit der Metallkomponente ausgerüsteten Adsorptionsmaterials, aufweisen und/oder adsorbieren bzw. binden.

Wie zuvor angeführt, kann es im Rahmen des erfindungsgemäßen Verfahrens vorgesehen sein, dass der chemische Schadstoff insbesondere in dem inneren Porensystem des Adsorptionsmaterial zumindest teilweise abgebaut bzw. zersetzt bzw. umgesetzt wird. In diesem Zusammenhang kann der Abbau bzw. die Zersetzung unter Molekülspaltung, vorzugsweise unter Dehalogenierung bzw. Dehydrohalogenierung bzw. Hydrolyse, des chemischen Schadstoffs erfolgen. Gleichermaßen kann der Abbau bzw. die Zersetzung des in Rede stehenden Schadstoffs auf Basis einer stöchiometrischen bzw. reaktiven Um- bzw. Zersetzung bzw. einer katalytischen Um- bzw. Zersetzung bzw. auf Basis von Oxidation erfolgen. Durch die zugrundeliegenden Zersetzungs- bzw. Abbauprozesse resultieren dabei ausgehend von den in Rede stehenden Schadstoffen weniger toxische bzw. nicht toxische Substanzen, welche im Allgemeinen eine höhere Flüchtigkeit bzw. einen höheren Dampfdruck als die zugrundeliegenden Ausgangssubstanzen aufweisen und im Allgemeinen desorbiert werden bzw. aus dem Adsorptionsmaterial freigesetzt werden können.

Der durch das Adsorptionsmaterial aufgenommene Schadstoff kann somit insbesondere aufgrund stöchiometrischer bzw. reaktiver bzw. katalytischer Prozesse zu Produkten umgewandelt bzw. abgebaut werden, welche eine geringere bzw. keine Toxizität aufweisen. Das Metalloxid in dem Adsorptionsmaterial kann dabei sozusagen als reaktive bzw. katalytische Komponente fungieren.

Beispielsweise kann die als Surrogat für den chemischen Kampfstoff Senfgas eingesetzte Verbindung CEES gemäß dem nachfolgenden Schema unter Einwirkung des Metalloxids zu den angeführten Produkten abgebaut bzw. zersetzt werden:

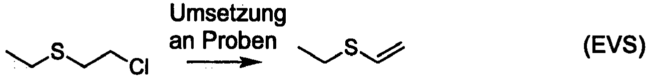

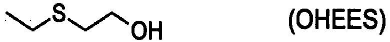

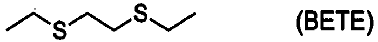

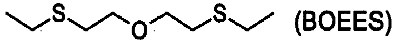

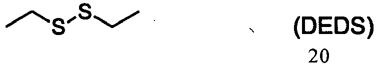

In der vorgenannten Abbildung bedeuten die Abkürzungen EVS = Ethylenvinylsulfid, OHEES = Hydroxyethylethylsulfid, BETE = Bisethylthioethan, BOEES = Bisoxaethylethylsulfid und DEDS = Diethyldisulfid. Die entsprechenden Reaktionsprodukte weisen dabei im Gegensatz zu CEES keine Chlorethylsulfidgruppe auf und sind daher weniger toxisch als die zugrundeliegende Ausgangssubstanz CEES.

Insbesondere können im Rahmen der erfindungsgemäß vorliegenden Abbauprozesse die nachfolgenden Reaktionen bzw. Umsetzungen insbesondere von CEES vorliegen:

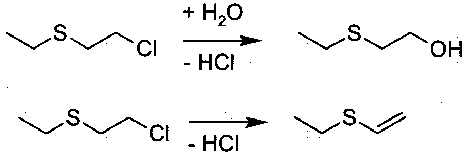

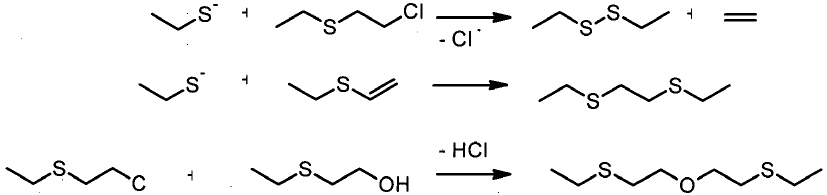

Erfindungsgemäß kann es in diesem Zusammenhang vorgesehen sein, dass - wie zuvor angeführt - die Abbau- bzw. Zersetzungsprodukte des chemischen Schadstoffs zumindest teilweise desorbiert bzw. in die das Adsorptionsmaterial umgebende Atmosphäre abgeben bzw. freigesetzt werden. Hierbei können zuvor durch den chemischen Schadstoff belegte Adsorptionsstellen bzw. Anbindungsstellen des Adsorptionsmaterials zur Anbindung bzw. Adsorption von weiterem Schadstoff freigegeben werden, so dass erfindungsgemäß sozusagen ein kontinuierlicher Prozess vorliegen kann, welcher die Adsorption des in Rede stehenden Schadstoffs sowie die entsprechende Zersetzung unter Einwirkung des Metalloxids und die nachfolgende Freigabe bzw. Desorption der resultierenden Produkte aus der Aktivkohle und die sich anschließende Neubesetzung der Adsorptionsstellen durch weiteren Schadstoff umfasst. Dies gilt insbesondere für eine Verfahrensführung unter dynamischen Bedingungen.

Insbesondere kann das Inkontaktbringen des Adsorptionsmaterial mit dem chemischen Schadstoff und/oder die Zersetzung bzw. der Abbau des chemischen Schadstoffs bei Temperaturen im Bereich von - 20 °C bis 100 °C, insbesondere im Bereich von 5 °C bis 60 °C, vorzugsweise im Bereich von 10 °C bis 40 °C, durchgeführt werden. Gleichermaßen kann das Inkontaktbringen des Adsorptionsmaterials mit dem chemischen Schadstoff und/oder die Zersetzung bzw. der Abbau des chemischen Schadstoffs für eine Zeitdauer im Bereich von 1 min bis 5.000 min, insbesondere im Bereich von 10 min bis 4.000 min, vorzugsweise im Bereich von 60 min bis 2.500 min, bevorzugt im Bereich von 100 min bis 2.000 min, durchgeführt werden. Dabei kann insbesondere unter Normaldruck (d. h. 1013 mbar) verfahren werden. Im einfachsten Fall kann die Selbstdetoxifizierung des Adsorptionsmaterials beispielsweise bei der Lagerung bzw. Aufbewahrung des Adsorptionsmaterials, beispielsweise auf Basis der bzw. in Form der entsprechenden Verwendungsform, beispielsweise eines Schutzanzugs oder Gasfilters, und/oder unmittelbar unter Einsatzbedingungen bzw. im Einsatz des Adsorptionsmaterials, insbesondere auf Basis der entsprechenden Verwendungsform, erfolgen.

Erfindungsgemäß ist es zudem vorgesehen, dass das Adsorptionsmaterial nach der Ausbildung der Metallkomponente zu dem Metalloxid bzw. während des Inkontaktbringens mit dem chemischen Schadstoff mit Wasser in Kontakt gebracht wird, insbesondere mit Wasser ausgerüstet bzw. beladen wird. Hierzu kann das Wasser in Form von Wasserdampf eingesetzt werden. Gleichermaßen kann das Wasser, insbesondere in Form von Wasserdampf, der das Adsorptionsmaterial umgebenden Atmosphäre, insbesondere der den chemischen Schadstoff enthaltenden Reaktionsatmosphäre, zugegeben werden. Gleichermaßen kann es erfindungsgemäß aber auch vorgesehen sein, dass eine Ausrüstung der Aktivkohle mit Wasser durch bereits in der (Umgebungs-) Atmosphäre vorhandenes Wasser durchgeführt wird bzw. erfolgt.

Denn die Anmelderin hat in diesem Zusammenhang in völlig überraschender Weise herausgefunden, dass die dem erfindungsgemäßen Verfahren zugrundeliegenden Abbau- bzw. Zersetzungsprozesse des Schadstoffs zu den entsprechenden Produkten unter Anwesenheit von Wasser, insbesondere in dem Porensystem der Aktivkohle, beschleunigt bzw. verbessert wird. Mit steigender Feuchtigkeit bzw. steigendem Wassergehalt in dem Adsorptionsmaterial wird somit der Abbau des zugrundeliegenden Schadstoffs erhöht bzw. beschleunigt, insbesondere durch eine Erhöhung der zugrundeliegenden Reaktionsgeschwindigkeitskonstanten.

In diesem Zusammenhang hat es sich als vorteilhaft erwiesen, wenn die Atmosphäre, welche das Adsorptionsmaterial umgibt, eine relative Luftfeuchtigkeit ("rF") von mindestens 1 %, insbesondere mindestens 5 %, vorzugsweise mindestens 10 %, bevorzugt mindestens 20 %, besonders bevorzugt mindestens 30 %, ganz besonders bevorzugt mindestens 40 %, aufweist. Insbesondere kann die Atmosphäre eine relative Luftfeuchtigkeit im Bereich von 1 % bis 100 %, insbesondere im Bereich von 5 % bis 99 %, vorzugsweise im Bereich von 10 % bis 95 %, bevorzugt im Bereich von 20 % bis 95 %, besonders bevorzugt im Bereich von 30 % bis 90 %, ganz besonders bevorzugt im Bereich von 40 % bis 80 %, aufweisen.

Die zuvor angeführten Werte für die relative Luftfeuchtigkeit bzw. relative Feuchte beziehen sich insbesondere auf die Atmosphäre unter Einsatz- bzw. Lagerbedingungen, insbesondere Regenerationsbedingungen, des im Rahmen des erfindungsgemäßen Verfahrens eingesetzten Adsorptionsmaterials.

Was das Adsorptionsmaterial als solches anbelangt, so ist es vorgesehen, dass dieses einen Feuchte- bzw. Wassergehalt im Bereich von 1 Gew.-% bis 60 Gew.-%, insbesondere im Bereich von 2 Gew.-% bis 55 Gew.-%, vorzugsweise im Bereich von 3 Gew.-% bis 50 Gew.-%, bevorzugt im Bereich von 5 Gew.-% bis 50 Gew.-%, bezogen auf das Trockengewicht des mit der Metallkomponente ausgerüsteten Adsorptionsmaterials, aufweist. Die entsprechenden Feuchte- bzw. Wassergehalte können in dem Fachmann an sich bekannter Weise (beispielsweise durch gravimetrische, insbesondere thermogravimetrische Differenzbestimmung der modifizierten Adsorbentien oder auf Basis der zugrundeliegenden Wasserdampfsorptionsisothermen der modifizierten Adsorbentien) ermittelt werden.

Das Adsorptionsmaterial wird aus kugelförmiger Aktivkohle gebildet.

Die jeweiligen und zuvor spezifizierten Adsorptionsmaterialien sind dem Fachmann als solche wohlbekannt und der Fachmann ist jederzeit in der Lage, die jeweiligen partikelbildenden Materialien im Lichte des erfindungsgemäßen Verfahrens auszuwählen und aufeinander abzustimmen. Erfindungsgemäß verwendbare Aktivkohlen, welche insbesondere auf Basis kugelförmiger Aktivkohle eingesetzt werden können, sind beispielsweise von der Blücher GmbH, Erkrath, Deutschland, oder von der Adsor-Tech GmbH, Premnitz, Deutschland, erhältlich. Zudem kann in Bezug auf die erfindungsgemäß einsetzbare Aktivkohle verwiesen werden auf die auf die Anmelderin selbst zurückgehende europäische Patentanmeldung EP 1 918 022 A1 sowie auf die parallele US 2008/0107589 A1 deren jeweilige Offenbarung hiermit durch Bezugnahme vollumfänglich eingeschlossen ist.

Die eingesetzte Aktivkohle, insbesondere Kornkohle oder Kugelkohle, ist somit nach bekannten Verfahren des Standes der Technik erhältlich: Erfindungsgemäß werden zu diesem Zweck korn- bzw. kugelförmige, sulfonierte organische Polymere, insbesondere auf Basis von divinylbenzolvernetztem Polystyrol, carbonisiert und anschließend zu der betreffenden Aktivkohle aktiviert. Für weitergehende diesbezügliche Einzelheiten kann beispielsweise auch auf die Druckschriften DE 43 28 219 A1, DE 43 04 026 A1 sowie DE 196 00 237 verwiesen werden, deren jeweilige Offenbarung hiermit durch Bezugnahme vollumfänglich eingeschlossen ist. Bei dem erfindungsgemäß eingesetzten Adsorptionsmaterial kann es sich insbesondere um eine polymerbasierte sphärische Aktivkohle (PBSAC) handeln.

Die nachfolgend aufgeführten Parameterangaben bezüglich der eingesetzten Adsorptionsmaterialien werden mit genormten oder explizit angegebenen Bestimmungsverfahren oder mit dem Fachmann an sich geläufigen Bestimmungsmethoden bestimmt. Insbesondere die Parameterangaben betreffend die Charakterisierung der Porosität, der Porengrößenverteilung und anderer Adsorptionseigenschaften ergeben sich im Allgemeinen jeweils aus den betreffenden Stickstoffsorptionsisothermen der betreffenden Adsorptionsmaterialien bzw. der vermessenen Produkte.

Das erfindungsgemäß eingesetzte Adsorptionsmaterial kann Teilchengrößen, insbesondere Teilchendurchmesser, im Bereich von 0,001 bis 3 mm, insbesondere im Bereich von 0,005 bis 2,5 mm, vorzugsweise im Bereich von 0,01 bis 2 mm, besonders bevorzugt im Bereich von 0,02 bis 1,5 mm, ganz besonders bevorzugt im Bereich von 0,05 bis 1 mm, aufweisen. Zudem kann das Adsorptionsmaterial mittlere Teilchengrößen, insbesondere mittlere Teilchendurchmesser (D50), im Bereich von 0,01 bis 2 mm, insbesondere im Bereich von 0,05 bis 1,5 mm, vorzugsweise im Bereich von 0,1 bis 1 mm, aufweisen.

Die entsprechenden Teilchengrößen bzw. -durchmesser können beispielsweise auf Basis der Methode nach ASTM D2862-97/04 bestimmt werden. Zudem können die vorgenannten Größen mit Bestimmungsmethoden auf Basis einer Siebanalyse, auf Basis von Röntgenbeugung, Laserdiffraktometrie oder dergleichen bestimmt werden. Die jeweiligen Bestimmungsmethoden sind dem Fachmann als solche wohlbekannt, so dass es diesbezüglich keiner weiteren Ausführungen bedarf.

Das erfindungsgemäß eingesetzte Adsorptionsmaterial kann eine spezifische Oberfläche (BET-Oberfläche) von mindestens 500 m²/g, insbesondere mindestens 750 m²/g, vorzugsweise mindestens 1.000 m²/g, besonders bevorzugt mindestens 1.200 m²/g, aufweisen. Zudem kann das Adsorptionsmaterial eine spezifische Oberfläche (BET-Oberfläche) im Bereich von 500 bis 4.000 m²/g, insbesondere im Bereich von 750 bis 3.000 m²/g, vorzugsweise im Bereich von 900 bis 2.500 m²/g, besonders bevorzugt im Bereich von 950 bis 2.000 m²/g, aufweisen.

Die Bestimmung der spezifischen Oberfläche gemäß BET ist dem Fachmann grundsätzlich als solche bekannt, so dass diesbezüglich keine weitergehenden Einzelheiten ausgeführt zu werden brauchen. Sämtliche BET-Oberflächenangaben beziehen sich auf die Bestimmung gemäß ASTM D6556-04. Im Rahmen der vorliegenden Erfindung wird zur Bestimmung der BET-Oberfläche - im Allgemeinen und sofern nachfolgend nicht ausdrücklich abweichend angegeben - die so genannte MultiPoint-BET-Bestimmungsmethode (MP-BET) in einem Partialdruckbereich p/p₀ von 0,05 bis 0,1 angewendet.

In Bezug auf weitergehende Einzelheiten zu der Bestimmung der BET-Oberfläche bzw. zu der BET-Methode kann auf die vorgenannte ASTM D6556-04 sowie auf Römpp Chemielexikon, 10. Auflage, Georg Thieme Verlag, Stuttgart/New York, Stichwort: "BET-Methode", einschließlich der dort referierten Literatur, und auf Winnacker-Küchler (3. Auflage), Band 7, Seiten 93 ff. sowie auf Z. Anal. Chem. 238, Seiten 187 bis 193 (1968), verwiesen werden.

Zudem ist es von Vorteil, wenn das Adsorptionsmaterial einen Berstdruck von mindestens 5 Newton, insbesondere einen Berstdruck im Bereich von 5 Newton bis 50 Newton, pro Teilchen und/oder Partikel aufweist.

Zudem kann das Adsorptionsmaterial ein Adsorptionsvolumen V_{ads} von mindestens 250 cm³/g, insbesondere mindestens 300 cm³/g, vorzugsweise mindestens 350 cm³/g, besonders bevorzugt mindestens 400 cm³/g, aufweisen. Zudem kann das Adsorptionsmaterial ein Adsorptionsvolumen V_{ads} im Bereich von 250 bis 3.000 cm³/g, insbesondere im Bereich von 300 bis 2.000 cm³/g, vorzugsweise im Bereich von 350 bis 2.500 cm³/g, aufweisen.

Bei dem Adsorptionsvolumen V_{ads} handelt es sich um eine dem Fachmann wohlbekannte Größe zur Charakterisierung der eingesetzten partikulären Adsorptionsmaterialien. Auch die diesbezüglichen Bestimmungsmethoden sind dem Fachmann an sich wohlbekannt. Insbesondere handelt es sich bei dem Adsorptionsvolumen V_{ads} um das gewichtsbezogene adsorbierte N₂-Volumen, welches im Allgemeinen bei einem Partialdruck p/p₀ von 0,995 bestimmt wird.

Das erfindungsgemäß eingesetzte Adsorptionsmaterial kann ein Gesamtporenvolumen nach Gurvich von mindestens 0,50 cm³/g, insbesondere mindestens 0,55 cm³/g, vorzugsweise mindestens 0,60 cm³/g, besonders bevorzugt mindestens 0,65 cm³/g, ganz besonders bevorzugt mindestens 0,70 cm³/g, aufweisen. Zudem kann das Adsorptionsmaterial ein Gesamtporenvolumen nach Gurvich im Bereich von 0,50 bis 2,0 cm³/g, insbesondere im Bereich von 0,55 bis 1,5 cm³/g, vorzugsweise im Bereich von 0,60 bis 1,2 cm³/g, besonders bevorzugt im Bereich von 0,65 bis 1,0 cm³/g, aufweisen.

Was die Bestimmung des Gesamtporenvolumens nach Gurvich anbelangt, so handelt es sich um eine dem Fachmann auf diesem Gebiet an sich bekannte Mess-Bestimmungsmethode. Zu weitergehenden Einzelheiten bezüglich der Bestimmung des Gesamtporenvolumens nach Gurvich kann beispielsweise verwiesen werden auf L. Gurvich (1915), J. Phys. Chem. Soc. Russ. 47, 805, sowie auf S. Lowell et al., Characterization of Porous Solids and Powders: Surface Area Pore Size and Density, Kluwer Academic Publishers, Article Technology Series, Seiten 111 ff.

Weiterhin kann es gemäß der erfindungsgemäßen Konzeption vorgesehen sein, dass hinsichtlich des eingesetzten partikulären Adsorptionsmaterials die diesbezüglichen Gesamtporenvolumina nach Gurvich gezielt eingestellt bzw. variiert werden.

Gleichermaßen kann es erfindungsgemäß vorgesehen sein, dass das Adsorptionsmaterial eine Gesamtporosität im Bereich von 10 % bis 80 %, insbesondere im Bereich von 20 % bis 75 %, vorzugsweise im Bereich von 25 % bis 70 %, bezogen auf das Teilchenvolumen des Adsorptionsmaterials, aufweist.

Erfindungsgemäß ergeben sich zudem unter Verwendung von Aktivkohle mit hoher Makro- und Mesoporosität besonders gute Eigenschaften hinsichtlich der Selbstdetoxifizierung.

Insbesondere kann es im Rahmen der vorliegenden Erfindung vorgesehen sein, dass das Adsorptionsmaterial ein durch Poren mit Porendurchmessern ≤ 20 Å gebildetes Porenvolumen, insbesondere Mikroporenvolumen nach Carbon Black, im Bereich von 0,1 bis 2 cm³/g, insbesondere 0,2 bis 1,5 cm³/g, vorzugsweise im Bereich von 0,3 bis 1,1 cm³/g, besonders bevorzugt im Bereich von 0,4 bis 1 cm³/g, aufweist und/oder dass 10 % bis 80 %, insbesondere 20 % bis 75 %, vorzugsweise 30 % bis 70 %, des Gesamtporenvolumens des Adsorptionsmaterials durch Poren mit Porendurchmessern von ≤ 20 Å, insbesondere durch Mikroporen, gebildet werden.

Die Bestimmungsmethode nach Carbon Black ist dem Fachmann an sich bekannt, so dass es diesbezüglich keiner weitergehenden Einzelheiten bedarf. Zudem kann für weitergehenden Einzelheiten der Bestimmung der Porenoberfläche und des Porenvolumens nach Carbon Black beispielsweise verwiesen werden auf R. W. Magee, Evaluation of the External Surface Area of Carbon Black by Nitrogen Adsorption, Presented at the Meeting of the Rubber Division of the American Chem. Soc., October 1994, z. B. referiert in: Quantachrome Instruments, AUTOSORB-1, AS1 WinVersion 1.50, Operating Manual, OM, 05061, Quantachrome Instruments 2004, Florida, USA, Seiten 71 ff.

Gemäß einer weiteren erfindungsgemäßen Ausführungsform kann es vorgesehen sein, dass das Adsorptionsmaterial ein spezifisches Gesamtporenvolumen im Bereich von 0,01 bis 4,0 cm³/g, insbesondere im Bereich von 0,1 bis 3,0 cm³/g, vorzugsweise im Bereich von 0,2 bis 2,0 cm³/g, aufweist. Diesbezüglich kann der Anteil an Poren mit Porendurchmessern ≤ 75 Å, mindestens 65 %, insbesondere mindestens 70 %, vorzugsweise mindestens 75 %, betragen. Zudem kann das Adsorptionsmaterial, bezogen auf das Gesamtporenvolumen, insbesondere auf das Gesamtporenvolumen nach Gurvich, einen Anteil an Mikroporen, insbesondere an Mikroporen mit Porendurchmessern von s 30 Å, insbesondere ≤ 25 Å, vorzugsweise ≤ 20 Å, von mindestens 30 %, insbesondere mindestens 35 %, vorzugsweise mindestens 45 %, und/oder einen Anteil an Mikroporen, insbesondere an Mikroporen mit Porendurchmessern von ≤ 30 Å, insbesondere ≤ 25 Å, vorzugsweise ≤ 20 Å, im Bereich von 30 % bis 90 %, insbesondere im Bereich von 35 % bis 85 %, vorzugsweise im Bereich von 45 % bis 80 %, aufweisen.

Das im Rahmen des erfindungsgemäßen Verfahrens zur Selbstdetoxifizierung bzw. Selbstreinigung eingesetzte Adsorptionsmaterial kann durch ein Verfahren zur Herstellung des ein inneres Porensystem aufweisenden Adsorptionsmaterials mit selbstdetoxifizierenden und/oder selbstreinigenden Eigenschaften erhalten werden. Das Verfahren zeichnet sich dadurch aus, dass das innere Porensystem des Adsorptionsmaterials mit mindestens einer Metallkomponente ausgerüstet wird, insbesondere mindestens eine Metallkomponente in das innere Porensystem eingebracht bzw. eingelagert wird, wobei die Metallkomponente als Metalloxid in zumindest teilweise und/oder partiell kristalliner Form ausgebildet wird.

Bezüglich weiterer Ausführungen zu dem Verfahren zur Herstellung des erfindungsgemäß eingesetzten Adsorptionsmaterials kann auf die diesbezüglich relevanten Ausführungen zu dem erfindungsgemäßen Verfahren gemäß dem ersten Aspekt der vorliegenden Erfindung sowie auf die nachfolgenden Ausführungen zu den weiteren erfindungsgemäßen Aspekten verwiesen werden, welche in Bezug auf das vorliegende Verfahren entsprechend gelten.

Im Rahmen der vorliegenden Erfindung ist ein selbstdetoxifizierendes bzw. selbstreinigendes Adsorptionsmaterial mit einem inneren Porensystem, insbesondere selbstregenerierbares partikuläres poröses Adsorbens durch ein Verfahren gemäß dem ersten Aspekt der vorliegenden Erfindung bzw. durch ein vorgenanntes Verfahren erhältlich. Diesbezüglich kann auf obige Ausführungen verwiesen werden.

Das innere Porensystem des selbstdetoxifizierenden bzw. selbstreinigenden Adsorptionsmaterials als solchen, insbesondere des selbstregenerierbaren partikulären porösen Adsorbens, ist in diesem Zusammenhang mit mindestens einer Metallkomponente ausgerüstet, wobei die Metallkomponente in das innere Porensystem eingebracht bzw. eingelagert ist, wobei die Metallkomponente als Metalloxid, vorzugsweise in zumindest partiell kristalliner Form, ausgebildet ist.

In diesem Zusammenhang ist es vorgesehen, dass das Adsorptionsmaterial einen Feuchte- bzw. Wassergehalt im Bereich von 1 Gew.-% bis 60 Gew.-%, insbesondere im Bereich von 2 Gew.-% bis 55 Gew.-%, vorzugsweise im Bereich von 3 Gew.-% bis 50 Gew.-%, bevorzugt im Bereich von 5 Gew.-% bis 50 Gew.-%, bezogen auf das Trockengewicht des mit der Metallkomponente ausgerüsteten Adsorptionsmaterials, aufweist. Denn die Anmelderin hat in völlig überraschender Weise herausgefunden, dass durch die Gegenwart von Wasser bzw. Wasserdampf der Abbau der in Rede stehenden Schadstoffe signifikant beschleunigt werden kann. Diesbezüglich kann auf obige Ausführungen verwiesen werden.

Bezüglich weiterer Ausführungen zu dem Adsorptionsmaterial kann auf die entsprechenden Ausführungen zu weiteren Aspekten der vorliegenden Erfindung verwiesen werden, welche in Bezug auf das Adsorptionsmaterial entsprechend gelten.

Das Adsorptionsmaterial, wie zuvor definiert, kann zur Herstellung von Schutzmaterialien aller Art, insbesondere von Schutzbekleidung, insbesondere für den zivilen oder militärischen Bereich, wie Schutzanzügen, Schutzhandschuhen, Schutzschuhwerk, Schutzsocken, Kopfschutzbekleidung und dergleichen, und von Schutzabdeckungen aller Art, vorzugsweise alle vorgenannten Schutzmaterialien für den ABC-Einsatz, verwendet werden.

Weiterhin eignet sich das Adsorptionsmaterial, wie zuvor definiert, zur Herstellung von Filtern und Filtermaterialien aller Art, insbesondere zur Entfernung von Schad-, Geruchs- und Giftstoffen aller Art, insbesondere aus Luft- und/oder Gasströmen, wie ABC-Schutzmaskenfiltern, Geruchsfiltern, Flächenfiltern, Luftfiltern, insbesondere Filtern für die Raumluftreinigung, adsorptionsfähigen Trägerstrukturen und Filtern für den medizinischen Bereich.

Insbesondere eignet sich das Adsorptionsmaterial, wie zuvor definiert, für sorptive, insbesondere adsorptive Anwendungen, insbesondere als vorzugsweise reaktives bzw. katalytisches Adsorbens.

Gleichermaßen eignet sich das Adsorptionsmaterial für die Gasreinigung und/oder die Gasaufbereitung.

Weiterhin findet das Adsorptionsmaterial, wie zuvor definiert, Verwendung für die Entfernung von Schadstoffen, insbesondere gasförmigen Schadstoffen, oder von umweltschädlichen, gesundheitsschädlichen oder toxischen Substanzen oder Gasen.

Eine weitere Anwendungsmöglichkeit bzw. Verwendung des Adsorptionsmaterials, wie zuvor definiert, ist in der Aufbereitung bzw. Bereitstellung von Reinraumatmosphären, insbesondere für die Elektroindustrie, insbesondere für die Halbleiter- oder Chipherstellung, zu sehen.

Das Adsorptionsmaterial, wie zuvor definiert, kann Verwendung finden für Schutzmaterialien, insbesondere für den zivilen oder militärischen Bereich, insbesondere Schutzbekleidung, wie Schutzanzüge, Schutzhandschuhe, Schutzschuhwerk, Schutzsocken, Kopfschutzbekleidung und dergleichen, sowie Schutzabdeckungen, vorzugsweise alle vorgenannten Schutzmaterialien für den ABC-Einsatz, welche unter Verwendung des Adsorptionsmaterials, wie zuvor definiert, hergestellt sind bzw. welche das zuvor definierte Adsorptionsmaterial aufweisen.

Weiterhin betrifft die vorliegende Erfindung - gemäß einem **zweiten** Aspekt der vorliegenden Erfindung - Filter und Filtermaterialien zur Entfernung von Schad-, Geruchs- und Giftstoffen aller Art, insbesondere aus Luft- und/oder Gasströmen, wie ABC-Schutzmaskenfilter, Geruchsfilter, Flächenfilter, Luftfilter, insbesondere Filter für die Raumluftreinigung, adsorptionsfähige Trägerstrukturen und Filter für den medizinischen Bereich, welche unter Verwendung eines Adsorptionsmaterials, wie zuvor definiert, hergestellt sind bzw. welche das zuvor definierte Adsorptionsmaterial aufweisen, wobei das Adsorptionsmaterial aus kugelförmiger Aktivkohle gebildet ist, wobei die Aktivkohle erhalten ist durch Carbonisierung und anschließende Aktivierung von kugelförmigen, sulfonierten organischen Polymeren, und wobei das Adsorptionsmaterial ein inneres Porensystem aufweist, wobei das innere Porensystem des Adsorptionsmaterials mit mindestens einer Metallkomponente ausgerüstet ist, wobei die Metallkomponente in das innere Porensystem eingebracht und/oder eingelagert ist, wobei die Metallkomponente in Form eines Metalloxids in dem inneren Porensystem des Adsorptionsmaterials *in-situ* generiert ist, wobei die Metallkomponente als das Metalloxid in zumindest partiell kristalliner Form ausgebildet ist, wobei das Adsorptionsmaterial die Metallkomponente in Mengen im Bereich von 0,001 Gew.-% bis 40 Gew.-%, berechnet als Metall und bezogen auf das Gesamtgewicht des mit der Metallkomponente ausgerüsteten Adsorptionsmaterials, aufweist und wobei die Metallkomponente mindestens ein Metall in einer positiven Oxidationsstufe aufweist, wobei die Metallkomponente mindestens ein Metall, ausgewählt aus der Gruppe von Zn, Ag, Sn, Ni und Cu, vorzugsweise Cu, aufweist, wobei das Adsorptionsmaterial einen Feuchte- und/oder Wassergehalt im Bereich von 1 Gew.-% bis 60 Gew.-%, bezogen auf das Trockengewicht des mit der Metallkomponente ausgerüsteten Adsorptionsmaterials, aufweist und wobei das Adsorptionsmaterial auf einem Trägermaterial aufgebracht ist.

Die erfindungsgemäßen Filter und Filtermaterialien können dabei einen variablen Aufbau aufweisen.

Erfindungsgemäß ist es vorgesehen, dass das Adsorptionsmaterial auf einem Trägermaterial aufgebracht ist.

In diesem Zusammenhang kann es vorgesehen sein, dass das Trägermaterial gasdurchlässig, insbesondere luftdurchlässig, ausgebildet ist. In diesem Zusammenhang kann es erfindungsgemäß vorgesehen sein, dass das Trägermaterial eine Gasdurchlässigkeit, insbesondere Luftdurchlässigkeit, von mindestens 10 l·m⁻²·s⁻¹, insbesondere mindestens 30 l·m⁻²·s⁻¹, vorzugsweise mindestens 50 l·m⁻²·s⁻¹, besonders bevorzugt mindestens 100 l·m⁻²·s⁻¹, ganz besonders bevorzugt mindestens 500 l·m⁻²·s⁻¹, und/oder eine Gasdurchlässigkeit, insbesondere Luftdurchlässigkeit, von bis zu 10.000 l·m⁻²·s⁻¹, insbesondere bis zu 20.000 l·m⁻²·s⁻¹, bei einem Strömungswiderstand von 127 Pa aufweist.

Zudem kann es in diesem Zusammenhang gleichermaßen im Rahmen der, vorliegenden Erfindung vorgesehen sein, dass das Trägermaterial eine dreidimensionale Struktur aufweist. Diesbezüglich kann das Trägermaterial als vorzugsweise offenporiger Schaumstoff, besonders bevorzugt Polyurethanschaumstoff, ausgebildet sein.

Alternativ kann es jedoch auch vorgesehen sein, dass das Trägermaterial eine zweidimensionale bzw. flächige Struktur aufweist. Diesbezüglich kann das Trägermaterial als vorzugsweise textiles Flächengebilde ausgebildet sein.

Im Falle der zweidimensionalen Ausgestaltung des Trägermaterials kann es erfindungsgemäß vorgesehen sein, dass das Trägermaterial als ein textiles Flächengebilde, vorzugsweise ein luftdurchlässiges Textilmaterial, bevorzugt ein Gewebe, Gewirke, Gestricke, Gelege oder Textilverbundstoff, insbesondere Vlies (Nonwoven), ausgebildet ist. Insbesondere kann das Trägermaterial ein Flächengewicht von 5 bis 1.000 g/m², insbesondere 10 bis 500 g/m², bevorzugt 25 bis 450 g/m², aufweisen.

Insbesondere kann im Falle einer zweidimensionalen Ausgestaltung des Trägermaterials das Trägermaterial ein natürliche Fasern bzw. synthetische Fasern (Chemiefasern) enthaltendes oder hieraus bestehendes textiles Flächengebilde sein.

In diesem Zusammenhang können die natürlichen Fasern aus der Gruppe von Wollfasern und Baumwollfasern (CO) ausgewählt sein. Gleichermaßen können die synthetischen Fasern ausgewählt sein aus der Gruppe von Polyestern (PES); Polyolefinen, insbesondere Polyethylen (PE) und/oder Polypropylen (PP); Polyvinylchloriden (CLF); Polyvinylidenchloriden (CLF); Acetaten (CA); Triacetaten (CTA); Polyacryl (PAN); Polyamiden (PA), insbesondere aromatischen, vorzugsweise flammfesten Polyamiden; Polyvinylalkoholen (PVAL); Polyurethanen; Polyvinylestern; (Meth-)Acrylaten; Polymilchsäuren (PLA); Aktivkohle; sowie deren Mischungen.

Im Falle der Verwendung eines Trägermaterials ist es erfindungsgemäß bevorzugt, wenn das Adsorptionsmaterial an bzw. auf dem Trägermaterial fixiert ist. Dies kann beispielsweise mittels Verklebung, insbesondere mittels eines Klebstoffs oder in Folge von Eigenklebrigkeit oder von Eigenadhäsion realisiert sein.

Zusammenfassend wird im Rahmen der vorliegenden Erfindung erstmalig eine Konzeption bereitgestellt, welche zu einer effizienten Selbstdetoxifizierung bzw. Selbstreinigung von mit Schadstoffen beladenen Adsorbentien führt, wobei die zugrundeliegenden Schadstoffe zu weniger toxischen bzw. nicht toxischen Verbindungen abgebaut werden können, wodurch auch die Gesamtadsorption der eingesetzten Adsorptionsmaterialien vergrößert bzw. aufrechterhalten werden kann.

Im Folgenden wird die vorliegende Erfindung anhand von bevorzugte Ausführungsbeispiele darstellenden Zeichnungen bzw. Figurendarstellungen näher erläutert. Im Zusammenhang mit der Erläuterung dieser bevorzugten Ausführungsbeispiele der vorliegenden Erfindung, welche jedoch in Bezug auf die vorliegende Erfindung keinesfalls beschränkend sind, werden auch weitergehende Vorteile, Eigenschaften, Aspekte und Merkmale der vorliegenden Erfindung beschrieben.

In den Figurendarstellungen zeigt:

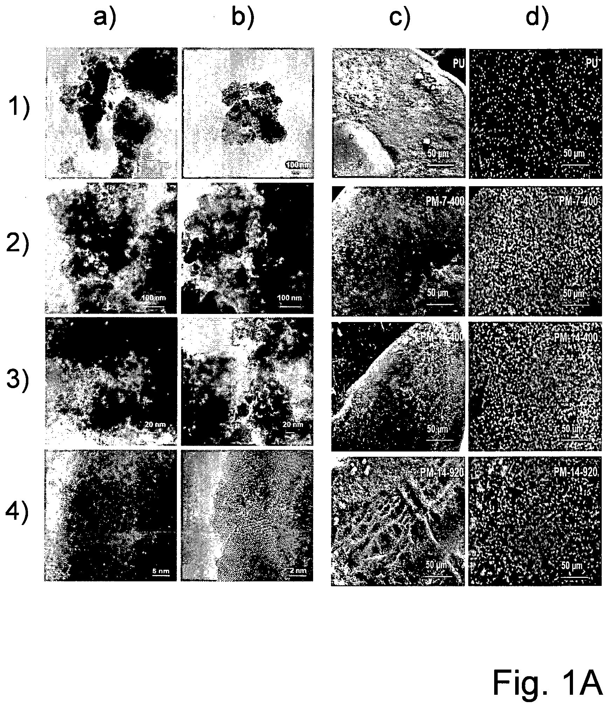

- Fig. 1A: Abbildungen auf Basis von Transmissionselektronenmikroskop-Untersuchungen (TEM) zur Bestimmung der Kristallitgröße sowie Abbildungen auf Basis von Rasterelektronenmikroskop-Untersuchungen (REM), kombiniert mit energiedispersiver Röntgenspektroskopie (XDR), für verschiedene Adsorptionsmaterialien auf Basis von Aktivkohle;

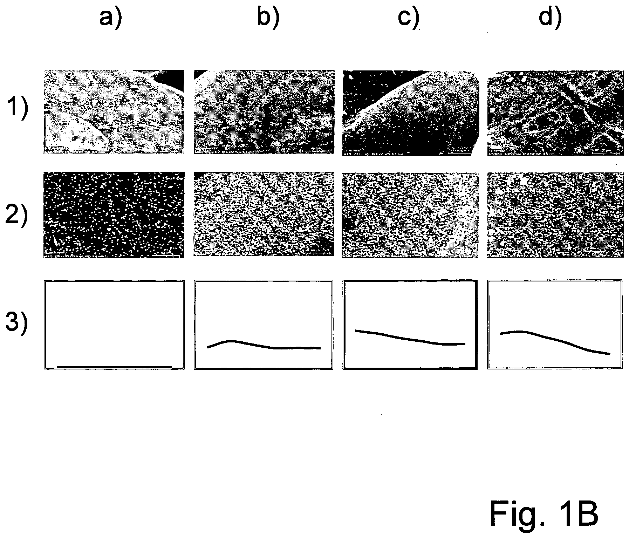

- Fig. 1B: Abbildungen auf Basis von RasterelektronenmikroskopieUntersuchungen (REM), kombiniert mit energiedispersiver Röntgenspektroskopie (XDR), für verschiedene Adsorptionsmaterialien auf Basis von Aktivkohle sowie graphische Darstellungen zur Verteilung des Metalloxids über den Kugelradius des zugrundeliegenden partikulären Adsorptionsmaterials;

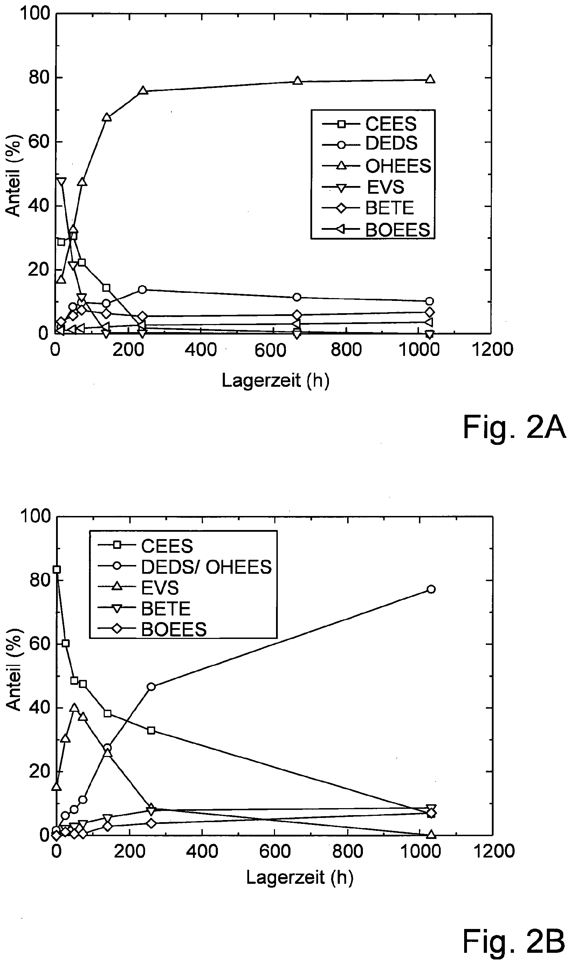

- Fig. 2A bis 2C: graphische Darstellungen der Zeitanhängigkeit des Abbaus der Ausgangsverbindung auf Basis von CEES sowie der Entstehung von Zwischen- bzw. Endprodukten;

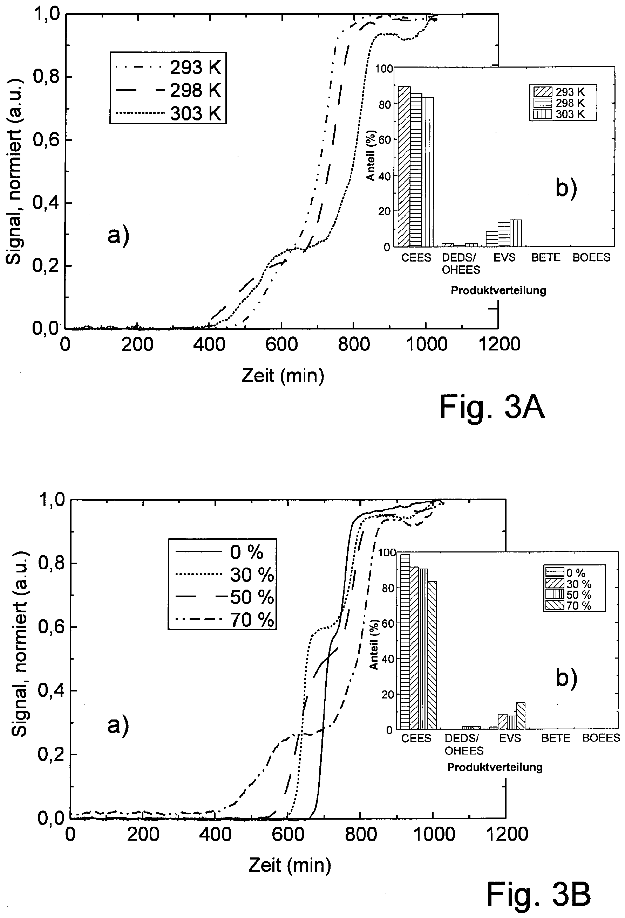

- Fig. 3A und 3B: graphische Darstellungen auf Basis von Durchbruchkurven zum Einfluss der Abbaubedingungen auf die Durchbruchcharakteristik, wobei die jeweiligen Darstellungen a) entsprechende Durchbruchkurven und die grafischen Darstellungen b) die zugrundeliegenden Produktverteilungen darstellen;

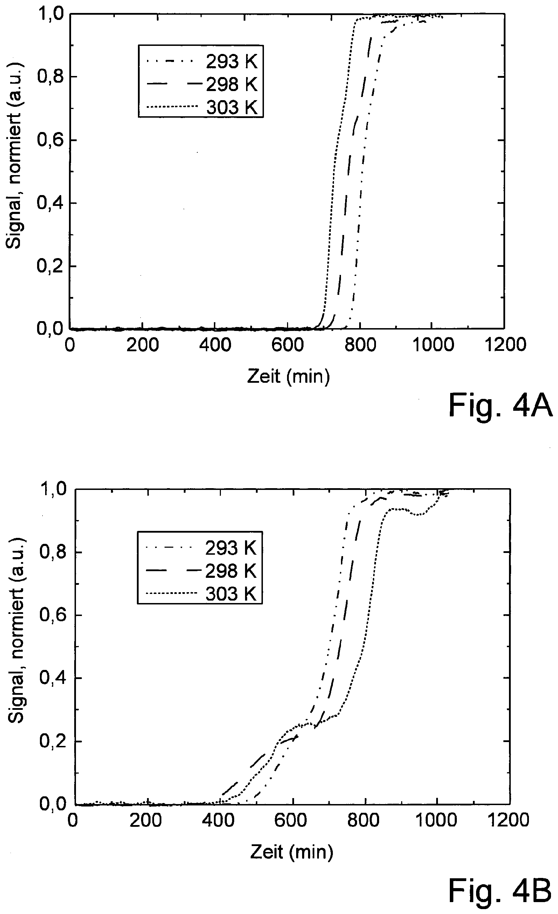

- Fig. 4A und 4B: graphische Darstellungen auf Basis von Durchbruchkurven unter verschiedenen Temperaturen und unterschiedlichen relativen Feuchten der eingesetzten Atmosphären;

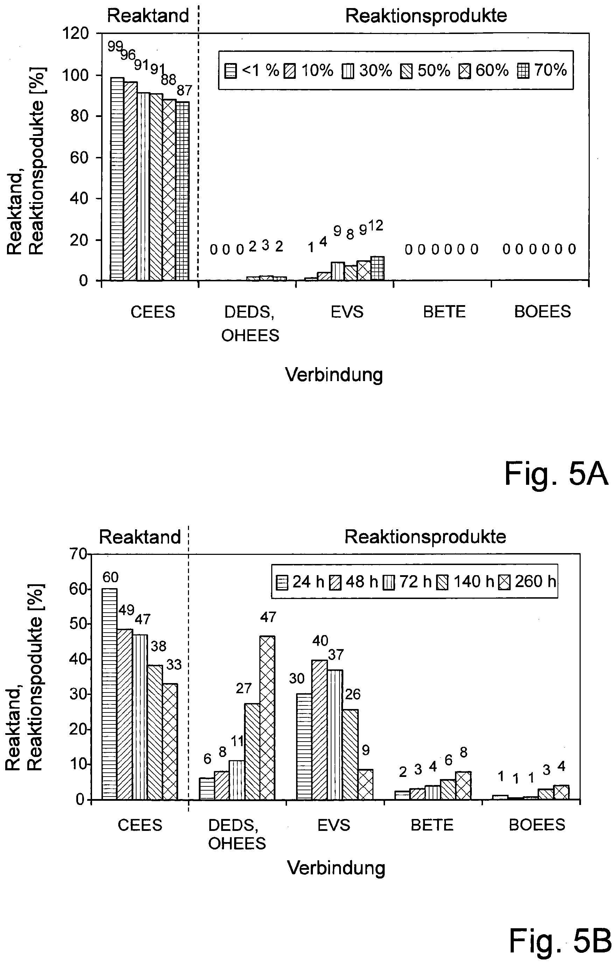

- Fig. 5A und 5B: graphische Darstellungen mit einem Vergleich des jeweiligen Anteils von Reaktand bzw. Ausgangsverbindung (CEES) und Zwischen- bzw. Endprodukten (Reaktionsprodukten) für verschiedene relative Feuchten der eingesetzten Atmosphären (Fig. 5A) sowie für verschiedene Abbaubzw. Lagerungszeiten der mit CEES beladenen Aktivkohle (Fig. 5B);

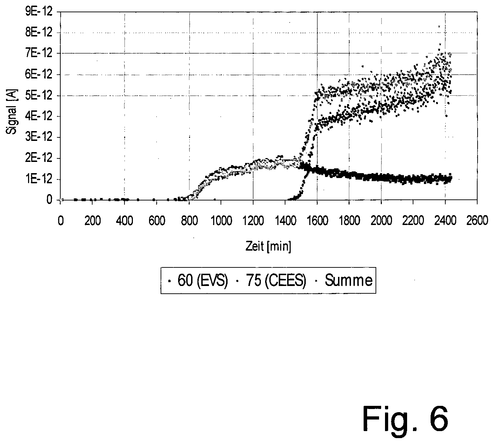

- Fig. 6: eine graphische Darstellung der Analyse des Gasdurchbruchs in Abhängigkeit von der Zeit für verschiedene Durchbruchkomponenten auf Basis von CEES als Ausgangssubstanz sowie EVS als Intermediat bzw. als Abbauprodukt der zugrundeliegenden Selbstdetoxifizierung des Adsorptionsmaterials.

Fig. 1A zeigt Abbildungen zur Analyse der resultierenden Kristallitgrößen des in dem Porensystem des Adsorptionsmaterials *in-situ* generierten Metalloxids (Spalten a) und b)), wobei es sich diesbezüglich jeweils um Kupferoxide handelt. Die Zeilen 1) bis 4) gemäß den Spalten a) und b) stellen das jeweils untersuchte Kristallitsystem in unterschiedlichen Auflösungen dar (von 200 nm bis 2 nm). Analysiert wurde eine mit einem Metalloxid in Form von Kupferoxid ausgerüstete Aktivkohle, welche einen Gehalt an Kupfer von etwa 7 Gew.-%, bezogen auf das Trockengewicht der modifizierten Aktivkohle, aufwies. Die Temperatur bei der Umsetzung des zum Erhalt des Metalloxids eingesetzten Kupfernitrats als Vorläufersubstanz betrug dabei 400 °C. Im Vergleich zu einer Aktivkohle mit einem höheren Gehalt an Kupfer bzw. im Vergleich zu einer Aktivkohle, bei welcher das Metalloxid ausgehend von der Vorläuferverbindung bei Temperaturen von 920 °C gewonnen wurde (nicht dargestellt), resultieren bei der in Fig. 1A gezeigten Aktivkohle kleinere Kristallite. Bei den Abbildungen gemäß den Spalten a) und b) handelt es sich um Aufnahmen bzw. Abbildungen, welche auf Basis von Transmissionselektronenmikroskopie (TEM) erhalten wurden.

Die Spalten c) und d) gemäß Fig. 1A zeigen die Verteilung der Metallkomponente im Querschnitt des partikulären Adsorptionsmaterials, wobei es sich bei dem Adsorptionsmaterial gemäß Zeile 1) um eine unmodifizierte Aktivkohle handelt. Bei der Aktivkohle gemäß Spalte 2) handelt es sich um eine Aktivkohle mit einem Gehalt an Kupfer von etwa 7 Gew.-%, bezogen auf das Trockengewicht der modifizierten Aktivkohle, wobei die Umwandlung ausgehend von der Vorläuferverbindung zu dem Metalloxid bei 400 °C durchgeführt wurde. Die Aktivkohle gemäß Spalte 3) entspricht derjenigen von Spalte 2) mit der Maßgabe, dass der Gehalt an Kupfer auf etwa 14 Gew.-%, bezogen auf das Trockengewicht der modifizierten Aktivkohle, eingestellt wurde. Bei der Aktivkohle gemäß Spalte 4) handelt es sich um eine Aktivkohle mit einem Gehalt an Kupfer von etwa 14 Gew.-%, bezogen auf das Trockengewicht der modifizierten Aktivkohle, wobei die Umwandlungsreaktion zum Erhalt des Metalloxids bei einer Temperatur von 920 °C durchgeführt worden ist. Bei den in Rede stehenden Abbildungen handelt es sich um rasterelektronenmikroskopische Aufnahmen (REM) bzw. um auf Basis von energiedispersiver Röntgenspektroskopie (RDX) erhaltene ortsaufgelöste semiquantitative Analysen der Verteilung der Metallkomponente über den Kugelquerschnitt des in Teilchenform vorliegenden Adsorptionsmaterials. Für die mit dem Metalloxid ausgerüstete Aktivkohle ergeben sich dabei zumindest im Wesentlichen homogene Verteilungen der Metallkomponente in Form des Metalloxids innerhalb der jeweiligen Partikel bzw. über das gesamte Porensystem des Adsorptionsmaterials insbesondere derart, dass auch der innerste Kernbereich der zugrundeliegenden Teilchen mit dem Metalloxid ausgerüstet ist.

In diesem Zusammenhang zeigt Fig. 1B in Anlehnung an die Spalten c) und d) gemäß Fig. 1A eine graphische Darstellung des Konzentrationsverlaufs des Metalls entlang des Kugelradius des zugrundeliegenden partikulären Adsorptionsmaterials, wobei Spalte a) gemäß Fig. 10 eine unmodifizierte Aktivkohle darstellt, während Spalte b) eine Aktivkohle mit einem Gehalt an Kupfer von etwa 7 Gew.-% bezogen auf das Trockengewicht der modifizierten Aktivkohle, bei einer Umwandlungstemperatur bei der Umsetzung der Vorläuferverbindung zu dem Metalloxid von 400 °C, Spalte c) eine Aktivkohle mit einem Gehalt an Kupfer von etwa 14 Gew.-%, bezogen auf das Trockengewicht der modifizierten Aktivkohle, bei einer Umwandlungstemperatur von 400 °C, sowie Spalte d) eine Aktivkohle mit einem Gehalt an Kupfer von etwa 14 Gew.-%, bezogen auf das Trockengewicht der modifizierten Aktivkohle, bei einer Umwandlungstemperatur von 920 °C, zeigt. In Zeile 3) gemäß Fig. 1B ist die Verteilung des Metalloxids, nämlich auf Basis von Kupfer, entlang des Radius der zugrundeliegenden Kugelform des Adsorptionsmaterials schematisch abgebildet, wobei die linke Seite in den jeweiligen Abbildungen der äußeren Kugeloberfläche und die rechte Seite den Mittelpunkt der Kugel des Adsorptionspartikels bedeutet. Es zeigt sich insgesamt eine vollständige Ausrüstung der Aktivkohle mit der Metalloxidkomponente über den gesamten Kugelradius.

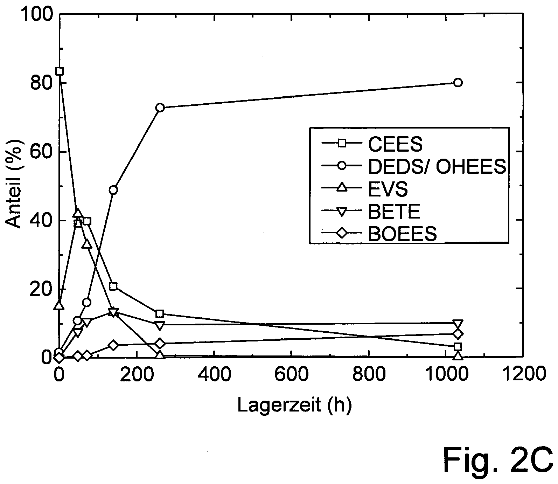

Fig. 2A bis 2C zeigen zudem die Abbauwerte der eingesetzten Ausgangssubstanzen in Form von CEES bzw. den Anteil der jeweiligen Zwischen- bzw. Endprodukte im zeitlichen Verlauf, wobei Fig. 2A auf einem stationären Versuchsansatz basiert, während Fig. 2B und 2C jeweils auf eine Lagerung nach einem dynamischen Versuch abzielen. Fig. 2C bezieht sich zudem auf einen Versuchsansatz, wonach zusätzlich 10 µl Wasser während der Lagerung der Aktivkohle hinzugegeben wurde. Die Figurendarstellungen belegen, dass CEES vollständig an kupfermodifizierten Aktivkohlen abgebaut werden kann, wobei die Versuchsdurchführungen auf Basis der Lagerung nach einem dynamischen Versuchsansatz zudem zeigen, dass EVS ein Zwischenprodukt des Abbaus von CEES darstellt. Zudem zeigen die Figuren, dass der Abbau bzw. die Zersetzung von CEES durch Zugabe von Wasser signifikant beschleunigt werden kann.

Fig. 3A und Fig. 3B zeigen gemäß den jeweiligen Darstellungen a) Durchbruchkurven, welche an einer mit Kupferoxid ausgerüsteten Aktivkohle bei verschiedenen Lagerungstemperaturen (Fig. 3A) und bei verschiedenen relativen Feuchten der eingesetzten Atmosphären (Fig. 3B) gewonnen wurden. Fig. 3A und 3B zeigen, dass bei höheren Temperaturen und höheren relativen Feuchten der Abbau der Ausgangssubstanz CEES größer ist. Fig. 3A und 3B zeigen somit den Einfluss der Abbaureaktion auf die Durchbruchcharakteristik. Den Figuren ist zudem zu entnehmen, dass das im Vergleich zu CEES als Ausgangssubstanz flüchtigere Reaktionsprodukt EVS in der Aktivkohle durch CEES verdrängt wird, was zu einer so genannten Reaktionsschulter in der entsprechenden Durchbruchkurve führt. Die Reaktionsschulterfläche ist dabei ein Maß für den Umsatz der Reaktion, und die Schulterhöhe ist ein Maß für den Austausch von EVS durch CEES pro Zeiteinheit.

Fig. 4A und Fig. 4B verdeutlichen den Einfluss der Abbaureaktion auf die Durchbruchcharakteristik in Abhängigkeit von der Temperatur und der relativen Feuchte ("rF"), wobei in Bezug auf Fig. 4A die relative Feuchte der Atmosphäre 0 % rF und in Bezug auf Fig. 4B die relative Feuchte 70 % rF in Bezug auf die jeweils eingesetzte Atmosphäre betrug. Es wird deutlich, dass erhöhte relative Feuchten zu einem höheren Umsatz bzw. zu einem vergrößerten Abbau der Ausgangssubstanz in Form von CEES führt.

Weiterhin zeigt Fig. 5A den Abbau bzw. die Zersetzung von CEES als Ausgangssubstanz zu den jeweiligen Zwischen- bzw. Endprodukten bei verschiedenen relativen Feuchten der eingesetzten Atmosphäre. Die Daten wurden bei folgenden Parametern bzw. Einstellungen ermittelt: Konzentration CEES: c_{CEES} = g/m³; m = 300 mg; Vol = 200 ml/min; rF = ≤ 1, 10, 30, 50, 60, 70 %; T = 30° C, Trägergas: N₂, Extraktion mit Dichlormethan.

Fig. 5A zeigt, dass mit zunehmender relativer Feuchte der eingesetzten Atmosphäre der Abbau von CEES vergrößert ist, wobei EVS das resultierende Hauptprodukt der Abbaureaktion ist. Ein weiteres Abbauprodukt, nämlich OHEES, tritt erst bei relativen Feuchten von mehr als 50 % rF auf.

Fig. 5B zeigt eine zu Fig. 5A entsprechende Untersuchung, wobei jedoch die relative Feuchte konstant auf einen Wert 70 % rF eingestellt wurde. Die weiteren Einstellungen bzw. Parameter entsprechen denjenigen gemäß Fig. 5A. Fig. 5B zeigt den Abbau der Ausgangssubstanz CEES in Abhängigkeit von der Zeit und das diesbezügliche Auftreten der jeweiligen Zwischen- bzw. Endprodukte. Fig. 5B veranschaulicht, dass OHEES das Hauptabbauprodukt für Lagerzeiten bzw. Abbauzeiten über 260 h ist, wogegen EVS das Hauptprodukt für Lager- bzw. Abbauzeiten von weniger als 140 h ist. Im Allgemeinen können zudem BETE, BOEES sowie DEDS als Abbauprodukte ermittelt werden.

Fig. 6 zeigt schließlich eine Analyse des Gasdurchbruchs auf Basis einer Untersuchung unter dynamischen Bedingungen mit einer differenzierten Betrachtung des zeitabhängigen Durchbruchs von CEES als Ausgangssubstanz und EVS als Abbau- bzw. Zersetzungsprodukt. Fig. 6 zeigt, dass der Schulterbereich der resultierenden Gesamtdurchbruchkurve durch EVS als Abbauprodukt gebildet wird.

Für die in den obigen Figurendarstellungen angeführten Versuche wurde jeweils eine mit Kupferoxid modifizierte Aktivkohle auf Basis einer polymerbasierten Aktivkohle (PBSAC) eingesetzt. Als Präkursor wurde Kupfernitrat verwendet.

Die angeführten Untersuchungen belegen im Ergebnis die hervorragenden Eigenschaften der im Rahmen des erfindungsgemäßen Verfahrens eingesetzten modifizierten Aktivkohle, insbesondere im Hinblick auf die zugrundeliegenden selbstdetoxifizierenden bzw. selbstreinigenden Eigenschaften.

Die vorstehend erläuterten Figurendarstellungen werden auch im Rahmen der erfindungsgemäßen Ausführungsbeispiele nochmals weiterführend erläutert.

Weitere Ausgestaltungen, Abwandlungen, Variationen, Modifikationen, Besonderheiten und Vorteile der vorliegenden Erfindung sind für den Fachmann beim Lesen der Beschreibung ohne weiteres erkennbar und realisierbar, ohne dass er dabei den Rahmen der vorliegenden Erfindung verlässt.

Die vorliegende Erfindung wird anhand der nachfolgenden Ausführungsbeispiele veranschaulicht, welche die vorliegende Erfindung jedoch keinesfalls beschränken sollen.

### AUSFÜHRUNGSBEISPIELE:

### 1. Herstellung von mit einem Metalloxid in Form von Kupferoxid ausgerüsteten Adsorptionsmaterialien

Im Rahmen der Ausrüstung des Adsorptionsmaterials mit der Metallkomponente wurde eine polymerbasierte sphärische Aktivkohle (PBSAC) eingesetzt, wobei der Aktivkohleträger ein Gesamtporenvolumen von 1,2 cm³/g bei einem Mikroporenanteil von etwa 50 Vol.-% aufwies. Die Ausrüstung der Aktivkohle bzw. des Aktivkohleträgers mit der Metallkomponente wurde im Rahmen eines Batch-Prozesses durchgeführt. Dabei wurde in einem ersten Schritt der Aktivkohleträger mittels einer Tauchmethode mit Kupfernitrat als Vorläuferverbindung der Metallkomponente aus einer wässrigen Lösung imprägniert. Anschließend wurde der so ausgerüsteten Aktivkohleträger unter inerten Bedingungen thermisch getrocknet und nachbehandelt.

Es wurden zwei unterschiedliche Beladungen mit der Metallkomponente realisiert, wobei eine Beladung mit etwa 7 Gew.-% und eine weitere Beladung mit etwa 14 Gew.-% mit der entsprechenden Kupferverbindung durchgeführt wurde, wobei zudem die Beladung mit etwa 14 Gew.-% der Kupferkomponente in zwei voneinander verschiedenen Ansätzen bei unterschiedlichen Temperaturen zur Umwandlung der Vorläuferverbindung auf Basis von Kupfernitrat zu dem Metalloxid in Form von Kupferoxid durchgeführt wurde, nämlich bei einer Temperatur von 400° C zum Erhalt einer erfindungsgemäß eingesetzten Aktivkohle B einerseits und 920° C zum Erhalt einer erfindungsgemäß eingesetzten Aktivkohle C andererseits. Im Gegensatz dazu wurde die Aktivkohle, welche mit etwa 7 Gew.-% der Kupferverbindung beladen wurde, bei 400° C nachbehandelt (erfindungsgemäß eingesetzte Aktivkohle A). Die entsprechenden Gewichtsangaben sind berechnet als Metall und beziehen sich jeweils auf das Trockengewicht der modifizierten Aktivkohle. Die Metallgehalte wurden mittels Elementaranalyse bestimmt.

Die oben angeführte Imprägnierung der Aktivkohleträger wurde in diesem Zusammenhang in einem Drehrohrreaktor durchgeführt. Hierzu wurden 3,1 l destilliertes Wasser als Lösemittel für 0,37 kg Kupfernitrat bzw. 1,1 kg Kupfernitrat eingesetzt, wobei die auf dieser Basis erhaltene höher konzentrierte Imprägnierlösung zur Ausrüstung des Aktivkohleträgers mit der Kupferverbindung in einer Menge von etwa 14 Gew.-% eingesetzt wurde, während die Imprägnierlösung mit der geringeren Konzentration zur Ausrüstung des Aktivkohleträgers mit der Kupferverbindung in einer Menge von etwa 7 Gew.-% eingesetzt wurde. Die jeweiligen Imprägnierlösungen wurden jeweils vollständig mit dem jeweiligen Aktivkohleträger in dem Drehrohrreaktor eingebracht. Nach einer Imprägnierdauer von 1 h wurde die verbleibende Restlösung abgelassen und nachfolgend eine thermische Nachbehandlung durchgeführt. Die thermische Nachbehandlung wurde ebenfalls in dem Drehrohrreaktor durchgeführt, wobei dieser mittels eines Drehrohrreaktorofens elektrisch beheizt wurde. Die Nachbehandlung wurde unter einer Inertgasatmosphäre auf Basis von Stickstoff durchgeführt. Der thermischen Umwandlung der Vorläuferverbindung in Form von Kupfernitrat zu dem Metalloxid in Form von Kupferoxid wurde dabei ein Trocknungsschritt vorgeschaltet, wobei diesbezüglich auf Temperaturen von 200 °C mit einer Haltezeit von 150 min aufgeheizt wurde. Im Anschluss an den Trocknungsschritt wurde der Drehrohrreaktor auf die jeweilige Umwandlungs- bzw. Zersetzungstemperatur von 400 °C bzw. 920 °C aufgeheizt, wobei die diesbezügliche Haltezeit 180 min bzw. 240 min betrug. Anschließend wurden die so erhaltenen modifizierten Aktivkohlen mit der Ausrüstung auf Basis des Metalloxids abgekühlt.

Die erfindungsgemäß eingesetzten Adsorptionsmaterialien wurden insbesondere im Hinblick auf die resultierende Kristallitgrößen des Metalloxids und der entsprechenden Verteilung innerhalb der Teilchen des Adsorptionsmaterials analysiert. Wie zuvor angeführt, ergeben sich dabei zumindest im Wesentlichen homogene Verteilungen der Metallkomponente in Form des Metalloxids innerhalb der jeweiligen Partikel bzw. über das gesamte Porensystem des Adsorptionsmaterials, so dass sozusagen an jeder Stelle innerhalb des Porensystems bzw. über das gesamte Porensystem des Adsorptionsmaterials eine Selbstdetoxifizierung stattfinden kann. Zudem zeigen die Analysen der Kristallitgrößen, dass diesbezüglich hinsichtlich der Selbstdetoxifizierung optimale Größen und optimale Oberflächenstrukturen vorliegen, welche gleichermaßen die Adsorptionseigenschaften als solche des Adsorptionsmaterials zumindest im Wesentlichen nicht beeinträchtigen. Insbesondere liegt keine Verstopfung oder Blockierung des Porensystems vor. Für weitere diesbezügliche Ausführungen kann auf die obigen Ausführungen zu Fig. 1A und Fig. 1B verwiesen werden.

In entsprechender Weise wurden darüber hinaus modifizierte Aktivkohlen hergestellt, welche eine Metalloxidkomponente auf Basis von Zink, Silber, Zinn sowie Nickel enthielten.

### 2. Weiterführende Analysen und Anwendungsbeispiele der mit einer Metallkomponente ausgerüsteten Adsorptionsmaterialien

### a) Zeitabhängigkeit des Abbaus von CEES sowie Einfluss der Lagerungsbedingungen auf die Abbaurate und das Auftreten bzw. die Verteilung der auftretenden Abbauprodukte

In diesem Versuchsansatz wurden die selbstdetoxifizierenden Eigenschaften der zuvor hergestellten Adsorptionsmaterialien unter Verwendung eines Surrogats bzw. Ersatzstoffs für Senfgas (HD), nämlich Chloroethylethylsulfid (CEES), bestimmt. CEES wurde insbesondere auch vor dem Hintergrund eingesetzt, dass aufgrund der Chemiewaffenkonvention, welche Deutschland im Jahre 1994 ratifiziert hat, Untersuchungen auf Basis der Verwendung von CWAs nicht zulässig sind. Wie zuvor ausgeführt, stellt CEES ein etabliertes Surrogat für Senfgas (HD) dar, so dass die diesbezüglichen Untersuchungsergebnisse entsprechend aussagekräftig sind.

Im Rahmen des vorliegenden Versuchsansatzes wurden mit CEES beladende Adsorbentien zum einen einer Lagerung unter stationären Bedingungen und andererseits einer Lagerung nach einem dynamischen Versuchsansatz unterzogen.

In diesem Zusammenhang zeigt Fig. 2A den zeitlichen Verlauf des Abbaus von CEES und das Auftreten von Zwischen- und Endprodukten der zugrundeliegenden Zersetzung von CEES infolge der Anwesenheit der Metallkomponente in Form von Kupferoxid: Während CEES im zeitlichen Verlauf vollständig abgebaut wird, kann die Entstehung der weniger bzw. nicht toxischen Substanzen DEDS, OHEES, BETE und BOEES beobachtet werden. Zudem kann ein weiterführender Abbau von EVS beobachtet werden.

Dass EVS als Intermediat bzw. Zwischenprodukt der Abbaureaktion von CEES fungiert, kann zudem Fig. 2B mit der veranschaulichten Lagerung nach dynamischer Beladung entnommen werden. Die Menge an EVS durchläuft dabei im zeitlichen Verlauf ein Maximum und nimmt danach kontinuierlich ab, was bei gleichzeitigem Rückgang der Menge an CEES auf einen weiterführenden Abbau von EVS zu weiteren Produkten hindeutet. In diesem Zusammenhang können DEDS und OHEES als Hauptabbauprodukte sowie zudem BETE und BOEES als darüber hinaus auftretende Produkte ermittelt werden.

Fig. 2C bezieht sich zudem auf einen Versuchsansatz mit einer Lagerung der Adsorbentien nach dynamischer Beladung mit CEES unter Zugabe von 10 µl Wasser während der Lagerung der Aktivkohle. Ein Vergleich zu Fig. 2B zeigt einen deutlich schnelleren Abbau von CEES und damit einhergehend einen rascheren Anstieg insbesondere der Menge an auftretendem DEDS und OHEES.

Die Untersuchungen zeigen somit, dass CEES vollständig an kupfermodifizierten Aktivkohlen abgebaut werden kann, wobei sich zudem zeigt, dass EVS als Zwischenprodukt bzw. Intermediat des Abbaus von CEES fungiert. Zudem ergeben die Versuche, dass der Abbau bzw. die Zersetzung von CEES durch Zugabe von Wasser signifikant beschleunigt werden kann. In diesem Zusammenhang kann auch auf obige Ausführungen zu Fig. 2A bis Fig. 2C verwiesen werden.

### b) Einfluss der Temperatur und Feuchtigkeit bzw. des Wassergehalts auf den Abbau von CEES

In einem weiteren Versuchskomplex wurde der Einfluss der Temperatur und des Feuchtigkeits- bzw. Wassergehalts der die Aktivkohle umgebenden bzw. umströmenden Atmosphäre auf das Abbauverhalten gegenüber CEES untersucht. Hierzu wurden unter dynamischen Bedingungen mit CEES beladene und mit Kupferoxid ausgerüstete Aktivkohlen auf Basis der Bestimmung entsprechender Durchbruchkurven analysiert, wie in Fig. 3A (Temperatureinfluss, Darstellung a)) und Fig. 3B (Einfluss der relativen Feuchte, Darstellung a)) dargestellt. Zudem wurden die zugrundeliegenden Stoffmengen an CEES und der entsprechenden Abbauprodukte zu einem definierten Zeitpunkt analysiert (jeweilige Darstellungen b)).

Bei höheren Temperaturen und höheren relativen Feuchten ist der Abbau der Ausgangssubstanz CEES jeweils beschleunigt, was der entsprechenden Verschiebung der Durchbruchkurven im unteren Signalbereich zu kürzeren Zeiten und der Ausbildung eines entsprechenden Schulterbereichs in den Durchbruchkurven bei höheren Temperaturen bzw. höheren relativen Feuchten zu entnehmen ist. In diesem Zusammenhang bildet das im Vergleich zu CEES als Ausgangssubstanz flüchtigere Reaktionsprodukt EVS den zuvor angeführten Schulterbereich in den entsprechenden Durchbruchkurven aus. Es wird deutlich, dass erhöhte Temperaturen sowie erhöhte relative Feuchten, unabhängig voneinander, zu einem höheren Umsatz bzw. zu einem verstärkten Abbau der Ausgangssubstanz in Form von CEES führt, insbesondere wobei pro Zeiteinheit eine größere Menge an CEES abgebaut wird.

Die Abbaureaktion von CEES hat somit einen Einfluss auf die Durchbruchcharakteristik, was sich durch die Ausbildung des in Rede stehenden Schulterbereichs in der Durchbruchkurve bemerkbar macht, insbesondere wobei das flüchtigere Reaktionsprodukt EVS im dynamischen Ansatz durch nachströmendes CEES an den Bindungstellen des Adsorptionsmaterials ersetzt bzw. verdrängt wird.

Für weitere Informationen kann auf obige Ausführungen zu Fig. 3A und Fig. 3B sowie zu Fig. 4A und Fig. 4B verwiesen werden.

### c) Einfluss der Feuchtigkeit bzw. des Wassergehalts auf den Abbau von CEES sowie zeitlicher Verlauf der Abbaureaktion; weiterführende Analyse der resultierenden Abbauprodukte

Darüber hinaus wurde der Einfluss der Temperatur auf die Abbauwege bzw. die Zersetzungsmechanismen von CEES als Ausgangssubstanz zu den jeweiligen Zwischen- bzw. Endprodukten untersucht.

Dabei zeigt sich, dass mit zunehmender relativer Feuchte der eingesetzten Atmosphäre der Abbau von CEES vergrößert ist, wobei EVS das resultierende Hauptprodukt der Abbaureaktion ist. Ein weiteres Abbauprodukt, nämlich OHEES, tritt erst bei relativen Feuchten von mehr als 50 % rF auf.

Was die diesbezügliche Einflussnahme auf die beim Abbau von CEES auftretenden Zwischen- und Endprodukte anbelangt, so zeigt sich, dass bei konstanter relativer Feuchte (70 % rF) OHEES das Hauptabbauprodukt für Lagerzeiten bzw. Abbauzeiten über 260 h ist, wogegen EVS das Hauptprodukt für Lager- bzw. Abbauzeiten von weniger als 140 h ist. Im Allgemeinen können zudem BETE, BOEES sowie DEDS als Abbauprodukte ermittelt werden. Es bestätigt sich, dass EVS unter den gegebenen Bedingungen der Abbaureaktion als Intermediat fungiert.

Für weitere Ausführungen kann auf obige Ausführungen zu Fig. 5A und Fig. 5B verwiesen werden.

Ohne sich auf diese Theorie beschränken zu wollen, können insbesondere im Rahmen einer dynamischen Adsorption als Primärreaktionen die Umsetzung von CEES zu EVS insbesondere für sämtliche relativen Feuchten sowie die Umsetzung von CEES zu OHEES insbesondere für relative Feuchten von mehr als 50 % rF angenommen werden. Zudem kann, gleichermaßen ohne sich auf diese Theorie festlegen zu wollen, insbesondere während der Lagerung und insbesondere im Sinne von Folgereaktionen DEDS das Produkt der Reaktion von -S-CH₂-CH₃ und CEES darstellen, während BETE das Produkt der Reaktion von -S-CH₂-CH₃ und EVS sowie BOEES das Produkt der Kondensationsreaktion von OHEES und CEES darstellen kann.

### d) Untersuchung zum Abbauverhalten von Vergleichsadsorptionsmaterialien gegenüber CEES

Weiterhin wurden Vergleichsadsorptionsmaterialien auf Basis einer unbehandelten Aktivkohle (Aktivkohle D) sowie einer lediglich mit Kupfernitrat ausgerüsteten Aktivkohle (Aktivkohle E) in Anlehnung an den obigen Ausführungen zu Fig. 2A und Fig. 2B durchgeführt. Für die unbehandelte Aktivkohle D konnte nach einem Lagerzeitraum von 600 h ein nur vernachlässigbarer Rückgang der CEES-Beladung von lediglich etwa 5 % ermittelt werden, und auch für die Aktivkohle E war der Rückgang der CEES-Beladung deutlich geringer als bei den modifizierten Aktivkohlen.

Die Versuche zeigen, dass das modifizierte Adsorptionsmaterial mit der speziellen Ausrüstung auf Basis einer insbesondere kristallinen Metalloxidkomponente anderen Adsorbentien hinsichtlich der Selbstreinigung bzw. Selbstdetoxifizierung weit überlegen ist. Insbesondere kann auf Basis der vorliegenden Erfindung ein Schadstoff (wie CEES) zu weniger toxischen Verbindungen abgebaut werden, wobei insbesondere sogar ein vollständiger Abbau des Schadstoffes realisiert werden kann. Dabei kann insbesondere auch die Gesamtadsorptionskapazität des Adsorptionsmaterials vergrößert werden, da aufgrund der insbesondere im Rahmen eines dynamischen Ansatzes vorliegenden Prozesse der Adsorption, Reaktion, Verdrängung der flüchtigeren Produkte und Neubesetzung freiwerdender Adsorptionsstellen die Aufnahmefähigkeit für zu sorbierende Stoffe erhöht ist.

Die Untersuchungen belegen im Ergebnis die hervorragenden Eigenschaften der modifizierten Aktivkohle, insbesondere im Hinblick auf ihre selbstdetoxifizierenden bzw. selbstreinigenden Eigenschaften.

## Patentansprüche

1. Verfahren zur Selbstdetoxifizierung und/oder Selbstreinigung mindestens eines ein inneres Porensystem aufweisenden Adsorptionsmaterials, wobei das Adsorptionsmaterial aus kugelförmiger Aktivkohle gebildet wird, wobei die Aktivkohle erhalten wird durch Carbonisierung und anschließende Aktivierung von kugelförmigen, sulfonierten organischen Polymeren,
wobei zunächst das innere Porensystem des Adsorptionsmaterials mit mindestens einer Metallkomponente ausgerüstet wird,
wobei die Metallkomponente in das innere Porensystem eingebracht und/oder eingelagert wird, wobei die Metallkomponente in Form eines Metalloxids in dem inneren Porensystem des Adsorptionsmaterials *in-situ* generiert wird, wobei die Metallkomponente als das Metalloxid in zumindest partiell kristalliner Form ausgebildet wird und wobei das Adsorptionsmaterial die Metallkomponente in Mengen im Bereich von 0,001 Gew.-% bis 40 Gew.-%, berechnet als Metall und bezogen auf das Gesamtgewicht des mit der Metallkomponente ausgerüsteten Adsorptionsmaterials, aufweist, wobei die Metallkomponente mindestens ein Metall in einer positiven Oxidationsstufe aufweist und wobei die Metallkomponente mindestens ein Metall, ausgewählt aus der Gruppe von Zn, Ag, Sn, Ni und Cu, vorzugsweise Cu, aufweist,
und nachfolgend das auf diese Weise ausgerüstete Adsorptionsmaterial mit mindestens einem chemischen Schadstoff in Kontakt gebracht wird, so dass eine zumindest partielle Zersetzung und/oder ein zumindest partieller Abbau des chemischen Schadstoffs erfolgt,
wobei der chemische Schadstoff mindestens eine gasförmige und/oder ein Aerosol ausbildende Verbindung umfasst und wobei der chemische Schadstoff ausgewählt wird aus der Gruppe von Abgasen aus Industrieprozessen und Kraftmaschinen oder wobei als chemischer Schadstoff ein chemischer Kampfstoff und/oder dessen Surrogat und/oder Ersatzstoff eingesetzt wird und
wobei das Adsorptionsmaterial nach der Ausbildung der Metallkomponente zu dem Metalloxid und/oder während des Inkontaktbringens mit dem chemischen Schadstoff mit Wasser in Kontakt gebracht wird, wobei das Adsorptionsmaterial einen Feuchte- und/oder Wassergehalt im Bereich von 1 Gew.-% bis 60 Gew.-%, bezogen auf das Trockengewicht des mit der Metallkomponente ausgerüsteten Adsorptionsmaterials, aufweist.

2. Verfahren nach Anspruch 1,
wobei die Metallkomponente zumindest teilweise in Teilchenform und/oder in partikulärer Form, insbesondere in kristalliner Form, vorzugsweise in Form von Kristalliten, vorliegt; und/oder
wobei die Teilchen und/oder Partikel, insbesondere die Kristallite, der Metallkomponente eine mittlere Teilchen- und/oder Partikelgröße, insbesondere eine mittlere Kristallitgröße, im Bereich von 0,1 nm bis 500 nm, insbesondere 1 nm bis 500 nm, vorzugsweise im Bereich von 2 nm bis 400 nm, bevorzugt im Bereich von 5 nm bis 300 nm, besonders bevorzugt im Bereich von 10 nm bis 200 nm, ganz besonders bevorzugt im Bereich von 15 nm bis 150 nm, aufweisen; und/oder
wobei die Metallkomponente eine Kristallinität, insbesondere einen Kristallinitätsgrad, von mindestens 10 %, insbesondere mindestens 30 %, vorzugsweise mindestens 50 %, bevorzugt mindestens 80 %, besonders bevorzugt mindestens 90 %, ganz besonders bevorzugt mindestens 95 %, bezogen auf die Metallkomponente, aufweist und/oder wobei die Metallkomponente eine Kristallinität, insbesondere einen Kristallinitätsgrad, im Bereich von 10% bis 99,5 %, insbesondere im Bereich von 30% bis 99 %, vorzugsweise im Bereich von 50 % bis 98 %, bevorzugt im Bereich von 80 % bis 95 %, aufweist; und/oder
wobei das Adsorptionsmaterial die Metallkomponente in Mengen im Bereich von 0,01 Gew.-% bis 35 Gew.-%, vorzugsweise im Bereich von 0,5 Gew.-% bis 30 Gew.-%, bevorzugt im Bereich von 1 Gew.-% bis 25 Gew.-%, besonders bevorzugt im Bereich von 2 Gew.-% bis 20 Gew.-%, berechnet als Metall und bezogen auf das Gesamtgewicht des mit der Metallkomponente ausgerüsteten Adsorptionsmaterials aufweist.

3. Verfahren nach Anspruch 1 oder 2,
wobei die Oxidationsstufe des Metalls im Bereich von +I bis +VII, insbesondere im Bereich von +I bis +IV, vorzugsweise im Bereich von +I bis +III, liegt und besonders bevorzugt +I oder +II ist; und/oder
wobei die Metallkomponente mindestens ein Metall, ausgewählt aus der Gruppe von Zn(+II), Ag(+I), Sn(+II), Sn(+IV), Ni(+II), Cu(+I) und Cu(+II), vorzugsweise Cu(+I) und/oder Cu(+II), besonders bevorzugt Cu(+I), aufweist; und/oder wobei die Metallkomponente in Form eines Kupferoxids vorliegt und/oder wobei die Metallkomponente in Form von CuO und/oder Cu₂O, insbesondere Cu₂O, vorliegt.

4. Verfahren nach einem der vorangehenden Ansprüche,
wobei die Metallkomponente in Form des Metalloxids in dem inneren Porensystem des Adsorptionsmaterials auf Basis mindestens einer Vorläuferverbindung und/oder Präkursorverbindung, insbesondere mindestens einem Präkursorsalz, der Metallkomponente, *in-situ* generiert wird, insbesondere wobei die Vorläuferverbindung und/oder Präkursorverbindung, insbesondere das Präkursorsalz, in Form einer vorzugsweise wässrigen Lösung und/oder Dispersion eingesetzt wird, insbesondere wobei die Lösung und/oder Dispersion die Vorläuferverbindung und/oder Präkursorverbindung, insbesondere das Präkursorsalz, in Mengen im Bereich von 0,5 Gew.-% bis 80 Gew.-%, insbesondere im Bereich von 1 Gew.-% bis 70 Gew.-%, vorzugsweise im Bereich von 2 Gew.-% bis 60 Gew.-%, bevorzugt im Bereich von 5 Gew.-% bis 50 Gew.-%, bezogen auf die Lösung und/oder Dispersion, enthält und/oder insbesondere wobei die wässrige Lösung und/oder Dispersion die Vorläuferverbindung und/oder Präkursorverbindung, insbesondere das Präkursorsalz, in Konzentrationen im Bereich von 0,2 mol/l bis 8 mol/l, insbesondere im Bereich von 0,5 mol/l bis 7 mol/l, vorzugsweise im Bereich von 1 mol/l bis 6 mol/l, bevorzugt im Bereich von 1,5 mol/l bis 5 mol/l, bezogen auf die Lösung und/oder Dispersion, enthält; und/oder
wobei die Metallkomponente in Form des Metalloxids in dem inneren Porensystem des Adsorptionsmaterials durch Ausrüstung, insbesondere Tränken und/oder Imprägnieren, des Adsorptionsmaterials erhalten wird mit mindestens einer vorzugsweise wässrigen Lösung und/oder Dispersion einer Vorläuferverbindung und/oder Präkursorverbindung, insbesondere mindestens eines Präkursorsalzes, der Metallkomponente und nachfolgende Umsetzung und/oder Umwandlung, insbesondere thermische Umsetzung und/oder thermische Umwandlung und/oder thermische Zersetzung, der mindestens einen Vorläuferverbindung und/oder Präkursorverbindung, insbesondere dem mindestens einen Präkursorsalz, zu dem Metalloxid, insbesondere wobei nach der Ausrüstung, insbesondere nach dem Tränken und/oder Imprägnieren, und/oder vor der Umsetzung und/oder Umwandlung eine Trocknung des mit der Vorläuferverbindung und/oder Präkursorverbindung, insbesondere dem Präkursorsalz, ausgerüsteten Adsorptionsmaterials durchgeführt wird, insbesondere wobei die Trocknung bei Temperaturen im Bereich von 50 °C bis 200 °C und/oder für eine Zeitdauer von bis zu 5 h durchgeführt wird.

5. Verfahren nach Anspruch 4,
wobei als Vorläuferverbindung und/oder Präkursorverbindung, insbesondere Präkursorsalz, ein anorganisches Salz eingesetzt wird und/oder wobei die Vorläuferverbindung und/oder Präkursorverbindung, insbesondere das Präkursorsalz, ausgewählt wird aus der Gruppe von Halogenidsalzen, Sulfaten, Sulfiden, Sulfiten, Nitraten, Nitriten, Nitriden, Phosphaten, Phosphiden, Phosphiten, Carbamaten, Alkoholaten, Carbonaten und Hydrogencarbonaten, insbesondere Nitraten; und/oder wobei als Vorläuferverbindung und/oder Präkursorverbindung, insbesondere Präkursorsalz, ein Nitrat eingesetzt wird und/oder wobei als Vorläuferverbindung und/oder Präkursorverbindung, insbesondere Präkursorsalz, ein Kupfersalz, insbesondere ein Kupfer(II)-Salz, vorzugsweise Kupfernitrat, bevorzugt Kupfer(II)-nitrat (Cu(NO₃)₂), eingesetzt wird; und/oder
wobei die Reaktions- und/oder Umsetzungsparameter, insbesondere die Temperatur und/oder die Atmosphäre, der Umsetzung und/oder Umwandlung der Vorläuferverbindung und/oder Präkursorverbindung, insbesondere des Präkursorsalzes, zu dem Metalloxid zur Einstellung und/oder Vorgabe der resultierenden Oxidationsstufe, insbesondere Oxidationszahl, des Metalls in dem resultierenden Metalloxid ausgewählt werden; und/oder
wobei die Umsetzung und/oder Umwandlung der Vorläuferverbindung und/oder Präkursorverbindung, insbesondere des Präkursorsalzes, zu dem Metalloxid in einer Inertgasatmosphäre, insbesondere in einer stickstoffhaltigen und/oder edelgashaltigen Inertgasatmosphäre, durchgeführt wird; und/oder
wobei die Umsetzung und/oder Umwandlung der Vorläuferverbindung und/oder Präkursorverbindung, insbesondere des Präkursorsalzes, zu dem Metalloxid bei Temperaturen im Bereich von 100 °C bis 1.000 °C, insbesondere im Bereich von 150 °C bis 950 °C, vorzugsweise 200 °C bis 920 °C, durchgeführt wird.

6. Verfahren nach Anspruch 4 oder 5,
wobei die Umsetzung und/oder Umwandlung und/oder Zersetzung der Vorläuferverbindung und/oder Präkursorverbindung, insbesondere des Präkursorsalzes, zu dem Metalloxid bei Temperaturen bis 400 °C und/oder bei Temperaturen im Bereich von 200 °C bis 400 °C durchgeführt wird, insbesondere wobei als Vorläuferverbindung und/oder Präkursorverbindung, insbesondere Präkursorsalz, ein Kupfernitrat, insbesondere Kupfer(II)-nitrat und/oder Cu(NO₃)₂, eingesetzt wird und/oder insbesondere wobei als Metalloxid mindestens ein Kupferoxid, insbesondere Kupfer(II)-oxid (CuO), erhalten wird; oder
wobei die Umsetzung und/oder Umwandlung der mindestens einen Vorläuferverbindung und/oder Präkursorverbindung, insbesondere des mindestens einen Präkursorsalzes, zu dem Metalloxid bei Temperaturen von mehr als 400 °C und/oder bei Temperaturen im Bereich von 400 °C bis 920 °C durchgeführt wird, insbesondere wobei als Vorläuferverbindung und/oder Präkursorverbindung, insbesondere Präkursorsalz, ein Kupfernitrat, insbesondere Kupfer(II)-nitrat und/oder Cu(NO₃)₂, eingesetzt wird und/oder insbesondere wobei als Metalloxid mindestens ein Kupferoxid, insbesondere Kupfer(I)-oxid (Cu₂O), erhalten wird.

7. Verfahren nach einem der vorangehenden Ansprüche,
wobei als chemischer Schadstoff ein chemischer Kampfstoff und/oder dessen Surrogat und/oder Ersatzstoff eingesetzt wird, wobei der chemische Kampfstoff ausgewählt wird aus der Gruppe von Senfgas, Soman, Sarin, Phosgen und Tabun sowie deren Mischungen, und/oder wobei als chemischer Kampfstoff ein Ersatzstoff und/oder ein Surrogat von Senfgas, Soman, Sarin, Phosgen und/oder Tabun, insbesondere Ersatzstoff und/oder ein Surrogat von Senfgas, eingesetzt wird und/oder wobei als chemischer Schadstoff, insbesondere chemischer und/oder biologischer Kampfstoff, Chloroethylethylsulfid (CEES) eingesetzt wird; und/oder
wobei das Inkontaktbringen des Adsorptionsmaterials mit dem chemischen Schadstoff derart erfolgt, dass der chemische Schadstoff zumindest teilweise in das innere Porensystem des Adsorptionsmaterials eindringt und/oder dass der chemische Schadstoff in das innere Porensystem des Adsorptionsmaterials einzudringen imstande ist; und/oder
wobei das Inkontaktbringen des Adsorptionsmaterials mit dem chemischen Schadstoff und/oder die Zersetzung und/oder der Abbau des chemischen Schadstoffs unter stationären oder dynamischen Bedingungen erfolgt bzw. erfolgen; und/oder
wobei der chemische Schadstoff einer Atmosphäre, insbesondere Reaktionsatmosphäre, zugesetzt wird und/oder wobei das Adsorptionsmaterial mit der den chemischen Schadstoff enthaltenden Atmosphäre in Kontakt gebracht wird, insbesondere wobei die Atmosphäre den chemischen Schadstoff in Mengen im Bereich von 0,1 g/m³ bis 20 g/m³, insbesondere im Bereich von 0,5 g/m³ bis 10 g/m³, vorzugsweise im Bereich von 0,75 g/m³ bis 5 g/m³, enthält; und/oder wobei der chemische Schadstoff insbesondere in dem inneren Porensystem des Adsorptionsmaterials zumindest teilweise gebunden wird, insbesondere auf Basis von Sorption, vorzugsweise Adsorption, insbesondere auf Basis von Physisorption und/oder Chemisorption; und/oder
wobei das Adsorptionsmaterial den chemischen Schadstoff in Mengen im Bereich von 0,01 Gew.-% bis 80 Gew.-%, insbesondere im Bereich von 0,05 Gew.-% bis 60 Gew.-%, vorzugsweise im Bereich von 0,1 Gew.-% bis 40 Gew.-%, bevorzugt im Bereich von 1 Gew.-% bis 30 Gew.-%, bezogen auf das Gesamtgewicht des mit der Metallkomponente ausgerüsteten Adsorptionsmaterials, aufweist und/oder adsorbiert bzw. bindet.

8. Verfahren nach einem der vorangehenden Ansprüche, wobei der chemische Schadstoff insbesondere in dem inneren Porensystem des Adsorptionsmaterial zumindest teilweise abgebaut und/oder zersetzt wird und/oder wobei der Abbau und/oder die Zersetzung unter Molekülspaltung, vorzugsweise unter Dehalogenierung und/oder Dehydrohalogenierung und/oder Hydrolyse, des chemischen Schadstoffs erfolgt und/oder wobei der Abbau auf Basis einer stöchiometrischen und/oder reaktiven Umsetzung und/oder einer katalytischen Umsetzung und/oder auf Basis von Oxidation erfolgt, insbesondere wobei die Abbauprodukte des chemischen Schadstoffs zumindest teilweise desorbiert und/oder in die das Adsorptionsmaterial umgebende Atmosphäre abgeben werden, insbesondere so dass zuvor durch den chemischen Schadstoff belegte Adsorptionsstellen und/oder Anbindungsstellen des Adsorptionsmaterials zur Anbindung von weiterem chemischen Schadstoff freigegeben werden.

9. Verfahren nach einem der vorangehenden Ansprüche, wobei das Inkontaktbringen des Adsorptionsmaterial mit dem chemischem Schadstoff und/oder die Zersetzung und/oder der Abbau des chemischen Schadstoffs bei Temperaturen im Bereich von - 20 °C bis 100 °C, insbesondere im Bereich von 5 °C bis 60 °C, vorzugsweise im Bereich von 10 °C bis 40 °C, durchgeführt wird und/oder wobei das Inkontaktbringen des Adsorptionsmaterials mit dem chemischen Schadstoff und/oder die Zersetzung und/oder der Abbau des chemischen Schadstoffs für eine Zeitdauer im Bereich von 1 min bis 5.000 min, insbesondere im Bereich von 10 min bis 4.000 min, vorzugsweise im Bereich von 60 min bis 2.500 min, bevorzugt im Bereich von 100 min bis 2.000 min, durchgeführt wird.

10. Verfahren nach einem der vorangehenden Ansprüche,
wobei das Adsorptionsmaterial mit Wasser ausgerüstet und/oder beladen wird; und/oder wobei das Adsorptionsmaterial einen Feuchte- und/oder Wassergehalt im Bereich von 2 Gew.-% bis 55 Gew.-%, vorzugsweise im Bereich von 3 Gew.-% bis 50 Gew.-%, bevorzugt im Bereich von 5 Gew.-% bis 50 Gew.-%, bezogen auf das Trockengewicht des mit der Metallkomponente ausgerüsteten Adsorptionsmaterials, aufweist.

11. Verfahren nach einem der vorangehenden Ansprüche, wobei das Adsorptionsmaterial Teilchengrößen, insbesondere Teilchendurchmesser, im Bereich von 0,001 bis 3 mm, insbesondere im Bereich von 0,005 bis 2,5 mm, vorzugsweise im Bereich von 0,01 bis 2 mm, besonders bevorzugt im Bereich von 0,02 bis 1,5 mm, ganz besonders bevorzugt im Bereich von 0,05 bis 1 mm, aufweist und/oder wobei das Adsorptionsmaterial mittlere Teilchengrößen, insbesondere mittlere Teilchendurchmesser (D50), im Bereich von 0,01 bis 2 mm, insbesondere im Bereich von 0,05 bis 1,5 mm, vorzugsweise im Bereich von 0,1 bis 1 mm, aufweist.

12. Verfahren nach einem der vorangehenden Ansprüche, wobei das Adsorptionsmaterial eine spezifische Oberfläche (BET-Oberfläche) von mindestens 500 m²/g, insbesondere mindestens 750 m²/g, vorzugsweise mindestens 1.000 m²/g, besonders bevorzugt mindestens 1.200 m²/g, aufweist und/oder wobei das Adsorptionsmaterial eine spezifische Oberfläche (BET-Oberfläche) im Bereich von 500 bis 4.000 m²/g, insbesondere im Bereich von 750 bis 3.000 m²/g, vorzugsweise im Bereich von 900 bis 2.500 m²/g, besonders bevorzugt 950 bis 2.000 m²/g, aufweist.

13. Filter und Filtermaterialien zur Entfernung von Schad-, Geruchs- und Giftstoffen aller Art, hergestellt unter Verwendung eines selbstdetoxifizierenden und/oder selbstreinigenden Adsorptionsmaterials und/oder aufweisend ein selbstdetoxifizierendes und/oder selbstreinigendes Adsorptionsmaterial,
wobei das Adsorptionsmaterial aus kugelförmiger Aktivkohle gebildet ist, wobei die Aktivkohle erhalten ist durch Carbonisierung und anschließende Aktivierung von kugelförmigen, sulfonierten organischen Polymeren, und
wobei das Adsorptionsmaterial ein inneres Porensystem aufweist, wobei das innere Porensystem des Adsorptionsmaterials mit mindestens einer Metallkomponente ausgerüstet ist, wobei die Metallkomponente in das innere Porensystem eingebracht und/oder eingelagert ist, wobei die Metallkomponente in Form eines Metalloxids in dem inneren Porensystem des Adsorptionsmaterials *in-situ* generiert ist, wobei die Metallkomponente als das Metalloxid in zumindest partiell kristalliner Form ausgebildet ist, wobei das Adsorptionsmaterial die Metallkomponente in Mengen im Bereich von 0,001 Gew.-% bis 40 Gew.-%, berechnet als Metall und bezogen auf das Gesamtgewicht des mit der Metallkomponente ausgerüsteten Adsorptionsmaterials, aufweist und wobei die Metallkomponente mindestens ein Metall in einer positiven Oxidationsstufe aufweist, wobei die Metallkomponente mindestens ein Metall, ausgewählt aus der Gruppe von Zn, Ag, Sn, Ni und Cu, vorzugsweise Cu, aufweist, wobei das Adsorptionsmaterial einen Feuchte- und/oder Wassergehalt im Bereich von 1 Gew.-% bis 60 Gew.-%, bezogen auf das Trockengewicht des mit der Metallkomponente ausgerüsteten Adsorptionsmaterials, aufweist und
wobei das Adsorptionsmaterial auf einem Trägermaterial aufgebracht ist.

14. Filter und Filtermaterialien nach Anspruch 13,
wobei das Trägermaterial eine dreidimensionale Struktur aufweist, insbesondere wobei das Trägermaterial als vorzugsweise offenporiger Schaumstoff, besonders bevorzugt Polyurethanschaumstoff, ausgebildet ist; oder
wobei das Trägermaterial eine zweidimensionale und/oder flächige Struktur aufweist, insbesondere wobei das Trägermaterial als vorzugsweise textiles Flächengebilde ausgebildet ist,
insbesondere wobei das Trägermaterial als ein textiles Flächengebilde, vorzugsweise ein luftdurchlässiges Textilmaterial, bevorzugt ein Gewebe, Gewirke, Gestricke, Gelege oder Textilverbundstoff, insbesondere Vlies (Nonwoven), ausgebildet ist und/oder insbesondere wobei das Trägermaterial ein Flächengewicht von 5 bis 1.000 g/m², insbesondere 10 bis 500 g/m², bevorzugt 25 bis 450 g/m², aufweist und/oder insbesondere wobei das Trägermaterial ein natürliche Fasern und/oder synthetische Fasern (Chemiefasern) enthaltendes oder hieraus bestehendes textiles Flächengebilde ist und/oder
insbesondere wobei das Adsorptionsmaterial an und/oder auf dem Trägermaterial fixiert ist, vorzugsweise mittels Verklebung, insbesondere mittels eines Klebstoffs oder infolge von Eigenklebrigkeit oder von Eigenadhäsion.

## Claims

1. Method for self-detoxification and/or self-decontamination of at least one adsorption material including an internal system of pores, wherein the adsorption material is formed of spherical activated carbon, wherein the activated carbon is obtained by carbonization and subsequent activation of spherical sulphonated organic polymers,
wherein initially the internal system of pores in the adsorption material is endowed with at least one metallic component,
wherein the metallic component is introduced and/or imported into the internal system of pores, wherein the metallic component is generated in situ in the form of a metal oxide in the internal system of pores in the adsorption material, wherein the metallic component is formed as the metal oxide in at least partly crystalline form and wherein the adsorption material includes the metallic component in amounts ranging from 0.001 wt% to 40 wt%, reckoned as metal and based on the overall weight of the adsorption material endowed with the metallic component, wherein the metallic component includes at least one metal in a positive oxidation state and wherein the metallic component includes at least one metal selected from the group of Zn, Ag, Sn, Ni and Cu, preferably Cu,
and thereafter the adsorption material thus endowed is brought into contact with at least one chemical noxiant so that an at least partial decomposition and/or degradation of the chemical noxiant takes place,
wherein the chemical noxiant comprises at least one gaseous and/or aerosol-forming compound and wherein the chemical noxiant is selected from the group of offgases from engines and industrial processes or wherein the chemical noxiant employed is a chemical warfare agent and/or its surrogate and/or substitute, and
wherein the adsorption material is brought into contact with water after the metallic component has been formed into the metal oxide and/or during the step of bringing into contact with the chemical noxiant, wherein the adsorption material has a moisture and/or water content ranging from 1 wt% to 60 wt%, based on the dry weight of the adsorption material endowed with the metallic component.

2. Method according to Claim 1,
wherein the metallic component is at least partly present in corpuscle form and/or in particulate form, in particular in crystalline form, preferably in the form of crystallites; and/or
wherein the corpuscles and/or particles, in particular the crystallites, of the metallic component have an average corpuscle and/or particle size, in particular an average crystallite size, in the range from 0.1 nm to 500 nm, in particular 1 nm to 500 nm, preferably in the range from 2 nm to 400 nm, preferably in the range from 5 nm to 300 nm, more preferably in the range from 10 nm to 200 nm and most preferably in the range from 15 nm to 150 nm; and/or
wherein the metallic component has a crystallinity, in particular a degree of crystallinity, of not less than 10%, particularly not less than 30%, preferably not less than 50%, more preferably not less than 80%, yet more preferably not less than 90%, most preferably not less than 95%, based on the metallic component, and/or wherein the metallic component has a crystallinity, in particular a degree of crystallinity, in the range from 10% to 99.5%, particularly in the range from 30% to 99%, preferably in the range from 50% to 98%, more preferably in the range from 80% to 95%; and/or
wherein the adsorption material includes the metallic component in amounts ranging from 0.01 wt% to 35 wt%, preferably from 0.5 wt% to 30 wt%, more preferably from 1 wt% to 25 wt% and yet more preferably from 2 wt% to 20 wt%, reckoned as metal and based on the overall weight of the adsorption material endowed with the metallic component.

3. Method according to Claim 1 or 2,
wherein the oxidation state of the metal is in the range from +I to +VII, particularly in the range from +I to +IV, preferably in the range from +I to +III and more preferably is +I or +II; and/or
wherein the metallic component includes at least one metal selected from the group of Zn(+II), Ag(+I), Sn(+II), Sn(+IV), Ni(+II), Cu(+I) and Cu(+II), preferably Cu(+I) and/or Cu(+II), more preferably Cu(+I); and/or
wherein the metallic component is in the form of a copper oxide and/or wherein the metallic component is in the form of CuO and/or Cu₂O, in particular Cu₂O.

4. Method according to any preceding claim,
wherein the metallic component in the form of the metal oxide is generated in situ in the internal system of pores in the adsorption material on the basis of at least one precedent and/or precursor compound, in particular at least one precursor salt, of the metallic component, more particularly wherein the precedent and/or precursor compound, in particular the precursor salt, is employed in the form of a preferably aqueous solution and/or dispersion, more particularly wherein the solution and/or dispersion contains the precedent and/or precursor compound, in particular the precursor salt, in amounts ranging from 0.5 wt% to 80 wt%, particularly from 1 wt% to 70 wt%, preferably from 2 wt% to 60 wt%, more preferably from 5 wt% to 50 wt%, based on the solution and/or dispersion, and/or more particularly wherein the aqueous solution and/or dispersion contains the precedent and/or precursor compound, in particular the precursor salt, in concentrations ranging from 0.2 mol/l to 8 mol/l, particularly from 0.5 mol/l to 7 mol/l, preferably from 1 mol/l to 6 mol/l, more preferably from 1.5 mol/l to 5 mol/l, based on the solution and/or dispersion; and/or
wherein the metallic component in the form of the metal oxide is obtained in the internal system of pores in the adsorption material by endowing, more particularly drenching and/or impregnating, the adsorption material with at least one preferably aqueous solution and/or dispersion of a precedent and/or precursor compound, in particular at least one precursor salt, of the metallic component and subsequent conversion and/or transformation, more particularly thermal conversion and/or thermal transformation and/or thermal decomposition, of the at least one precedent and/or precursor compound, in particular the at least one precursor salt, into the metal oxide, more particularly wherein after the step of endowing, more particularly drenching and/or impregnating, and/or before the step of converting and/or transforming there is carried out a step of drying the adsorption material endowed with the precedent and/or precursor compound, in particular the precursor salt, more particularly wherein the step of drying is carried out at temperatures in the range from 50°C to 200°C and/or for a period of up to 5 h.

5. Method according to Claim 4,
wherein the precedent and/or precursor compound, in particular precursor salt, used is an inorganic salt, and/or wherein the precedent and/or precursor compound, in particular the precursor salt, is selected from the group of halide salts, sulphates, sulphides, sulphites, nitrates, nitrites, nitrides, phosphates, phosphides, phosphites, carbamates, alkoxides, carbonates and bicarbonates, in particular nitrates; and/or
wherein the precedent and/or precursor compound, in particular precursor salt, used is a nitrate, and/or wherein the precedent and/or precursor compound, in particular precursor salt, used is a copper salt, in particular a copper(II) salt, preferably copper nitrate, preferably copper(II) nitrate (Cu(NO₃)₂); and/or
wherein the reaction and/or conversion parameters, in particular the temperature and/or the atmosphere, of the conversion and/or transformation of the precedent and/or precursor compound, in particular the precursor salt, into the metal oxide are selected so as to establish and/or predetermine the resulting oxidation state, in particular oxidation number, of the metal in the resulting metal oxide; and/or
wherein the conversion and/or transformation of the precedent and/or precursor compound, in particular the precursor salt, into the metal oxide is carried out in an inert gas atmosphere, more particularly in a nitrogen-containing and/or noble gas-containing inert gas atmosphere; and/or
wherein the conversion and/or transformation of the precedent and/or precursor compound, in particular the precursor salt, into the metal oxide is carried out at temperatures ranging from 100°C to 1000°C, particularly from 150°C to 950°C, preferably from 200°C to 920°C.

6. Method according to Claim 4 or 5,
wherein the conversion and/or transformation and/or decomposition of the precedent and/or precursor compound, in particular the precursor salt, into the metal oxide is carried out at temperatures up to 400°C and/or at temperatures ranging from 200°C to 400°C, more particularly wherein the precedent and/or precursor compound, in particular precursor salt, used is a copper nitrate, in particular copper(II) nitrate and/or Cu(NO₃)₂, and/or more particularly wherein the metal oxide obtained is at least one copper oxide, in particular copper(II) oxide (CuO); or
wherein the conversion and/or transformation of the at least one precedent and/or precursor compound, in particular the at least one precursor salt, into the metal oxide is carried out at temperatures of more than 400°C and/or at temperatures ranging from 400°C to 920°C, more particularly wherein the precedent and/or precursor compound, in particular precursor salt, used is a copper nitrate, more particularly copper(II) nitrate and/or Cu(NO₃)₂; and/or more particularly wherein the metal oxide obtained is at least one copper oxide, in particular copper(I) oxide (Cu₂O).

7. Method according to any preceding claim,
wherein the chemical noxiant employed is a chemical warfare agent and/or its surrogate and/or substitute, wherein the chemical warfare agent is selected from the group of mustard gas, soman, sarin, phosgene and tabun and also their mixtures, and/or wherein the chemical warfare agent used is a substitute and/or a surrogate of mustard gas, soman, sarin, phosgene and/or tabun, more particularly wherein a substitute and/or surrogate of mustard gas is employed, and/or wherein the chemical noxiant employed, more particularly the chemical and/or biological warfare agent employed, is chloroethyl ethyl sulphide (CEES); and/or
wherein the step of bringing the adsorption material into contact with the chemical noxiant is effected such that the chemical noxiant penetrates at least partly into the internal system of pores in the adsorption material, and/or such that the chemical noxiant is able to penetrate into the internal system of pores in the adsorption material; and/or
wherein the step of bringing the adsorption material into contact with the chemical noxiant and/or the decomposition and/or breakdown of the chemical noxiant is effected under steady-state or dynamic conditions; and/or
wherein the chemical noxiant is added to an atmosphere, more particularly to a reaction atmosphere; and/or wherein the adsorption material is brought into contact with the atmosphere containing the chemical noxiant, more particularly wherein the atmosphere contains the chemical noxiant in amounts ranging from 0.1 g/m³ to 20 g/m³, particularly from 0.5 g/m³ to 10 g/m³, preferably from 0.75 g/m³ to 5 g/m³; and/or
wherein the chemical noxiant is more particularly at least partly bonded in the internal system of pores in the adsorption material, more particularly on the basis of sorption, preferably adsorption, more particularly on the basis of physisorption and/or chemisorption; and/or
wherein the adsorption material includes and/or adsorbs/attaches the chemical noxiant in amounts ranging from 0.01 wt% to 80 wt%, particularly from 0.05 wt% to 60 wt%, preferably from 0.1 wt% to 40 wt%, more preferably from 1 wt% to 30 wt%, based on the overall weight of the adsorption material endowed with the metallic component.

8. Method according to any preceding claim, wherein the chemical noxiant is more particularly at least partly broken down and/or decomposed in the internal system of pores in the adsorption material, and/or wherein the breakdown and/or the decomposition take place by molecular scission, preferably by dehalogenation and/or dehydrohalogenation and/or hydrolysis, of the chemical noxiant, and/or wherein the breakdown is effected on the basis of a stoichiometric and/or reactive conversion and/or a catalytic conversion and/or on the basis of oxidation, more particularly wherein the breakdown products of the chemical noxiant are at least partly desorbed and/or released into the atmosphere surrounding the adsorption material, more particularly so that adsorption and/or attachment sites of the adsorption material which were previously occupied by the chemical noxiant are freed for attachment of further chemical noxiant.

9. Method according to any preceding claim, wherein the step of bringing the adsorption material into contact with the chemical noxiant and/or the decomposition and/or the breakdown of the chemical noxiant are carried out at temperatures in the range from -20°C to 100°C, particularly in the range from 5°C to 60°C, preferably in the range from 10°C to 40°C, and/or wherein the step of bringing the adsorption material into contact with the chemical noxiant and/or the decomposition and/or the breakdown of the chemical noxiant are carried out for a time period ranging from 1 min to 5000 min, particularly from 10 min to 4000 min, preferably from 60 min to 2500 min, more preferably from 100 min to 2000 min.

10. Method according to any preceding claim,
wherein the adsorption material is endowed and/or loaded with water; and/or
wherein the adsorption material has a moisture and/or water content in the range from 2 wt% to 55 wt%, preferably in the range from 3 wt% to 50 wt%, more preferably in the range from 5 wt% to 50 wt%, based on the dry weight of the adsorption material endowed with the metallic component.

11. Method according to any preceding claim, wherein the adsorption material has corpuscle sizes, in particular corpuscle diameters, in the range from 0.001 to 3 mm, particularly in the range from 0.005 to 2.5 mm, preferably in the range from 0.01 to 2 mm, more preferably in the range from 0.02 to 1.5 mm, most preferably in the range from 0.05 to 1 mm, and/or wherein the adsorption material has average corpuscle sizes, more particularly median corpuscle diameters (D50), in the range from 0.01 to 2 mm, particularly in the range from 0.05 to 1.5 mm, preferably in the range from 0.1 to 1 mm.

12. Method according to any preceding claim, wherein the adsorption material has a specific surface area (BET surface area) of not less than 500 m²/g, particularly not less than 750 m²/g, preferably not less than 1000 m²/g, more preferably not less than 1200 m²/g, and/or wherein the adsorption material has a specific surface area (BET surface area) in the range from 500 to 4000 m²/g, particularly in the range from 750 to 3000 m²/g, preferably in the range from 900 to 2500 m²/g, more preferably in the range from 950 to 2000 m²/g.

13. Filters and filter materials for removing noxiant, odorant and poisonous chemistries of any kind, obtained by using a self-detoxifying and/or self-decontaminating adsorption material and/or including a self-detoxifying and/or self-decontaminating adsorption material,
wherein the adsorption material is formed of spherical activated carbon, wherein the activated carbon is obtained by carbonization and subsequent activation of spherical sulphonated organic polymers, and
wherein the adsorption material includes an internal system of pores, wherein the internal system of pores in the adsorption material has been endowed with at least one metallic component, wherein the metallic component is introduced and/or imported into the internal system of pores, wherein the metallic component is generated in situ in the form of a metal oxide in the internal system of pores in the adsorption material, wherein the metallic component is formed as the metal oxide in at least partly crystalline form, wherein the adsorption material includes the metallic component in amounts ranging from 0.001 wt% to 40 wt%, reckoned as metal and based on the overall weight of the adsorption material endowed with the metallic component and wherein the metallic component includes at least one metal in a positive oxidation state, wherein the metallic component includes at least one metal selected from the group of Zn, Ag, Sn, Ni and Cu, preferably Cu,
wherein the adsorption material has a moisture and/or water content ranging from 1 wt% to 60 wt%, based on the dry weight of the adsorption material endowed with the metallic component, and
wherein a carrier material supports the adsorption material.

14. Filters and filter materials according to Claim 13,
wherein the carrier material has a three-dimensional structure, more particularly wherein the carrier material is configured as a preferably open-cell foam, more preferably polyurethane foam; or
wherein the carrier material has a two-dimensional and/or sheetlike structure, more particularly wherein the carrier material is configured as a preferably textile fabric,
more particularly wherein the carrier material is configured as a textile fabric, preferably an air-permeable textile material, more preferably a woven, knitted, laid or bonded textile fabric, more particularly a nonwoven, and/or more particularly wherein the carrier material has a basis weight of 5 to 1000 g/m², particularly 10 to 500 g/m², preferably 25 to 450 g/m², and/or
more particularly wherein the carrier material is a textile fabric containing or consisting of natural fibres and/or synthetic fibres (manufactured fibres), and/or
more particularly wherein the adsorption material is fixed to and/or on the carrier material, preferably via adherence, more particularly via an adhesive or as a consequence of self-tackiness or of autoadhesion.

## Revendications

1. Procédé pour l'autodétoxification et/ou l'autopurification d'au moins un matériau d'adsorption présentant un système interne de pores, le matériau d'adsorption étant formé à partir de charbon actif sphérique, le charbon actif étant obtenu par carbonisation et activation consécutive de polymères organiques sulfonés, sphériques,
le système interne de pores du matériau d'adsorption étant d'abord pourvu d'au moins un composant métallique,
le composant métallique étant introduit et/ou incorporé dans le système interne de pores, le composant métallique étant généré in situ sous forme d'un oxyde métallique dans le système interne de pores du matériau d'adsorption, le composant métallique étant réalisé en tant qu'oxyde métallique sous une forme au moins partiellement cristalline et le matériau d'adsorption présentant le composant métallique en des quantités dans la plage de 0,001% en poids à 40% en poids, calculés sous forme de métal et par rapport au poids total du matériau d'adsorption pourvu du composant métallique, le composant métallique présentant au moins un métal dans un étage d'oxydation positif et le composant métallique présentant au moins un métal choisi dans le groupe formé par Zn, Ag, Sn, Ni et Cu, de préférence Cu,
et le matériau d'adsorption ainsi apprêté étant ensuite mis en contact avec au moins une substance chimique nuisible de manière telle qu'il se produit une décomposition au moins partielle et/ou une dégradation au moins partielle de la substance chimique nuisible,
la substance chimique nuisible comprenant au moins un composé gazeux et/ou formant un aérosol et la substance chimique nuisible étant choisie dans le groupe des effluents gazeux de processus industriels et de machines ou un gaz chimique de combat et/ou son succédané et/ou son produit de remplacement étant utilisé(s) comme substance chimique nuisible et
le matériau d'adsorption, après la formation du composant métallique en oxyde métallique et/ou pendant la mise en contact avec la substance chimique nuisible, étant mis en contact avec de l'eau, le matériau d'adsorption présentant une teneur en humidité et/ou en eau dans la plage de 1% en poids à 60% en poids, par rapport au poids sec du matériau d'adsorption pourvu du composant métallique.

2. Procédé selon la revendication 1,
le composant métallique se trouvant au moins partiellement sous forme de fragments et/ou sous forme particulaire, en particulier sous forme cristalline, de préférence sous forme de cristallites ; et/ou les fragments et/ou les particules, en particulier les cristallites, du composant métallique présentant une grosseur moyenne de fragment et/ou de particule, en particulier une grosseur moyenne de cristallite, dans la plage de 0,1 nm à 500 nm, en particulier de 1 nm à 500 nm, de préférence dans la plage de 2 nm à 400 nm, de préférence dans la plage de 5 nm à 300 nm, de manière particulièrement préférée dans la plage de 10 nm à 200 nm, de manière tout particulièrement préférée dans la plage de 15 nm à 150 nm ; et/ou le composant métallique présentant une cristallinité, en particulier un degré de cristallinité, d'au moins 10% en particulier d'au moins 30%, de préférence d'au moins 50%, de préférence d'au moins 80%, de manière particulièrement préférée d'au moins 90%, de manière tout particulièrement préférée d'au moins 95%, par rapport au composant métallique et/ou le composant métallique présentant une cristallinité, en particulier un degré de cristallinité, dans la plage de 10% à 99,5%, en particulier dans la plage de 30% à 99%, de préférence dans la plage de 50% à 98%, de préférence dans la plage de 80% à 95% ; et/ou
le matériau d'adsorption présentant le composant métallique en des quantités dans la plage de 0,01% en poids à 35% en poids, de préférence dans la plage de 0,5% en poids à 30% en poids, de préférence dans la plage de 1% en poids à 25% en poids, de manière particulièrement préférée dans la plage de 2% en poids à 20% en poids, calculé sous forme de métal et par rapport au poids total du matériau d'adsorption pourvu du composant métallique.

3. Procédé selon la revendication 1 ou 2,
l'étage d'oxydation du métal étant situé dans la plage de +I à +VII, en particulier dans la plage de +I à +IV, de préférence dans la plage de +I à +III et étant de manière particulièrement préférée +I ou +II ; et/ou le composant métallique présentant au moins un métal, choisi dans le groupe formé par Zn(+II), Ag(+I), Sn(+II), Sn(+IV), Ni(+II), Cu(+I) et Cu(+II), de préférence Cu(+I) et/ou Cu(+II), de manière particulièrement préférée Cu(+I) ; et/ou le composant métallique se trouvant sous forme d'un oxyde de cuivre et/ou le composant métallique se trouvant sous forme de CuO et/ou de Cu₂O, en particulier de Cu₂O.

4. Procédé selon l'une quelconque des revendications précédentes,
le composant métallique étant généré in situ sous forme de l'oxyde métallique dans le système interne de pores du matériau d'adsorption sur base d'au moins un composé précurseur, en particulier d'au moins un sel précurseur, du composant métallique, le composé précurseur, en particulier le sel précurseur, étant utilisé sous forme d'une solution et/ou d'une dispersion de préférence aqueuse(s), la solution et/ou la dispersion contenant en particulier le composé précurseur, en particulier le sel précurseur, en des quantités dans la plage de 0,5% en poids à 80% en poids, en particulier dans la plage de 1% en poids à 70% en poids, de préférence dans la plage de 2% en poids à 60% en poids, de préférence dans la plage de 5% en poids à 50% en poids, par rapport à la solution et/ou à la dispersion, et/ou la solution et/ou la dispersion aqueuse(s) contenant en particulier le composé précurseur, en particulier le sel précurseur, en des concentrations dans la plage de 0,2 mole/l à 8 moles/l, en particulier dans la plage de 0,5 mole/l à 7 moles/l, de préférence dans la plage de 1 mole/l à 6 moles/l, de préférence dans la plage de 1,5 mole/l à 5 moles/l, par rapport à la solution et/ou à la dispersion ; et/ou
le composant métallique étant obtenu sous forme de l'oxyde métallique dans le système interne de pores du matériau d'adsorption par apprêt, en particulier par imbibition et/ou par imprégnation, du matériau d'adsorption avec au moins une solution et/ou dispersion de préférence aqueuse(s) d'un composé précurseur, en particulier d'au moins un sel précurseur, du composant métallique et par transformation et/ou conversion consécutive(s), en particulier par transformation thermique et/ou conversion thermique et/ou décomposition thermique, dudit au moins un composé précurseur, en particulier dudit au moins un sel précurseur, en oxyde métallique, un séchage des matériaux d'adsorption pourvu du composé précurseur, en particulier du sel précurseur, étant réalisé en particulier après l'apprêt, en particulier après l'imbibition et/ou l'imprégnation, et/ou avant la transformation et/ou la conversion, le séchage étant en particulier réalisé à des températures dans la plage de 50°C à 200°C et/ou pendant une durée de jusqu'à 5 h.

5. Procédé selon la revendication 4,
un sel inorganique étant utilisé comme composé précurseur, en particulier comme sel précurseur, et/ou le composé précurseur, en particulier le sel précurseur, étant choisi dans le groupe formé par les sels d'halogénure, les sulfates, les sulfures, les sulfites, les nitrates, les nitrites, les nitrures, les phosphates, les phosphures, les phosphites, les carbamates, les alcoolates, les carbonates et les hydrogénocarbonates, en particulier les nitrates ; et/ou
un nitrate étant utilisé comme composé précurseur, en particulier comme sel précurseur, et/ou un sel de cuivre étant utilisé comme composé précurseur, en particulier comme sel précurseur, en particulier un sel de cuivre (II), de préférence le nitrate de cuivre, de préférence le nitrate de cuivre (II) (Cu(NO₃)₂) ; et/ou les paramètres de réaction et/ou de transformation, en particulier la température et/ou l'atmosphère, de la transformation et/ou de la conversion du composé précurseur, en particulier du sel précurseur, en oxyde métallique étant choisis pour le réglage et/ou en vue de l'étage d'oxydation résultant, en particulier du nombre d'oxydation, du métal dans l'oxyde métallique résultant ; et/ou
la transformation et/ou la conversion du composé précurseur, en particulier du sel précurseur, en oxyde métallique étant réalisée(s) dans une atmosphère de gaz inerte, en particulier dans une atmosphère de gaz inerte contenant de l'azote et/ou contenant un gaz noble ; et/ou
la transformation et/ou la conversion du composé précurseur, en particulier du sel précurseur, en oxyde métallique étant réalisée(s) à des températures dans la plage de 100°C à 1000°C, en particulier dans la plage de 150°C à 950°C, de préférence de 200°C à 920°C.

6. Procédé selon la revendication 4 ou 5,
la transformation et/ou la conversion et/ou la décomposition du composé précurseur, en particulier du sel précurseur, en oxyde métallique étant réalisée(s) à des températures de jusqu'à 400°C et/ou à des températures dans la plage de 200°C à 400°C,
un nitrate de cuivre, en particulier un nitrate de cuivre (II) et/ou du Cu(NO₃)₂, étant en particulier utilisé comme composé précurseur, en particulier comme sel précurseur, et/ou au moins un oxyde de cuivre, en particulier l'oxyde de cuivre (II) (CuO), étant en particulier obtenu comme oxyde métallique ; ou
la transformation et/ou la conversion dudit au moins un composé précurseur, en particulier dudit au moins un sel précurseur, en oxyde métallique étant réalisée(s) à des températures supérieures à 400°C et/ou à des températures dans la plage de 400°C à 920°C,
un nitrate de cuivre, en particulier un nitrate de cuivre (II) et/ou du Cu(NO₃)₂, étant en particulier utilisé comme composé précurseur, en particulier comme sel précurseur, et/ou au moins un oxyde de cuivre, en particulier l'oxyde de cuivre (I) (Cu₂O), étant en particulier obtenu comme oxyde métallique.

7. Procédé selon l'une quelconque des revendications précédentes,
un gaz de guerre chimique et/ou son succédané et/ou son produit de remplacement étant utilisé(s) comme substance chimique nuisible, la substance chimique nuisible étant choisie dans le groupe formé par le gaz moutarde, le soman, le sarin, le phosgène et le tabon ainsi que leurs mélanges, et/ou un produit de remplacement et/ou un succédané du gaz moutarde, du soman, du sarin, du phosgène et/ou du tabon, en particulier un produit de remplacement et/ou un succédané du gaz moutarde, étant utilisé(s) comme gaz de guerre chimique et/ou du sulfure chloroéthyléthyle (CEES) étant utilisé comme substance chimique nuisible, en particulier comme gaz de guerre chimique et/ou biologique ; et/ou
la mise en contact du matériau d'adsorption avec la substance chimique nuisible ayant lieu de manière telle que la substance chimique nuisible pénètre au moins partiellement dans le système interne de pores du matériau d'adsorption et/ou de manière telle que la substance chimique nuisible est en mesure de pénétrer dans le système interne de pores du matériau d'adsorption ; et/ou
la mise en contact du matériau d'adsorption avec la substance chimique nuisible et/ou la décomposition et/ou la dégradation de la substance chimique nuisible ayant lieu dans des conditions stationnaires ou dynamiques ; et/ou
la substance chimique nuisible étant additionnée à une atmosphère, en particulier à une atmosphère de réaction, et/ou le matériau d'adsorption étant mis en contact avec l'atmosphère contenant la substance chimique nuisible, l'atmosphère contenant en particulier la substance chimique nuisible en des quantités dans la plage de 0,1 g/m³ à 20 g/m³, en particulier dans la plage de 0,5 g/m³ à 10 g/m³, de préférence dans la plage de 0,75 g/m³ à 5 g/m³ ; et/ou la substance chimique nuisible étant en particulier au moins partiellement liée dans le système interne de pores du matériau d'adsorption, en particulier sur base d'une sorption, de préférence une adsorption, en particulier sur base d'une physisorption et/ou d'une chimisorption ; et/ou
le matériau d'adsorption présentant et/ou adsorbant et/ou liant la substance chimique nuisible en des quantités dans la plage de 0,01% en poids à 80% en poids, en particulier dans la plage de 0,05% en poids à 60% en poids, de préférence dans la plage de 0,1% en poids à 40% en poids, de préférence dans la plage de 1% en poids à 30% en poids, par rapport au poids total du matériau d'adsorption pourvu du composant métallique.

8. Procédé selon l'une quelconque des revendications précédentes,
la substance chimique nuisible étant en particulier au moins partiellement dégradée et/ou décomposée dans le système interne de pores du matériau d'adsorption et/ou la dégradation et/ou la décomposition ayant lieu par dissociation de molécules, de préférence avec déshalogénation et/ou déshydrohalogénation et/ou hydrolyse, de la substance chimique nuisible, et/ou la dégradation ayant lieu sur base d'une transformation stoechiométrique et/ou réactive et/ou sur base d'une transformation catalytique et/ou sur base d'une oxydation, les produits de décomposition de la substance chimique nuisible étant en particulier au moins partiellement désorbés et/ou libérés dans l'atmosphère entourant le matériau d'adsorption, en particulier de manière telle que les sites d'adsorption et/ou les sites de fixation du matériau d'adsorption occupés au préalable par la substance chimique nuisible sont libérés pour la fixation de substance chimique nuisible supplémentaire.

9. Procédé selon l'une quelconque des revendications précédentes,
la mise en contact du matériau d'adsorption avec la substance chimique nuisible et/ou la décomposition et/ou la dégradation de la substance chimique nuisible étant réalisée(s) à des températures dans la plage de -20°C à 100°C, en particulier dans la plage de 5°C à 60°C, de préférence dans la plage de 10°C à 40°C, et/ou la mise en contact du matériau d'adsorption avec la substance chimique nuisible et/ou la décomposition et/ou la dégradation de la substance chimique nuisible étant réalisée(s) pendant une durée dans la plage de 1 min à 5000 min, en particulier dans la plage de 10 min à 4000 min, de préférence dans la plage de 60 min à 2500 min, de préférence dans la plage de 100 min à 2000 min.

10. Procédé selon l'une quelconque des revendications précédentes,
le matériau d'adsorption étant apprêté et/ou chargé par de l'eau ; et/ou
le matériau d'adsorption présentant une teneur en humidité et/ou en eau dans la plage de 2% en poids à 55%, de préférence dans la plage de 3% en poids à 50% en poids, de préférence dans la plage de 5% en poids à 50% en poids, par rapport au poids sec du matériau d'adsorption pourvu du composant métallique.

11. Procédé selon l'une quelconque des revendications précédentes,
le matériau d'adsorption présentant des grosseurs de particule, en particulier des diamètres de particule dans la plage de 0,001 à 3 mm, en particulier dans la plage de 0,005 à 2,5 mm, de préférence dans la plage de 0,01 à 2 mm, de manière particulièrement préférée dans la plage de 0,02 à 1,5 mm, de manière tout particulièrement préférée dans la plage de 0,05 à 1 mm et/ou
le matériau d'adsorption présentant des grosseurs moyennes de particule, en particulier des diamètres moyens de particule (D50), dans la plage de 0,01 à 2 mm, en particulier dans la plage de 0,05 à 1,5 mm, de préférence dans la plage de 0,1 à 1 mm.

12. Procédé selon l'une quelconque des revendications précédentes,
le matériau d'adsorption présentant une surface spécifique (surface BET) d'au moins 500 m²/g, en particulier d'au moins 750 m²/g, de préférence d'au moins 1000 m²/g, de manière particulièrement préférée d'au moins 1200 m²/g et/ou
le matériau d'adsorption présentant une surface spécifique (surface BET) dans la plage de 500 à 4000 m²/g, en particulier dans la plage de 750 à 3000 m²/g, de préférence dans la plage de 900 à 2500 m²/g, de manière particulièrement préférée dans la plage de 950 à 2000 m²/g.

13. Filtres et matériaux de filtre pour l'élimination de substances nuisibles, de substances odoriférantes et de substances toxiques de tous types, fabriqués avec utilisation d'un matériau d'adsorption autodétoxifiant et/ou autopurifiant et/ou présentant un matériau d'adsorption autodétoxifiant et/ou autopurifiant,
le matériau d'adsorption étant formé à partir de charbon actif sphérique, le charbon actif étant obtenu par carbonisation et activation consécutive de polymères organiques sulfonés, sphériques, et
le matériau d'adsorption présentant un système interne de pores, le système interne de pores du matériau d'adsorption étant pourvu d'au moins un composant métallique, le composant métallique étant introduit et/ou incorporé dans le système interne de pores, le composant métallique étant généré in situ sous forme d'un oxyde métallique dans le système interne de pores du matériau d'adsorption, le composant métallique étant réalisé en tant qu'oxyde métallique sous une forme au moins partiellement cristalline, le matériau d'adsorption présentant le composant métallique en des quantités dans la plage de 0,001% en poids à 40% en poids, calculés sous forme de métal et par rapport au poids total du matériau d'adsorption pourvu du composant métallique et le composant métallique présentant au moins un métal dans un étage d'oxydation positif, le composant métallique présentant au moins un métal choisi dans le groupe formé par Zn, Ag, Sn, Ni et Cu, de préférence Cu,
le matériau d'adsorption présentant une teneur en humidité et/ou en eau dans la plage de 1% en poids à 60% en poids, par rapport au poids sec du matériau d'adsorption pourvu du composant métallique et
le matériau d'adsorption étant appliqué sur un matériau support.

14. Filtres et matériaux de filtre selon la revendication 13,
le matériau support présentant une structure tridimensionnelle, le matériau support étant en particulier conçu sous forme d'une mousse, de manière particulièrement préférée d'une mousse de polyuréthane, de préférence à pores ouverts ; ou
le matériau support présentant une structure bidimensionnelle et/ou plane, le matériau support étant en particulier conçu sous forme de structure plane de préférence textile,
le matériau support étant en particulier conçu sous forme d'une structure plane textile, de préférence un matériau textile perméable à l'air, de préférence un tissu, une étoffe à mailles, un tricot, une nappe ou un matériau composite textile, en particulier un non-tissé (Nonwoven), et/ou le matériau support présentant en particulier un poids surfacique de 5 à 1000 g/m², en particulier de 10 à 500 g/m², de préférence de 25 à 450 g/m² et/ou
le matériau support étant en particulier une structure plane textile contenant ou constituée par des fibres naturelles et/ou des fibres synthétiques (fibres chimiques) et/ou
le matériau d'adsorption étant en particulier fixé au et/ou sur le matériau support, de préférence au moyen d'un collage, en particulier au moyen d'un adhésif ou suite à l'autoadhésivité ou l'autoadhérence.
